(19) Europäisches Patentamt

European Patent Office

Office européen des brevets

(11) EP 1 350 795 A1

(12) **EUROPEAN PATENT APPLICATION**
published in accordance with Art. 158(3) EPC

(43) Date of publication:
08.10.2003 Bulletin 2003/41

(51) Int Cl.⁷: **C07K 1/00**, C07K 14/705,
G06F 17/30, G06F 17/50

(21) Application number: 01965587.7

(22) Date of filing: 12.09.2001

(86) International application number:
PCT/JP01/07918

(87) International publication number:
WO 02/022653 (21.03.2002 Gazette 2002/12)

(84) Designated Contracting States:
AT BE CH CY DE DK ES FI FR GB GR IE IT LI LU
MC NL PT SE TR
Designated Extension States:
AL LT LV MK RO SI

(30) Priority: 14.09.2000 JP 2000279155
17.11.2000 JP 2000350378

(71) Applicant: Umeyama, Hideaki
Chiba 279-0011 (JP)

(72) Inventors:
• UMEYAMA, Hideaki
Urayasu-shi, Chiba 279-0011 (JP)
• IWADATE, Mitsuo
Hanyu-shi, Saitama 348-0026 (JP)

(74) Representative:
Leson, Thomas Johannes Alois, Dipl.-Ing. et al
Tiedtke-Bühling-Kinne & Partner GbR,
TBK-Patent,
Bavariaring 4
80336 München (DE)

(54) **METHOD OF CONSTRUCTING THREE-DIMENSIONAL STRUCTURE OF TRANSMEMBRANE PROTEIN**

(57) An object of the present invention is to provide a method for constructing the steric structure of an arbitrary 7-transmembrane G-protein-coupled receptor with good precision.

The essential feature of the present invention is a method for constructing the steric structure of a transmembrane protein, which comprises selecting the steric structure to be referred to from the known steric structures of 7-transmembrane G-protein-coupled receptors using a database of amino acid sequences of the receptors (GCRDb) as a profile, performing alignment of a desired protein having an unknown steric structure, and producing the steric structure of the desired protein having an unknown steric structure as a G-protein-coupled receptor to construct the steric structure of a transmembrane protein.

FIG.1

DESIRED AMINO ACID SEQUENCE — 10

DATA BASE RETRIEVAL AND SEQUENCE ALIGNMENT — 20

CONSTRUCTION AND OPTIMIZATION OF Cα ATOM INITIAL COORDINATE — 30

CONSTRUCTION AND OPTIMIZATION OF MAIN CHAIN ATOMIC COORDINATE — 40

CONSTRUCTION AND OPTIMIZATION OF SIDE CHAIN ATOMIC COORDINATE — 50

FINAL STRUCTURE — 60

SEQUENCE LISTING

<110> Umeyama, Hideaki and Mitsubishi Chemical Corporation

<120> A method for determination of three-dimensional structure of gua

nine nucleotide-binding protein (G-protein)-coupled receptor

<130>

<150> JP 2000-279155

<151> 2000-09-14

<150> JP 2000-350378

<151> 2000-11-17

<160> 4


<210> 1

<211> 9

<212> PRT

<213> Bos taurus

<220>

<223>

<400> 1

Tyr Phe Val Phe Gly Pro Thr Gly Cys

   1                 5


<210> 2

<211> 28

<212> PRT

<213> Bos taurus

```
<220>

<223>

<400> 2

Gly Trp Ser Arg Tyr Ile Pro Glu Gly Met Gln Cys Ser Cys Gly Ile
1               5               10              15

Asp Tyr Tyr Thr Pro His Glu Glu Thr Asn Asn Glu
                20              25


<210> 3

<211> 413

<212> PRT

<213> Homo sapiens

<220>

<223>

<400> 3

Met Gly Gln Pro Gly Asn Gly Ser Ala Phe Leu Leu Ala Pro Asn Arg
1               5               10              15

Ser His Ala Pro Asp His Asp Val Thr Gln Gln Arg Asp Glu Val Trp
                20              25              30

Val Val Gly Met Gly Ile Val Met Ser Leu Ile Val Leu Ala Ile Val
                35              40              45

Phe Gly Asn Val Leu Val Ile Thr Ala Ile Ala Lys Phe Glu Arg Leu
                50              55              60

Gln Thr Val Thr Asn Tyr Phe Ile Thr Ser Leu Ala Cys Ala Asp Leu
65              70              75              80
```

Val Met Gly Leu Ala Val Val Pro Phe Gly Ala Ala His Ile Leu Met

                    85                  90                  95

Lys Met Trp Thr Phe Gly Asn Phe Trp Cys Glu Phe Trp Thr Ser Ile

                100                 105                 110

Asp Val Leu Cys Val Thr Ala Ser Ile Glu Thr Leu Cys Val Ile Ala

                115                 120                 125

Val Asp Arg Tyr Phe Ala Ile Thr Ser Pro Phe Lys Tyr Gln Ser Leu

            130                 135                 140

Leu Thr Lys Asn Lys Ala Arg Val Ile Ile Leu Met Val Trp Ile Val

        145                 150                 155                 160

Ser Gly Leu Thr Ser Phe Leu Pro Ile Gln Met His Trp Tyr Arg Ala

                165                 170                 175

Thr His Gln Glu Ala Ile Asn Cys Tyr Ala Asn Glu Thr Cys Cys Asp

                180                 185                 190

Phe Phe Thr Asn Gln Ala Tyr Ala Ile Ala Ser Ser Ile Val Ser Phe

            195                 200                 205

Tyr Val Pro Leu Val Ile Met Val Phe Val Tyr Ser Arg Val Phe Gln

        210                 215                 220

Glu Ala Lys Arg Gln Leu Gln Lys Ile Asp Lys Ser Glu Gly Arg Phe

    225                 230                 235                 240

His Val Gln Asn Leu Ser Gln Val Glu Gln Asp Gly Arg Thr Gly His

                245                 250                 255

Gly Leu Arg Arg Ser Ser Lys Phe Cys Leu Lys Glu His Lys Ala Leu

            260                 265                 270

Lys Thr Leu Gly Ile Ile Met Gly Thr Phe Thr Leu Cys Trp Leu Pro

```
                275              280              285

Phe Phe Ile Val Asn Ile Val His Val Ile Gln Asp Asn Leu Ile Arg

        290              295              300

Lys Glu Val Tyr Ile Leu Leu Asn Trp Ile Gly Tyr Val Asn Ser Gly

305              310              315              320

Phe Asn Pro Leu Ile Tyr Cys Arg Ser Pro Asp Phe Arg Ile Ala Phe

                325              330              335

Gln Glu Leu Leu Cys Leu Arg Arg Ser Ser Leu Lys Ala Tyr Gly Asn

            340              345              350

Gly Tyr Ser Ser Asn Gly Asn Thr Gly Glu Gln Ser Gly Tyr His Val

        355              360              365

Glu Gln Glu Lys Glu Asn Lys Leu Leu Cys Glu Asp Leu Pro Gly Thr

    370              375              380

Glu Asp Phe Val Gly His Gln Gly Thr Val Pro Ser Asp Asn Ile Asp

385              390              395              400

Ser Gln Gly Arg Asn Cys Ser Thr Asn Asp Ser Leu Leu

            405              410
```

<210> 4

<211> 348

<212> PRT

<213> Bos taurus

<220>

<223>

<400> 4

128

Met Asn Gly Thr Glu Gly Pro Asn Phe Tyr Val Pro Phe Ser Asn Lys
1           5           10          15

Thr Gly Val Val Arg Ser Pro Phe Glu Ala Pro Gln Tyr Tyr Leu Ala
        20          25          30

Glu Pro Trp Gln Phe Ser Met Leu Ala Ala Tyr Met Phe Leu Leu Ile
        35          40          45

Met Leu Gly Phe Pro Ile Asn Phe Leu Thr Leu Tyr Val Thr Val Gln
        50          55          60

His Lys Lys Leu Arg Thr Pro Leu Asn Tyr Ile Leu Leu Asn Leu Ala
        65          70          75          80

Val Ala Asp Leu Phe Met Val Phe Gly Gly Phe Thr Thr Thr Leu Tyr
            85          90          95

Thr Ser Leu His Gly Tyr Phe Val Phe Gly Pro Thr Gly Cys Asn Leu
        100         105         110

Glu Gly Phe Phe Ala Thr Leu Gly Gly Glu Ile Ala Leu Trp Ser Leu
        115         120         125

Val Val Leu Ala Ile Glu Arg Tyr Val Val Val Cys Lys Pro Met Ser
        130         135         140

Asn Phe Arg Phe Gly Glu Asn His Ala Ile Met Gly Val Ala Phe Thr
        145         150         155         160

Trp Val Met Ala Leu Ala Cys Ala Ala Pro Pro Leu Val Gly Trp Ser
            165         170         175

Arg Tyr Ile Pro Glu Gly Met Gln Cys Ser Cys Gly Ile Asp Tyr Tyr
            180         185         190

Thr Pro His Glu Glu Thr Asn Asn Glu Ser Phe Val Ile Tyr Met Phe

```
              195            200            205

     Val Val His Phe Ile Ile Pro Leu Ile Val Ile Phe Phe Cys Tyr Gly

         210            215            220

     Gln Leu Val Phe Thr Val Lys Glu Ala Ala Ala Gln Gln Gln Glu Ser

     225            230            235            240

     Ala Thr Thr Gln Lys Ala Glu Lys Glu Val Thr Arg Met Val Ile Ile

                245            250            255

     Met Val Ile Ala Phe Leu Ile Cys Trp Leu Pro Tyr Ala Gly Val Ala

                260            265            270

     Phe Tyr Ile Phe Thr His Gln Gly Ser Asp Phe Gly Pro Ile Phe Met

         275            280            285

     Thr Ile Pro Ala Phe Phe Ala Lys Thr Ser Ala Val Tyr Asn Pro Val

         290            295            300

     Ile Tyr Ile Met Met Asn Lys Gln Phe Arg Asn Cys Met Val Thr Thr

     305            310            315            320

     Leu Cys Cys Gly Lys Asn Pro Leu Gly Asp Asp Glu Ala Ser Thr Thr

                325            330            335

     Val Ser Lys Thr Glu Thr Ser Gln Val Ala Pro Ala

         340            345
```

**Description**

TECHNICAL FIELD

The present invention relates to a method for preparing the steric structure of a protein. More particularly, this invention relates to a method for preparing the steric structure of a 7-transmembrane G-protein-coupled receptor with good precision, a database and the like containing atomic coordinates defining the steric structure of a protein obtained by the method.

It is said that a 7-transmembrane G-protein-coupled receptors account for more than half of receptors which have been cloned up to now, and the method and the database and the like of the present invention are extremely, useful in the field of molecular design of medicines and agricultural chemicals.

BACKGROUND ART

An approach has made it possible to construct the steric structure of a desired protein having an unknown steric structure based on information of the alignment with the desired protein wherein the alignment is obtained by utilizing information about proteins having a known steric structure. This approach is usually called homology modeling. Although the precision of the steric structure constructed by homology modeling has been remarkably improved recently, there are still many problems to be solved.

On the other hand, since a membrane protein is difficult to crystallize experimentally, it has been difficult to apply X-ray crystal diffraction which is a main stream for determining a structure in structural biology. As an attempt has been tried to determine a structure of a membrane protein with an electron microscope from a two-dimensional crystal, a structure with some extent of precision is obtained to an extent of a precision and, therefrom, it has become possible to construct a model. However, its precision has not yet reached that of single crystal diffraction by X-ray, and structural information of a loop part which is not a transmembrane region and of a side chain part of an amino acid has been poor.

Among membrane proteins, a 7-transmembrane G-protein-coupled receptor (guanine nucleotide-binding protein-coupled receptor; hereinafter this may be abbreviated as "GCPRs" or "GPCR") is a protein which has the common nature of activating a G-protein when receives various extracellular stimulations such as calcium ion, amines, various hormones, peptides and proteins, and transmitting information into cells, which and has an important function in a living body. The common characteristics of structures of GPCRs are 7-transmembrane $\alpha$ helix structures and loop structures of a variety of lengths connecting them, and it is considered that these common characteristics determine the natures of GPCRs. As seen in trypsins of a serine protease having the characteristic relationship between its structure and function, also in GPCRs, it is presumed that the above-mentioned structural characteristics serve as an information transmitting mechanism.

Since GPCRs are particularly difficult to crystallize, information about their steric structures is poor. Even under this situation, regarding the model construction, for example, there are reports such as modeling combined with a site-specific mutation experiment of a CB (2) receptor (Eur J Pharmacol 2000 Jul 28; 401 (1): 17-25), modeling combined with a site-specific mutation experiment of a CCK (1) receptor (Eur J Pharmacol 2000 Jul 21; 400 (2-3): 185-194), a theoretical model referring to the function (Protein Eng 2000 Jul; 13 (7): 477-90), construction of a NK2 receptor combined with a mutation experiment (Biochem J 1999 Apr 1; 339 (Pt 1): 55-61), modeling regarding frog rhodopsin (J Mol Biol 1998, Aug 28; 281 (4): 741-54), modeling of a transmembrane receptor combined with a site-specific mutation (Biophys J 1998 Mar; 74 (3): 1203-14) and the like. However, the satisfactory results have not been necessarily obtained in these modelings.

The cause why a decisive steric structure of a membrane protein has not been proposed up to now is that a membrane protein hardly becomes a single crystal due to the nature of the membrane protein orienting in a membrane of a lipid bilayer and thus it is difficult to apply X-ray diffraction. Since a membrane protein is fundamentally of $\alpha$ helix and there is no diversity in its steric structure, there is the background that it is difficult to determine the structure by normal approaches of NMR and a distance geometry method. For that reason, the structural information of a membrane protein is generally analyzed by electron beam diffraction of a two-dimensional crystal orienting in a membrane, but its resolution is 6 to 7 angstrom, not reaching the precision of crystal diffraction at all.

In addition, a modeling method combined with a site-specific mutation is a method of performing substitution of an amino acid at a site which is considered to be important for the activity, and examining the influence on the activity of an agonist or an antagonist. Although this method has such an advantage that detailed discussion can be made as to the surroundings of the active site, a model can not be proposed until a mutated strain of a bacterium producing a number of substituted bodies is provided, until a reaction mechanism of stimulation to GPCRs is well known and, additionally, until geometric information is obtained, and operations are troublesome and, thus, it can hardly be said that this method will give the results with good precision.

Like this, as for a membrane protein, there has been only a scarcely minute structure obtained by electron beam diffraction, or a structure from bacteriorhodopsin or the like which is not functionally equal to GPCRs, and the situation has been such that there were conventionally no choices for a reference protein. In addition, since details of a side chain of a reference protein are not determined, the situation has not been such that the performance of a software is fully displayed despite that it is possible to determine even the detailed structure of a side chain by the software. In addition, conventionally, a protein with low homology including GPCRs could not be modeled and, even when there is one experimentally determined structure, it is judged that construction of a model is impossible at an alignment stage before construction of a model in many cases.

On the other hand, it is considered that rhodopsin, one of a GPCRs family accounting for a majority of membrane proteins, is activated by light stimulation and transmits information. Recently, it has been reported that the steric structure of this protein (PDB ID:1F88) including a side chain is determined in detail by X-ray crystal analysis (Science, vol. 289, 4 August 2000, 739-745).

DISCLOSURE OF THE INVENTION

In view of the above-mentioned situation, the present invention has been achieved in order to provide a method for constructing the steric structure of arbitrary GPCRs with good precision, a database and the like of atomic coordinates defining the steric structure of GPCRs which can be utilized for molecular design of medicines and agricultural chemicals.

The present inventors have intensively studied in order to attain the above-mentioned object and, as a result, found that the precision of the steric structure model of arbitrary GPCRs can be remarkably improved by using a database of amino acid sequences of GPCRs as a profile and referring to the steric structure of rhodopsin (PDB ID: 1F88), compared to the time when only a conventional structure having a different helix arrangement and not being classified into GCRDb of bacteriorhodopsin has been obtained. That is, we found that the steric structure of arbitrary GPCRs can be constructed with good precision by using the database of amino acid sequences of PCRs "GCRDb" as a motif profile of PSI-BLAST, and performing alignment referring to the steric structure of rhodopsin (PDB ID:1F88). The present invention is completed based on these findings.

That is, according to the method of the present invention, there is provided (1) a method for constructing the steric structure of a transmembrane protein, which comprises selecting a protein having the steric structure to be referred to from the known steric structures of 7-transmembrane G-protein-coupled receptors using a database of amino acid sequences of the receptors as a profile, performing alignment of a desired protein having an unknown steric structure, and producing the steric structure of the desired protein having an unknown steric structure as a G-protein-coupled receptor.

According to a preferable aspect of the present invention, there are provided (2) the method described in the above (1), wherein alignment is performed by selecting a protein having the steric structure to be referred to from a database of known steric structures of G-protein-coupled receptors, and by juxtaposing an amino acid sequence of the selected reference protein and an amino acid sequence of the desired protein; (3) the method described in (2), wherein a structure to be referred to is the steric structure of rhodopsin (PDB ID:1F88); (4) the method described in any one of the above (1) to (4), wherein the database of amino acid sequences used as a profile is selected from the group consisting of GCRDb, GPCRDB, ExPASy and ORDB; and (5) the above-mentioned method, wherein the database of amino acid sequences is used as a motif profile of PSI-BLAST.

According to a preferable aspect of the present invention, there are provided (6) the method described in above (5), wherein in the alignment performed by PSI-BLAST, the identity between a final site-specific score matrix and the reference protein is calculated as an E value and, when the E value has a value of 0.1 or smaller, the steric structure is produced, (7) the method described in any one of the above (1) to (6), wherein regarding given alignment information, re-alignment is performed in view of a combination of disulfide bonds, and the steric structure is produced based on this re-alignment information, and (8) the method described in any one of the above (1) to (7), wherein production of the steric structure comprises obtaining a coordinate from the steric structure which is referred to with respect to C$\alpha$ atoms in an amino acid based on the resulting alignment information, optimizing an atomic coordinate of C$\alpha$ so as to minimize an objective function, adding other atoms of a main chain to an optimized atomic coordinate of C$\alpha$ to optimize an atomic coordinate of a main chain so as to minimize an objective function, and adding other atoms of a side chain to an optimized atomic coordinate of a main chain to optimize so as to minimize an objective function.

According to another aspect of the present invention, there are provided (9) an atomic coordinate defining the steric structure of a 7-transmembrane G-protein-coupled receptor obtained by the method described in any one of the above (1) to (8), (10) a computer readable recording medium in which the atomic coordinate described in the above (9) is recorded, and (11) a database, which comprises the atomic coordinate described in the above (9).

According to still another aspect of the present invention, there is provided (12) a method of presuming the function of a protein, which method comprises selecting a protein having the steric structure to be referred to from the known steric structures of 7-transmembrane G-protein-coupled receptors using a database of amino acid sequences of the receptors as a profile, performing alignment of a desired protein having the unknown structure and, when the steric structure of the desired protein can be produced, judging that the desired protein is a 7-transmembrane G-protein-coupled receptor.

According to a preferable aspect of the present invention, there is provided (13) the method described in the above (12), wherein the alignment is performed by selecting a protein having the steric structure to be referred to from a database of the known steric structures of G-protein-coupled receptors, and juxtaposing an amino acid sequence of the selected reference protein and an amino acid sequence of the desired protein, the database of amino acid sequences used as a profile is selected from the group consisting of GCRDb, CPCRDB, ExPASy and ORDB and, when alignment has a reliability of 98% or more, it is judged that the steric structure of the desired protein can be produced.

According to still another aspect of the present invention, there are provided (14) a method of designing a drug molecule, which comprises identifying, retrieving, assessing or designing a desired drug molecule based on the interaction between the steric structure of a drug candidate molecule and the steric structure of a 7-transmembrane G-protein-coupled receptor produced by using the atomic coordinate as defined in the above (9), or the atomic coordinate of the recording medium as defined in the above (10) or of the database as defined in the above (11), (15) a method for screening a medicine or an agricultural chemical, which comprises examining the activity and the safety of the drug molecule obtained by the method as defined in the above (14) as a medicine or an agricultural chemical by a desired pharmacological or physiological test, and selecting a drug molecule having the desired nature, (16) a method for manufacturing a medicine or an agricultural chemical, which comprises formulating by blending a drug molecule obtained by the method as defined in the above (15) and a pharmaceutically or agriculturally or horticulturally acceptable carrier, and (17) a drug molecule obtained by the method as defined in the above (14).

BRIEF DESCRIPTION OF THE DRAWINGS

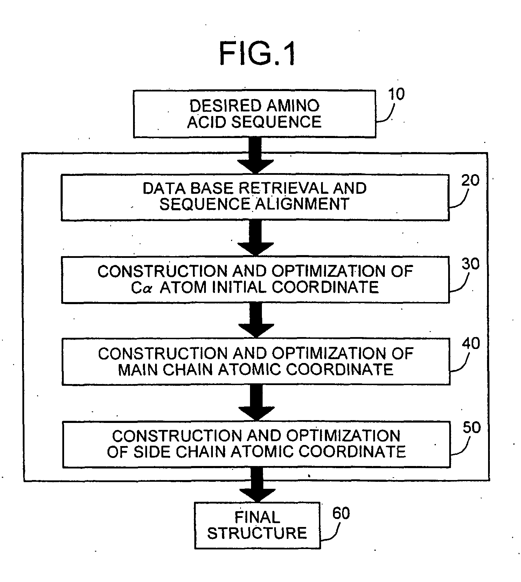

Fig. 1 is a flowchart that shows one example of a method for constructing the steric structure of GPCRs of the present invention.

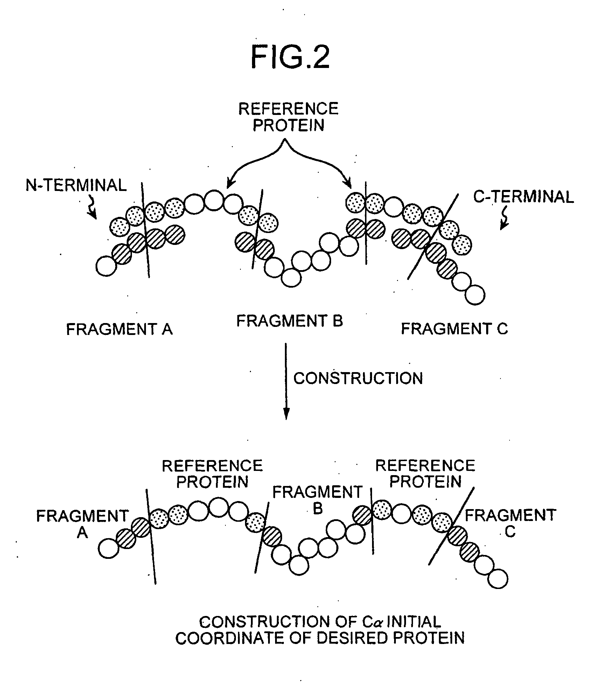

Fig. 2 is a view that shows a method for constructing a C$\alpha$ atomic coordinate at the step 30. An identified part of alignment is obtained from a reference protein and, as to an absene part, minimum rmsd of overlapping of two residues having overlapped both N and C terminals is obtained from a database.

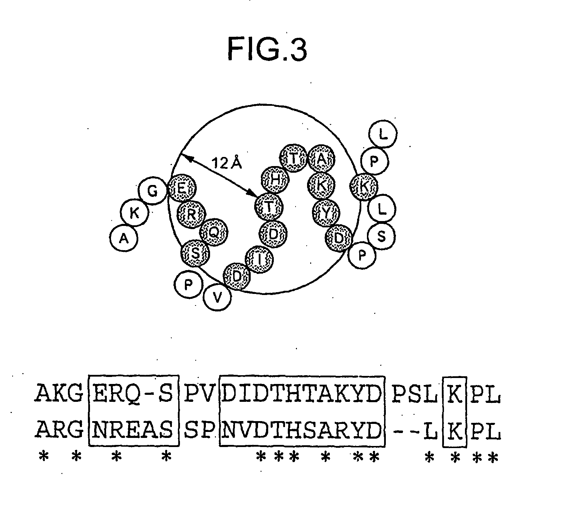

Fig. 3 is a view that shows local space homology (LSH). In calculating regarding T residue in the figure, netted (gray) residues are taken into consideration. A part surrounded by a square in an alignment at a low part of the figure is a residue pair to be considered, and a ratio at * mark is LSH. In this case, LSH is 56.2%.

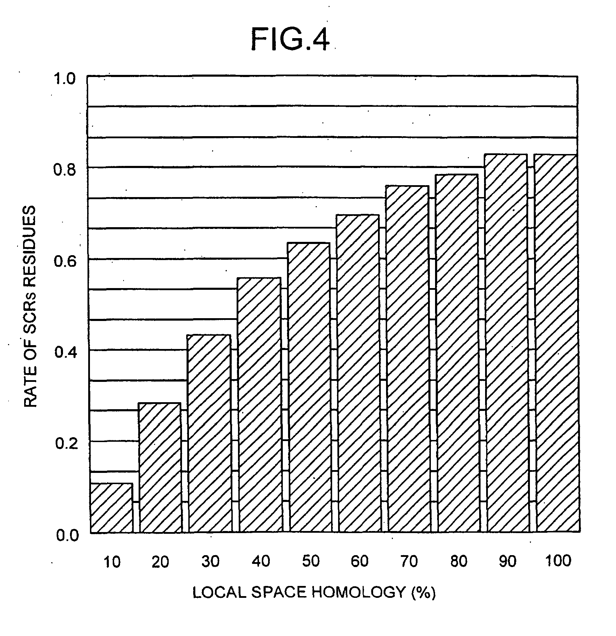

Fig. 4 is a view that shows the relationship between LSH and a ratio at structurally conserved regions (SCRs). LSH is calculated from the overlapping of C$\alpha$ atoms between the desired protein and the reference protein, and a ratio at SCRs is the number of residues in SCRs relative to the total number of residues of the desired protein.

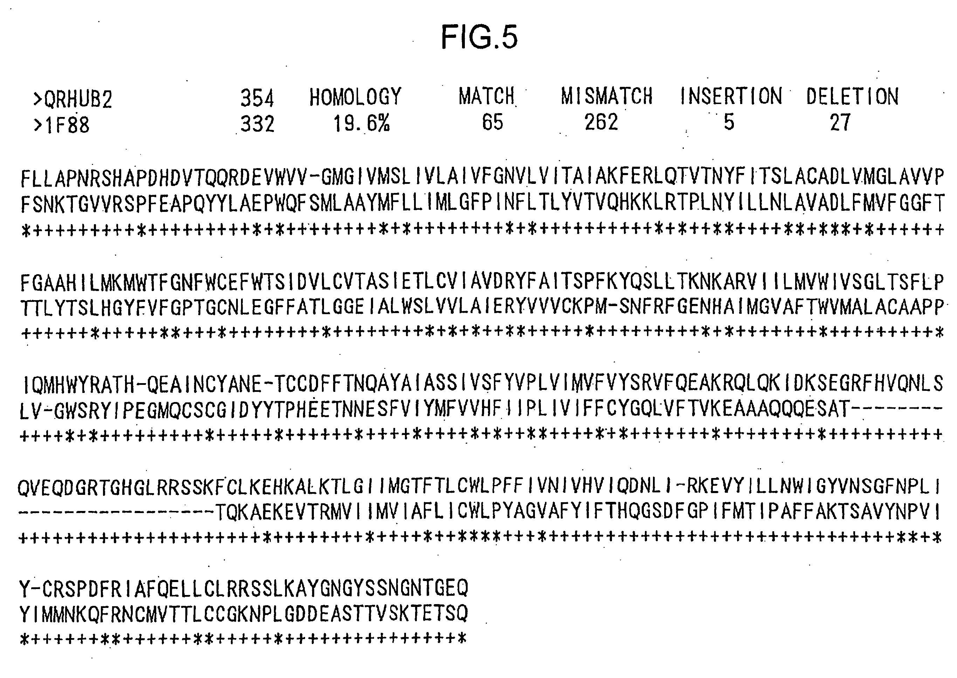

Fig. 5 is a view that shows alignment of QRHUB2 ($\beta$ adrenaline receptor) obtained by using 1F88 (rhodopsin) as a reference protein. In the figure, numerals on the right of QRHUB2 and 1F88 are the number of amino acids subjected to alignment in an amino acid sequence of each protein. In addition, the upper sequence indicates QRHUB2 ($\beta$ adrenaline receptor) and the lower sequence indicates 1F88 (rhodopsin). An amino acid sequence of each protein is shown by a single letter symbol.

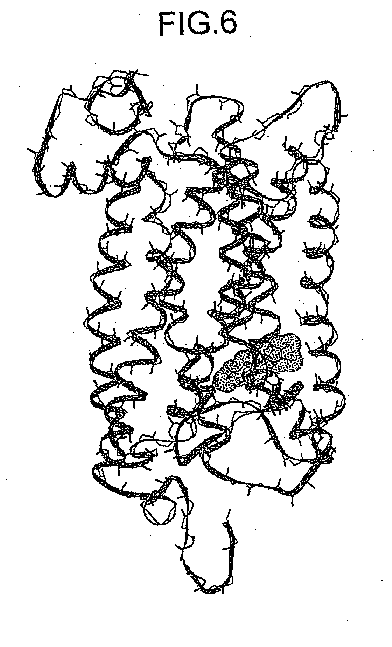

Fig. 6 is a printout of a display that shows bonding between the steric structure model constructed by the present method and an agonist (isoproterenol). The netted part in the figure indicates the agonist. A circumstance can be seen in which this agonist in R conformation is hydrogen-bonded to a receptor.

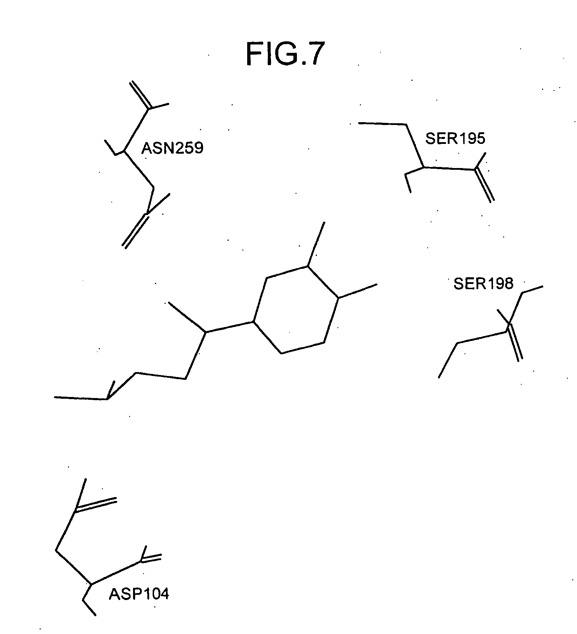

Fig. 7 is a printout of a display that shows the relative position of amino acid residues which are hydrogen-bonded to an agonist when the agonist in R form is introduced in seven $\alpha$ helices of a $\beta$2 adrenaline receptor in Fig. 6.

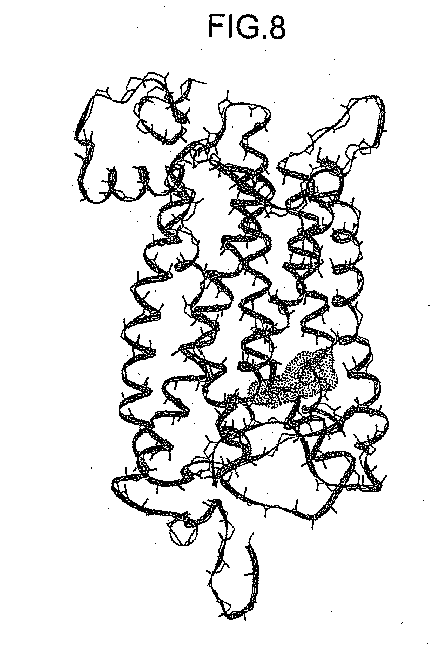

Fig. 8 is a printout of a display that shows bonding between the steric structure model constructed by the present method and an agonist (isoproterenol). The netted part in the figure indicates the agonist. A circumstance can be seen in which this agonist in S conformation is hydrogen-bonded to a receptor.

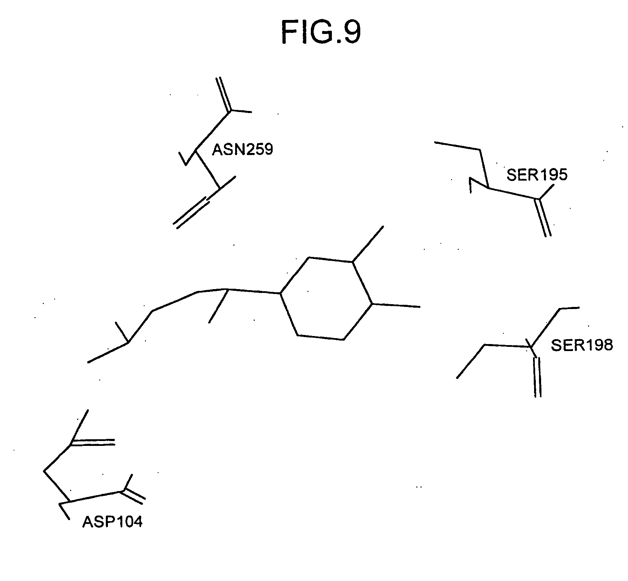

Fig. 9 is a printout of a display that shows the relative position of amino acid residues which are hydrogen-bonded to this agonist when an agonist in S form is introduced in seven $\alpha$ helices of a $\beta$2 adrenaline receptor in Fig. 8. A circumstance can be seen in which the hydrogen bond distance between this agonist and a receptor is instabilized to some extent due to its S comformation.

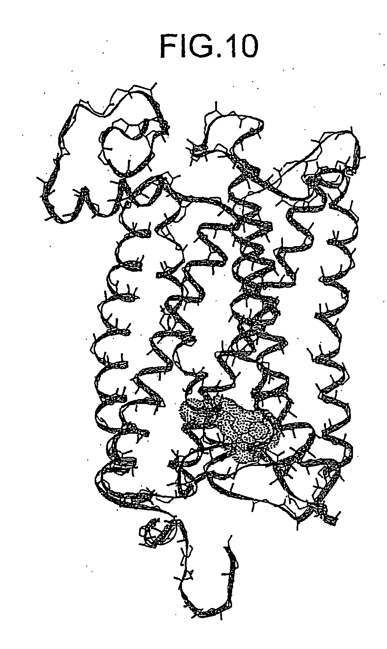

Fig. 10 is a printout of a display that shows bonding between the steric structure model constructed by the present method and an antagonist in R form (propranolol). In the figure, a netted part is an antagonist. A circumstance can be seen in which an antagonist in R form is introduced in seven $\alpha$ helices of a $\beta$2 adrenaline receptor.

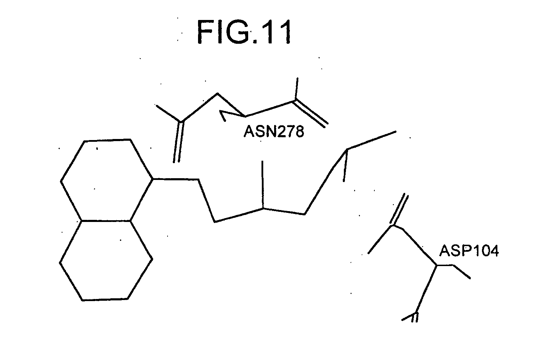

Fig. 11 is a printout of a display that shows the relative position of amino acid residues which are hydrogen-bonded to this antagonist when an antagonist in R form is introduced in seven α helices of a β2 adrenaline receptor in Fig. 10. A circumstance can be seen in which a hydrogen bond distance between this antagonist and a receptor is extended due to its R comformation.

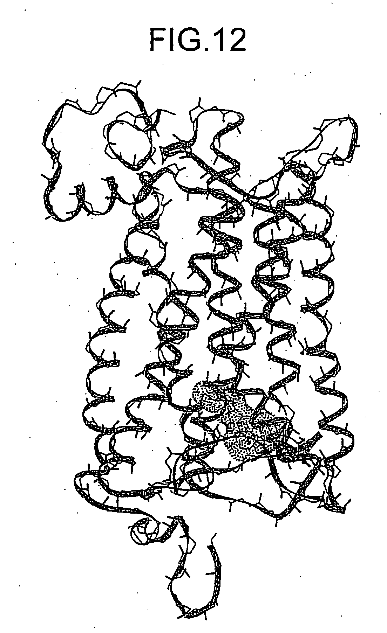

Fig. 12 is a printout of a display that shows bonding between the steric structure model constructed by the present method and an antagonist in S form (propranolol). In the figure, the netted part is the antagonist. A circumstance can be seen in which an antagonist in S form is introduced in seven α helices of a β2 adrenaline receptor.

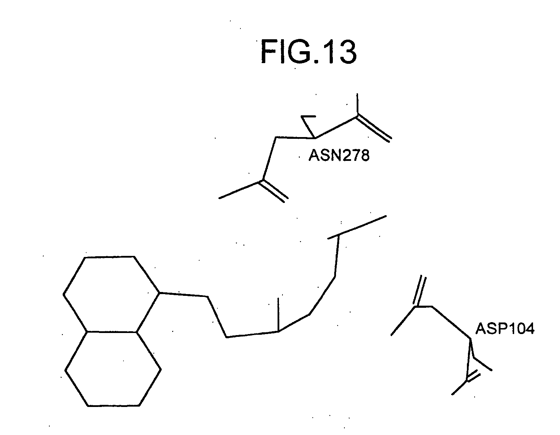

Fig. 13 is a printout of a display that shows the relative position of amino acid residues which are and hydrogen-bonded to this antagonist with some inatalization when the antagonist in S form is introduced in seven α helices of a β2 adrenaline receptor in Fig. 12.

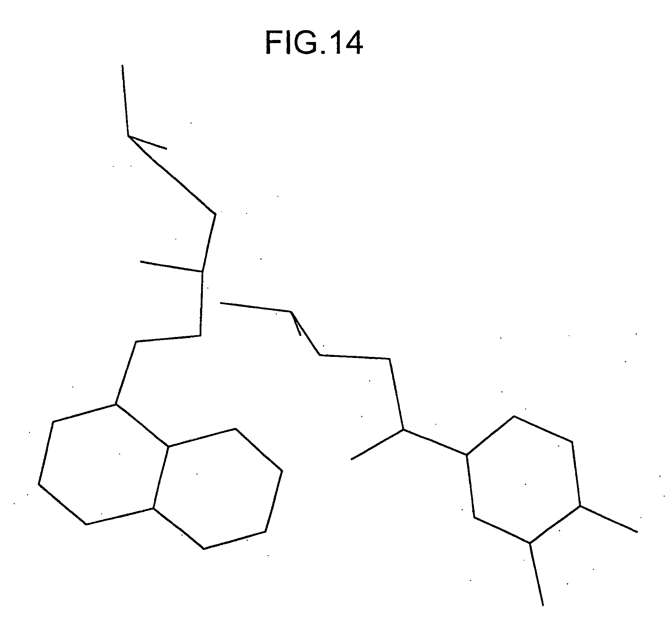

Fig. 14 is a printout of a display that shows the relative position of an antagonist (left) and an agonist (right) in a β2 adrenaline receptor in Figs. 6 to 13. From the figure, it can be seen that an agonist and an antagonist are introduced into pockets at different positions, respectively, as expected from an experiment of site-specific mutation.

## BEST MODE FOR CARRYING OUT THE INVENTION

The present invention will be explained in more detail below.

In the present invention, a "desired protein" means a protein whose complete steric structure has not been determined by X-ray crystal analysis, NMR analysis or the like and which is a subject for steric structure construction and function presumption in the present invention. This protein includes proteins whose complete steric structure has not been obtained while whose partial structure has been analyzed, proteins whose functions have been already specified as GPCRs, proteins presumed to be GPCRs, and proteins whose functions are not clear at all while their amino acid sequences are determined. "A reference protein" is a protein whose minute steric structure has been already determined by X-ray crystal analysis or NMR analysis, and which is to be referred to for alignment and optimization of an atomic coordinate. "Alignment" means obtaining of the corresponding relationship between amino acid sequences of two or more kinds of proteins, and a method therefor will be described in detail below at explanation of each step.

Then, the present invention will be explained by referring to the drawings. Fig. 1 is a flowchart that shows one example of a method for constructing the steric structure of GPCRS of the present invention.

First, at the step 10, an amino acid sequence of a desired protein (sequence) is inputted. At the step 20, a structure to be referred to is selected from database of steric structures, and alignment (juxtaposition) with an amino acid sequence of the selected reference protein is performed. At the step 30, based on information of the alignment, a coordinate with regard to a Cα atom which is one of the constituent atoms in the amino acid is obtained from the structure of the reference protein prescribed at the step 20, and an atomic coordinate of Cα is optimized so as to minimize an objective function by a simulated annealing method. At the step 40, other atoms of a main chain are added to an atomic coordinate of Cα at the step 30, followed by the optimization to minimize an objective function by a simulated annealing method. At the step 50, atoms of a side chain are added to an atomic coordinate of a main chain at the step 40, which is optimized so as to minimize an objective function by a simulated annealing method. Thus, the steric structure of GPCRs can be constructed with good precision.

Each step will be described in detail below.

Step 10: An amino acid sequence of a desired protein

First, an amino acid sequence of a desired protein having an unknown steric structure is inputted. As the amino acid sequence used, it is particularly preferable to use sequences registered in a database as GPCRs. These sequences can be obtained from GCRDb (The G-protein-coupled Receptor Database), the details of which are described in "An Internet review: the complete neuroscientist scours the World Wide Web "Bloom FE, Science 1996; 274 (5290): 1104-9:

http://www.gcrdb.uthscsa.edu/, GPCRDB: httD://www.gpcr.org/7tm/, ExPASy: http://www.expasy.ch/cgi-bin/sm-gpcr.pl, ORDB:

http://ycmi.med.yale.edu/senselab/ordb/ and the like.

Not only sequences registered in databases as GPCRs, but also any amino acid sequences of such as human (H. sapiens), drosophila (D. melanogaster), nematode (C. elagans), yeast (S. cerevisiae), and thalecress (A. thaliana) registered in databases such as GeneBank:

ftp://ncbi.nlm.nih.gov/genbank/genomes/. PIR: http://www-nbrf.georget own.edu/pir/ (National Biomedical Research Foundation(NBRF)), Swiss Plot: http://www.expasy.ch/sprot/sprot-top.html (Swiss Institute of Bioinform atics(SIB), European Bioinfomatics Institute(EBI)), TrEMBL (URL and the administrator are both the same as those of Swiss Plot), TrEMBLNEW (URL and the administrator are both the same as those of Swiss Plot), NAD: ftp://ftp.ddbj.nig.ac.ip (Japanese DNA databank) and the like can be used. These databases are merely an example, and any databases can be used as far as they are databases in which amino acid sequences of proteins are registered.

Step 20: Database retrieval and sequence alignment

After selecting a protein having a structure to be referred to from the steric structure database (Protein Data Bank) using a database of amino acid sequences of GPCRs as a profile, alignment (juxtaposition) is performed between an amino acid sequence of the selected reference protein and an amino acid sequence of the desired protein. As a structure to be referred to, the steric structure of rhodopsin (PDB ID:1F88) is preferable. Hereinafter, alignment using a database of amino acid sequences as a profile may be simply referred to as "profile alignment".

Herein, a "profile" adds information characterizing the position of a letter in a sequence to the letter. In the present invention, this means a matrix. This matrix is a round-robin combination of amino acids, and has 20*20 elements. This matrix also has meanings of information of the degree of amino acid conservation, the degree of susceptibility, and the similarity between amino acids.

As a software for performing profile alignment regarding an amino acid inputted at the step 10, it is preferable to use, for example, PSI-BLAST (Position-specific Iterated BLAST). PSI-BLAST is programmed so as to perform profile alignment. Details of PSI-BLAST are described in "Matching a protein sequence against a collection of PSI-BLAST-constructed position-specific score matrices." Schaffer AA, Wolf YI, Ponting CP, Koonin EV, Aravind L and Altschul SF, Bioinformatics 1999, 12, 1000-11.

Examples of an amino acid of GPCRs used as a profile include the aforementioned GCRDb, GPCRDB, ExPASy and ORDB which are public databases. These databases are used alone or as a combination thereof. Amino acid sequences of a database used as a profile is not particularly limited, but it is more preferable to use all sequences of a database used.

PSI-BLAST for performing profile alignment can be said to be a tool having the highest performance at present, for detecting a similarity of sequences. This program extracts information only from the alignment relation with significance in a database of a profile (herein, in GCRDb), and produces a site-specific score matrix of an amino acid. Then, in the interior of a program, a sequence having a high identity with a site-specific score matrix produced in place of the sequence of the desired protein is retrieved from a database, and the site-specific matrix is sequentially renewed every time until a more significant alignment is not detected. And, the identity between a final site-specific score matrix and a reference protein is calculated as the E value.

The E value quantitatively describes a random background noise present during matching between sequences. This also shows how much degree two sequences match, has the nature to decrease exponentially relative to a score, and is useful for a method of setting a significant threshold for the result. In the present invention, when the alignment has a reliability of 98% or more in general, preferably of 99% or more, it is suitable to judge that the desired protein is GPCRs and its steric structure should be so produced. In PSI-BLAST, this corresponds to when the E value statistically has a value of 0.1 or smaller in general, preferably of 0.01 or smaller.

Considering that GPCRs share the common nature of producing a disulfide bond at one position, it is preferred to perform re-alignment of GPCRs with covering all possible combinations of the disulfide bond at the position and with placing stress on the disulfide bond (SS bond) by managing parameters using a suitable alignment software, for example, the clustalW program.

As used herein, clustalW is an alignment program which enables to change a weighing function matrix and a gap penalty depending on a position on an amino acid sequence. In the present invention, since the disulfide bond is important for the steric structure as to GPCRs whose steric structures are the subject to be constructed, it is preferred to perform re-alignment after setting the weighing function such that the cysteine residue forming the disulfide bond in the alignment calculated by PSI-BLAST would match when the cysteine residue wouldn't match. Details of ClustalW are described in Thompson, J.D., Higgins, D.G. and Gibson, T.J. (1994) CLUSTAL W: Improving the sensitivity of progressive multiple sequence alignment through sequence weighting, positions-specific gap penalties and weight matrix choice. Nucleic Acids Research, 22: 4673-4680.

That is, in re-alignment, (i) a three-dimensional structure of a protein to be referred to is preferably, for example, the aforementioned 1F88 of PDB (Protein Data Bank) (PDB ID:1F88), provided that 1F88 has an A chain and a B chain, and their coordinates have a great deficiency and are not complete, respectively. Then, it is preferable to perform modeling of the 1F88 structure using a modeling software, for example, FAMS (Journal of Molecular Graphics and Modeling 18, 258-272, 2000) and refer to the structure. (ii) A site of interest is a region of the desired protein corresponding to two parts, first site: YFVFGPTGC (SEQ ID NO. and second site: GWSRYIPEGMQCSCGIDYYTPHEETINNE (SEQ ID NO.2) which contains a cysteine residue. Outside of this is a secondary structure of an α helix and a β sheet in the 1F88 protein, and should not be moved . respecting the alignment of PSI-BLAST. In addition, (iii) an object is to effect the matched alignments where the desired protein corresponds to C (cysteine) of the first site and C of the second site, respectively. In the first site, a secondary structure follows immediately after C, and there is little degree of freedom for changing the alignment of PSI-BLAST.

Specific preferable procedures for re-alignment are as the following (1) to (6):

(1) Definition file of secondary structure part

First, a definition file for a secondary structure part of a reference protein, for example, the second site of 1F88: GWSRYIPEGMQCSCGIDYYTPHEETNNE (SEQ ID NO.2) is produced. This is for use in re-alignment for preventing a gap from entering into a secondary structure part as much as possible. Specifically, the definition file is the following form with the SwissProt file format as follows:

```
ID   IF88A STANDARD;    PRT;    28AA.
FT   STARND    13 17
SQ   SEQUENCE  28    AA;    000000    MW; XXXXXXXXXXXXXXXX CRC64;
     GWSRYIPEGM      QXSCGIDYYT PHEETNNE
```

(2) Excision of a part of interest from an original alignment file

Then, the original alignment file is read, and the sequence of the desired protein corresponding to the second site of the reference protein 1F88: GWSRYIPEGMQCSCGIDYYTPHEETNNE (SEQ ID NO.2) is written out (excised). As described in the above (iii), since a secondary structure follows immediately after C (cysteine) in the first site, and h there is little degree of freedom for changing the alignment of PSI-BLAST, only a sequence regarding a part corresponding to the second site is excised. Upon this, a file is produced in which a position of an excised part is recorded.

(3) Variation of a part of interest corresponding to a plurality of cysteines (Cys)

When a part of interest of the desired protein contains a plurality of Cys, for attempting as a case study, the alignment in which Cys of the reference protein are definitely corresponded to respective Cys, only one Cys of the desired protein is left as it is, and the remaining Cys are masked with X to generate sequences of a part of interest by the number of corresponding Cys.

(4) Re-alignment only of a part of interest by an alignment program

Regarding short parts produced in the above (1) and (2), alignment for avoiding a gap in a secondary structure is performed paying serious attention to a SS bond. Specifically, for example, the following parameters are imparted to clustalW.

| General gap opening penalty | 2.0 |
|---|---|
| Helix part gap opening penalty | 9.0 |
| Strand part gap opening penalty | 9.0 |
| General gap extension penalty | 2.0 |

Elements of a mutation matrix are as follows, for example, paying serious attention to a SS bond.

| Consistency of Cys-Cys | 99 |
|---|---|
| Consistency of Trp-Trp | 16 |
| Consistency in general residues | 4 |
| Between Glu-Gln | 3 |
| Between Asp-Aspn | 3 |
| Between Arg-Lys | 3 |
| Other relationship | 0 |

(5) Incorporation of a re-aligned part of interest into a whole alignment

Incorporation of a re-aligned part of interest into a whole alignment is performed by putting the re-alignment of a part of interest obtained in (4) into the original broad alignment of PSI-BLAST, by referring to an excised place recorded in (2) and (3). Upon this, the parts masked with X (C of GMQCS of a second site of a reference protein and, when a part of interest of the desired protein contains a plurality of Cys, Cys situated at a place other than the relevant Cys) are returned to the original sequence.

(6) Checking of a produced alignment

(6-1) Checking of a SS bond

In a process of the above (4), a sequence is given paying serious attention to a SS bond, but this is relative. Here, only the alignment having effected the of consistency of Cys-Cys is extracted.

(6-2) Checking of uniqueness

An alignment file starting from the above (2) and (3) may be consistent with the original alignment of (2) in some cases. In addition, the file may be consistent with the already modeled alignment file in some cases. Modeling by the same alignment file leaves only a unique file in order to save an amount of calculation since a great number of modeling is required at present.

(6-3) Checking of preservation

For caution's sake, it is checked that a sequence of the desired protein of the original alignment file and that of a reference protein are consistent except for a gap. This is for preventing incorrect data due to an operation mistake from being supplied to modeling.

By the aforementioned procedures, a re-alignment is produced in view of the common characteristic of GPCRs of making a disulfide bond at one place.

Based on the above-mentioned alignment information, construction of an atomic coordinate defining the steric structure, that is, production of the steric structure is performed. A method of producing the steric structure is not particularly limited, but for example, it is preferable to construct an atomic coordinate by a method of the following steps 30 to 50. In addition, calculation times, a constant, a cutoff value and the like described in explanation of the following steps 30 to 50 show one example of parameters which are thought to be most preferable by the present inventors, and this does not limit the scope of the present invention at all.

Step 30: Construction and optimization of an initial coordinate of a Cα atom

Receiving the results of the alignment from the step 20, information about the inserted and defective amino acid residues is obtained from a reference protein. A region having no gap in which consecutive three or more residues of amino acids are corresponding in the alignment is selected and, in this region, the same Cα atom as that of the reference protein is put into the desired protein in these residue pairs. When the Cα atom has not been obtained, a coordinate is adapted from a database of a pre-made fragment (see Fig.2). As used herein, a Cα atom means a carbon atom which is a center of a skeleton of each amino acid. A Cβ atom means a carbon atom binding to a side chain side of a Cα atom. In addition, a C atom means a carbon atom of a carbonyl group.

Step 31: Construction of a Cα atom by a simulated annealing method

The Cα atom produced in the above-mentioned step 30 is optimized using a function constructed of coordinates of a reference protein using a process of simulated annealing. This objective function is as described in the following equation (1):

$$U_{C\alpha} = U_{len} + U_{ang} + U_{pos} + U_{vdw} \tag{1}.$$

Herein, Ulen relates to a distance between Cα atoms of a pair of adjacent residue and Cys residue on a sequence, and is set as in the following equation (2):

$$U_{len} = K_l \sum_i (D_{i,i+1} - 3.8)^2 + K_{ss} \sum_i (D_i^{ss} - 5.4)^2 \quad \dots (2).$$

Herein, Di, i+1 is a distance between Cαs of a residue i and a residue i+1. DiSS is a distance between pairs of Cys residues forming a disulfide bond. K1 and KSS are constants, being set at 2 and 5, respectively.

Uang is a function as a binding angle of a $C_\alpha$ atom, and is as in the following equation (3):

$$U_{ang} = K_a \sum_i (\theta_i - \theta_0)^2 \qquad \ldots (3)$$

Herein, $\theta i$ (rad) is an angle of an $i^{th}$, $i+1^{th}$ or $1+2^{th}$ residue $C\alpha$ atom. $\theta_0$ is set at $(100/180)\cdot\pi$(rad) from the X-ray structure. Ka is a constant and is set at 1.

Upos is a function regarding a position of a $C\alpha$ atom, and is as in the following equation (4):

$$U_{pos} = K_{pos} \sum_i \frac{1}{M_i} \left\| x_i - \langle w_i x_i \rangle \right\|^2 \qquad \ldots (4).$$

Herein, $\|\cdot\|$ means norm, and Mi is an average distance between $C\alpha$ atoms situated at structurally equivalent positions on an alignment based on a structure. When a value of Mi can not be obtained for a residue i, a value of Mi is set at 10. Herein, it is an average coordinate of a $C\alpha$ atom, and is as in the following equation (5):

$$\langle w_i x_i \rangle = \frac{1}{W} \sum_j w_i^j x_i^j \qquad \ldots (5).$$

Herein, Xji is an atomic coordinate of $C_\alpha$ of an $i^{th}$ residue of a $j^{th}$ reference protein. Herein, wji is a weight of an $i^{th}$ residue of a $j^{th}$ reference protein. This weight is an important parameter for determining an approximate shape of the desired protein, and this is determined by a local value in the spatial vicinity within 12Å of an attentioned part called Local Space Homology (LSH) (see Fig.3). A correlation between LSH and a ratio of residue pairs present in a part in which a structure is well conserved (SCRs: Structural Conserved Regions) is very high as shown in Fig.4. This means that, when the LSH value is high, a position of a $C\alpha$ atom is statistically within 1 .0Å as compared with a reference protein structure.

Uvdw is as in the following equation (6):

$$U_{vdw} = K_{vdw} \sum_{i,j(>i+2)} \left\{ \left( \frac{3.8}{D_{i,j}} \right)^{12} - \left( \frac{3.8}{D_{i,j}} \right)^6 \right\} \qquad \ldots (6).$$

Herein, Kvdw is set at 0.01 (Di,j<3.2Å) and 0.001 (Di,j>3.2Å), and has a cutoff value of 6Å.

A $C\alpha$ atom is optimized using a simulated annealing method according to the equation (1). At this optimization stage, perturbation of a $C\alpha$ atom is set to be within 1.0Å. In addition, this annealing stage is calculated every 100 times regarding all $C\alpha$ atoms. And, a parameter corresponding to a temperature is reduced from 25 by 0.01 every 0.5 time, and the parameter is constant thereafter.

These great two stages, obtaining of structural information and construction of a $C_\alpha$ atom are repeated 10 times, and a coordinate of a $C_\alpha$ atom having the minimum objective function value is calculated as an optimum solution.

Step 40: Construction and optimization of a main chain atomic coordinate

Other atoms of a main chain are added to an atomic coordinate of $C\alpha$ at the step 30, and an objective function is minimized by a simulated annealing method. First, steric overlapping of $C\alpha$ atoms is performed, and residues having a distance between Cα atoms of 2.5Å or smaller are taken. Atomic coordinates of a main chain except for Cα are obtained from a coordinate of a reference protein so that a distance between Cα atoms becomes minimum and, thus, a model structure is obtained.

When there is no corresponding residue in a reference protein, atomic coordinates of a main chain are produced from a corresponding protein fragment of four residues in a database. During this process, main chain atoms of a residue i are selected from a residue having the minimum rmsd value between i-1th to i+2th Cα atoms. Upon this, at a N-terminal residue, a range of overlapping of Cα atom coordinates is from ith to i+3th and, at a C-terminal residue and a residue one before, the ranges are from i-3th to ith and from j-2th to i+1th, similarly.

Based on an objective function of main chain atoms, optimization is performed by a simulated annealing method.

An objective function is as in the following equation (7):

$$U_{main} = U_{bond} + U_{ang} + U_{non\text{-}bond} + U_{ss} + U_{pos} + U_{tor} + U_{chi} + U_{hydr} \tag{7}$$

Ubond is as in the following equation (8):

$$U_{bond} = K_b \sum_i (b_i - b_i^0)^2 \qquad \ldots (8).$$

Herein, bi0 is a standard bond length, and is different depending on a kind of each chemical bond. Kb is a constant, and is set at 225.

Uang is a function of a bonding angle, and is as in the following equation (9):

$$U_{ang} = K_a \sum_i (\theta_i - \theta_i^0)^2 \qquad \ldots (9).$$

Herein, θi is an ith bonding angle, and is different depending on a kind of a chemical bond. Ka is a constant, and is set at 45.

Unon-bond is a function of the non-bond interaction, and is as in the following equation (10):

$$U_{non\text{-}bond} = K_{non} \sum_{i,j} \varepsilon_{i,j} \left\{ \left( \frac{r_{i,j}^\star}{r_{i,j}} \right)^{12} - 2 \left( \frac{r_{i,j}^\star}{r_{i,j}} \right)^6 \right\} \qquad \ldots (10)$$

Herein, εi,j and ri,j * are constants, and are different depending on a kind of an atom.

Knon is a constant and is set at 0.25, and has cutoff of 8Å.

USS is a function of a disulfide bond produced by the Cys residue, and is as in the following equation (11):

$$U_{ss} = \sum_i \left\{ K_{C\alpha}^{ss} (D_i^{C\alpha} - 5.4)^2 + K_{C\beta}^{ss} (D_i^{C\beta} - 3.8)^2 \right\} \qquad \ldots (11).$$

Herein, KSSCα and KSSCβ are constants, and are set at 7.5.

Upos is a function regarding a position of an atom, and is as in the following equation (12):

$$U_{pos} = K_{pos} \sum_i \frac{1}{M_i} \left\| x_i - \langle w_i x_i \rangle \right\|^2 \qquad \ldots (12).$$

Herein, <WiXi> is given as in the following equation (13):

$$\langle w_i x_i \rangle = \frac{1}{W} \sum_j w_i^j x_i^j \qquad \ldots (13).$$

<WiXi> in the equation (12) is obtained from overlapping of structures between the desired protein and the reference protein.

Kpos is a constant, and is set at 0.3.

Utor is related to a helix angle of a main chain, and is as in the following equation (14):

$$U_{tor} = K_t \sum_i \sqrt{(\varphi_i - \varphi_i^0)^2 + (\psi_i - \psi_i^0)^2} + K_\omega \sum_i (\omega_i - \omega_i^0)^2 \qquad \ldots (14).$$

Herein, $\phi_i 0$ and $\varphi_i 0$ are let to be nearest helix angles $\theta_i$ and $\varphi_i$ on the Ramachandran map. In addition, $\omega_i 0$ is let to be 0 and, only in the case of cis-Pro residue, is let to be $\pi$(radian). Kt and $K\omega$ are constants, and are let to be 10 and 50, respectively.

Uchi is related to chirality of $C\alpha$, and is as in the following equation (15):

$$U_{chi} = K_{chi} \sum_i \left( \tau_i + \frac{2}{3}\pi \right)^2 \qquad \ldots (15).$$

Herein, $\pi_i$ is a helix angle defined by N-$C\alpha$-C $\beta$-C and Kchi is let to be 50.

Uhydr is related to a hydrogen bond of a main chain conserved in homologous proteins, and is determined as in the following equation (16):

$$U_{hydr} = K_{hydr} \sum_{i,j} (D_{i,j}^{N-O} - 2.9)^2 \qquad \ldots (16).$$

A hydrogen bond is set when a distance between a N atom and a O atom is 2.9±0.5 Å.

Upon determining whether a hydrogen bond is present in a reference protein, when it is recognized that 75% or more of reference proteins are present, it is determined that a hydrogen bond is present. Khydr is a constant, and is let to be 0.6.

Optimization of main chain atoms including C$\beta$ is performed by simulated annealing. During this annealing process, perturbation of atoms of a main chain and C$\beta$ is let to be within 1.0Å relative to initial positions. This annealing stage is performed 200 times on atoms of a main chain and C$\beta$. A parameter corresponding to a temperature starts from 50 or 25, is let to be 0.5-fold every time, and this is continued until 0.01 and, thereafter, is let to be a constant value.

In order to widely perform sampling of a steric arrangement of a main chain, according to the method of the present invention, the above-mentioned method is performed 6 times, and atomic coordinates of a main chain having the minimum objective function value are adopted as an optimum solution. And, a parameter corresponding to a temperature starts from 50 at first two times, and starts from 25 from third time.

Step 50: Construction and optimization of side chain atomic coordinates

Construction of a side chain is roughly classified into two stages: "construction of a side chain at a structure conserved part" (step 51) and "construction of a total side chain" (step 52).

Step 51: Construction of a side chain at a structure conserved part

Regarding calculated main chain atoms, a helix angle of a side chain is obtained from homologous proteins using the method in the previous study. Details of this method are described in "The role of played by environmental residues in side-chain torsional angles within homologous families of proteins: A new method of side chain modeling. " Ogata K and Umeyama H, Prot.Struct. Funct. Genet. 1998, 31, 255-369.

A ratio of side chains conserved in homologous proteins is calculated in this method, and, based on this information, modeling of a side chain is performed. Atomic coordinates of a side chain at a conserved site of a side chain are placed on main chain atoms. For example, when a $\chi 1$ angle of an arginine residue is conserved in homologous proteins, a coordinate of a C$\gamma$ atom can be placed and, when $_\chi 1$ and $_\chi 2$ angles are conserved in a Phe residue, all side chain atoms can be placed. A process of optimization of simulated annealing using the equation (7) is such that only atoms of a main chain and C$\beta$ are performed and perturbation of atoms is within 1.0Å. This stage of annealing atoms of a main chain and C$\beta$ are performed 200 times. And, a parameter corresponding to a temperature starts from 25, is let to be 0.5-fold every time, and is reduced until 0:01. Unon-bond in the equation (7) is performed on main chain atoms and partially produced side chain atoms. Upon this, coordinates of side chain atoms are rendered conserved during an optimization process.

Mi and a pair of N-O in a hydrogen bond which are information of a structure are used in an optimization process. In order to obtain arrangement of main chain atoms, the above-mentioned process is repeated three times, and coordinates of minimum main chain atoms of an objective function are adopted as a calculated structure.

Step 52: Construction of a total side chain

Construction of a side chain is performed under fixed main chain and C$\beta$ atoms. This is performed by the study results disclosed in the above-mentioned Ogata K and Umeyama H, Prot. Struct. Funct. Genet. 1998, 31, 255-369 and, by using this, a precise model can be given in a short time. Then, a main chain structure is optimized by a Monte Carlo method at a low temperature, a temperature is set at 0.001 and, by using an objective function Unon-bond of the equation (7), calculation is performed at all main chain and side chain atoms. And, during optimization of N, C$\alpha$, C and C$\beta$ atoms, coordinates of a side chain are re-arranged so as to retain a helix angle of a side chain at the optimized state. Perturbation of atoms is let to be within 0.5 Å. Then, a side chain is deleted, and the above-mentioned construction of a side chain is repeated. This process is repeated until collision of 2.4Å atoms is diminished, and a helix angle of N-C$\alpha$-C$\beta$-C becomes within a range of - 120$\pm$15°

Step 60: Construction of a final structure

Thus, by performing steps 10 to 50, atomic coordinates defining the steric structure of GPCRs can be obtained. The resulting atomic coordinates can be appropriately used for designing drug molecules such as medicines and agricultural chemicals described later, such as by producing the steric structure model of GPCR by a suitable program which can display the steric structure, for example, BIOCES (manufactured by NEC Corporation) and the like, and by assessing this and the bonding state with various ligands (drug molecules).

Each step regarding the aforementioned construction of the steric structure can be performed by using modeling software "FAMS" developed by the present inventors. Thereby, the steric structure can be constructed with excellent precision. Herein, details of FAMS are described in Koji Ogata and Hideaki Umeyama, Journal of Molecular Graphics and Modeling 18, 258-272, 2000.

Thus, atomic coordinates defining the steric structure of an arbitrary 7-transmembrane G-protein-coupled receptor can be obtained. Herein, an atomic coordinate describes the steric structure on a three-dimensional space. That is a relative distance in mutually perpendicular three directions letting a point on a space to be an origin, and is a vector consisting of three numerals per one atom except for a hydrogen atom present in a protein.

By containment of the resulting atomic coordinates in a suitable recording medium in a prescribed computer utilizable format, a database of the steric structure of a 7-transmembrane G-protein-coupled receptor can be constructed. The database of the present invention preferably contains, for example, information of alignment between a reference protein and a desired protein together with the above-mentioned atomic coordinates. In addition, in the present invention, a database means a computer system that writes the above-mentioned atomic coordinates in a suitable recording medium, and performs retrieval according to a prescribed program. Herein, examples of a suitable recording medium include magnetic media such as floppy disc, hard disk, magnetic tape and the like; optical disc, semiconductor memory and the like such as CD-ROM, MO, CD-R, CD-RW and the like.

According to another aspect of the present invention, there is provided a method for presuming the function of a protein, which comprises selecting the steric structure to be referred to from the known steric structure of a 7-transmembrane G-protein-coupled receptor using an amino acid sequence database of the receptor as a profile, performing alignment of a desired protein having the unknown function and, when the steric structure of the desired protein can be produced, judging that the desired protein is a 7-transmembrane G-protein-coupled receptor.

In the present invention, the steric structure of the desired protein having the unknown function is constructed by the above-mentioned method and, when construction is possible, it is judged that the desired protein is GPCRs. In the present invention, preferably, alignment is performed by selecting the steric structure to be referred to from a database of the known steric structure of a G-protein-coupled receptor, and aligning an amino acid sequence of the selected reference protein and an amino acid sequence of the desired protein, an amino acid sequence database used as a profile is selected from the group consisting of GCRDb, GPCRDB, ExPASy or ORDP and, when alignment has reliability of 98% or more, it is determined that the steric structure of the desired protein can be produced. The thus obtained atomic coordinates defining the steric structure of GPCRs are included in the database of the present invention.

Then, a method for designing a drug molecule using an atomic coordinate obtained by the present method will be described.

By using all or a part of atomic coordinates defining the steric structure (hereinafter, this is abbreviated as "steric structure coordinate") of GPCR which is a target of a drug molecule obtained by the above-mentioned method (hereinafter, this is referred to as "target GPCR"), or a database in which they are recorded, or atomic coordinates contained in a recording medium, it is possible to perform identification, retrieval, assessment or design of a drug molecule which interacts with the target GPCR (antagonist or agonist).

Identification, retrieval, assessment or design of a drug molecule is performed based on the presence or the absence and a degree of interaction between the steric structure coordinate of GPCR obtained by the present method and the steric structure coordinate of a drug molecule, in a computer in which a suitable program that can design a drug molecule operates. In the present specification, identification, retrieval, assessment, design and the like of a drug molecule is simply referred to as drug molecule design.

A computer used upon molecule design based on interaction between the steric structure coordinate of GPCR and the steric structure coordinate of a drug candidate molecule is not particularly limited as far as it is a computer that is adjusted so as to operate a suitable program. In addition, a recording medium of a computer is not particularly limited. Examples of a program used in molecular design include a computer program Insight ll and the like manufactured by Molecular Simulation. In particular, by using programs such as Ludi and DOCK which are especially produced for this purpose alone or in a combination thereof, a drug molecule can be more easily identified, retrieved, assessed, or designed. In addition, assessment of docking between the steric structure coordinate of GPCR and a drug molecule can be performed, for example, by using BIOCES (version 3.10) manufactured by NEC Corporation.

Herein, whether a drug molecule is a known drug molecule or a newly synthesized drug molecule having a novel chemical structure, any drug molecules can be used in the present method as far as the steric structure thereof can be obtained. The steric structure coordinate of a drug molecule may be a coordinate obtained by any method such as X-ray crystal analysis, modeling and the like. Determined steric structure coordinates can be obtained from a suitable database, for example, CCDC (Cambridge Crystallographic Data Centre: http://www.ccdc.cam.ac.uk/) and PDB (Protein Data Bank: http://www.rcsb.org/pdb/).

Further, a drug molecule can be designed also by the method described in Japanese Patent Application Laid-Open 2000-178209. Likewise, by using the steric structure coordinate of GPCR obtained by the present method, it becomes possible to design a drug molecule by a computer, provided that the molecular designing method of the present invention is not limited to methods using these programs and procedures.

There are usually conceptionally two stages in design of a drug molecule. The first stage is to find out a lead compound, and the next stage is to optimize the lead compound. Any of the stages can be performed by using the steric structure coordinate of target GPCR by a known per se method. Thereby, an optimum medicine and agricultural chemical candidate molecule can be obtained. The thus obtained drug molecule is included in the scope of the present invention.

Medicine and agricultural chemical candidate molecules identified, retrieved, assessed or designed by the above-mentioned method can be obtained, for example, by a known per se chemical synthesizing method depending on the natures of the molecules. However, a drug molecule may be a natural compound or a synthetic compound, or may be a high-molecular compound or a low-molecular compound. The obtained medicine and agricultural chemical candidate molecules are further examined for their activity and safety by a known per se method, or by an in vitro or in vivo pharmacological or physiological test, and medicine and agricultural chemical candidate molecules having the desired nature, that is, the activity and the safety necessary as a medicine or an agricultural chemical are selected (screening), whereby, molecules which can be actually applied as a medicine or an agricultural chemical can be obtained.

Medicines or agricultural chemicals, for example, medicine molecules selected by the above-mentioned screening method can be administered alone as they are to a patient with disease which is a subject of treatment, but can be also administered by mixing 2 or more active ingredients. In addition, it is preferable to prepare the substance into a pharmaceutical composition using a pharmacologically acceptable additive for pharmacy and administer the composition. For example, the substance may be used orally as sugar-coated tablets, capsules, granules, fine granules, powders, pills, microcapsules, liposome preparations, troches, sublingual agents, solutions, elixirs, emulsions, suspensions or the like, or parenterally as injectables prepared as sterile aqueous solutions or oily solutions, suppositories, ointments, patches or the like, as necessary. These may be prepared, for example, by kneading the substance together with'physiologically acceptable carrier, flavor, excipient, vehicle, preservative, stabilizer and binder in a unit dosage form required for implementing generally recognized preparations, by using the well-known methods in the art such as filling and compression. An amount of an active ingredient in these pharmaceutical compositions allows a suitable dose in an indicated range to be obtained.

When agricultural chemical molecules are actually used as an agricultural chemical, they are used by mixing with agriculturally or horticulturally acceptable carrier or diluent, additive and supplementing agent by the known method, and preparing into preparation forms (composition) which are usually used as an agricultural chemical, for example, powders, granules, hydrated agents, emulsions, water-soluble agents, flowables and the like.

Examples

The present invention will be further specifically explained by way of Examples below, but the following Examples should be regarded as helping obtain specific recognition of the present invention, and do not limit the scope of the present invention whatsoever.

Example 1 Construction of the steric structure of a β2 adrenaline receptor

According to the method described in detail in the aforementioned embodiments of the present invention, the steric structure model of a human-derived β2 adrenaline receptor was constructed, and a position into which an agonist (isoproterenol) is placed and a position into which an antagonist (propranolol) is placed were compared as follows:

Construction of the steric structure model was performed using workstation manufactured by NEC Corporation (Model:
Express5800/120Rc-2, CPU:PentiumIII 933MHz x 2, OS: Red Hat Linux 6.2J, memory: 1024 Mbytes). An amino acid sequence of the desired β2 adrenalin receptor was obtained from PIR;
http://www-nbrf.georgetown.edu/pir/ID:QRHUB2. The amino acid sequence is shown in SEQ ID NO:3.

Letting an amino acid sequence of this β2 adrenaline receptor to be a sequence of the desired protein, alignment by PSI-BLAST was performed. Upon this, the full sequence 892 of GCRDb; http://www.gcrdb.uthscsa.edu/ was used as a motif profile. And, by using a structure of a B chain of PDB (http://www.rcsb.org/pdb/) ID:IF88 (rhodopsin) as the steric structure of a reference protein, alignment to this B chain was obtained. There was a dimer having the approximately same steric structure composed of an A chain and a B chain in a crystal lattice of 1F88 (rhodopsin), and the B chain was used as a structure to be referred to. In addition, there was a great defect in coordinates of the A chain and the B chain, respectively, being not complete. Modeling of the 1F88 structure was performed using modeling software "FAMS", and that structure was referred to. An amino acid sequence of this 1F88 (rhodopsin) B chain is shown in SEQ ID NO.4.

As a result of the above-mentioned alignment, the E value calculated by PSI-BLAST was 1e-144 (minus 144 power of 10).

As described above, re-alignment was performed using a program of clutalW, paying serious attention to a disulfide bond. The resulting alignment information is shown in Fig.5. In Fig.5, an upper sequence denotes QRHUB2 (β adrenaline receptor), and a lower sequence denotes 1F88 (rhodopsin). As apparent from Fig.5, homology between QRHUB2 and 1F88 was 19.6%.

Based on the thus obtained alignment information, using the modeling software "FAMS" developed by the present inventors, construction and optimization of an initial coordinate of a Cα atom, construction and optimization of a main chain atom coordinate, and construction of a total side chain were performed as in the above-mentioned steps 30 to 50, to obtain atomic coordinates defining the steric structure of QRHUB2.

The resulting atomic coordinates are shown in Table 1. Table 1 describes atomic coordinates according to the generally used format of Protein Data Bank (PDB) which is a method of expressing the steric structure (three-dimensional structure) coordinate of a protein. Table 1 describes a three-dimensional coordinate of each atom at 30th line and thereafter except the last line. ATOM on a first column indicates that this row is a row of an atomic coordinate, a second column denotes an order of the atom, a third column denotes discrimination of atoms in an amino acid residue, a fourth column denotes an amino acid residue, a fifth column denotes an amino acid number corresponding to SEQ ID No.3 (corresponding to amino acid numbers 10 to 238, 264 to 363 in SEQ ID No.3), sixth, seventh and eighth columns denote a coordinate of the atom (in an order of $\alpha$-axis, b-axis and c-axis directions, in Å unit), a ninth column denotes a rate of occupation of the atom, and a tenth column denotes a temperature factor of the atom. The last line denotes the last line in this table. A part of amino acid numbers 239 to 263 in SEQ ID No.3 is an insertion sequence part for which a structure to be referred to can not be obtained. In addition, since a rate of occupation and a temperature factor of an atom can not be calculated, "0.00" are placed into ninth and tenth columns.

## Table 1 Atomic coordinate of β2 adrenaline receptor

```
HEADER      mode                                    05-SEP-00    mode

AUTHOR      FAMS

SEQRES   1   329   PHE LEU LEU ALA PRO ASN ARG SER HIS ALA PRO ASP HIS

SEQRES   2   329   ASP VAL THR GLN GLN ARG ASP GLU VAL TRP VAL VAL GLY

SEQRES   3   329   MET GLY ILE VAL MET SER LEU ILE VAL LEU ALA ILE VAL

SEQRES   4   329   PHE GLY ASN VAL LEU VAL ILE THR ALA ILE ALA LYS PHE

SEQRES   5   329   GLU ARG LEU GLN THR VAL THR ASN TYR PHE ILE THR SER

SEQRES   6   329   LEU ALA CYS ALA ASP LEU VAL MET GLY LEU ALA VAL VAL
```

SEQRES    7  329   PRO PHE GLY ALA ALA HIS ILE LEU MET LYS MET TRP THR

SEQRES    8  329   PHE GLY ASN PHE TRP CYS GLU PHE TRP THR SER ILE ASP

SEQRES    9  329   VAL LEU CYS VAL THR ALA SER ILE GLU THR LEU CYS VAL

SEQRES   10  329   ILE ALA VAL ASP ARG TYR PHE ALA ILE THR SER PRO PHE

SEQRES   11  329   LYS TYR GLN SER LEU LEU THR LYS ASN LYS ALA ARG VAL

SEQRES   12  329   ILE ILE LEU MET VAL TRP ILE VAL SER GLY LEU THR SER

SEQRES   13  329   PHE LEU PRO ILE GLN MET HIS TRP TYR ARG ALA THR HIS

SEQRES   14  329   GLN GLU ALA ILE ASN CYS TYR ALA ASN GLU THR CYS CYS

SEQRES   15  329   ASP PHE PHE THR ASN GLN ALA TYR ALA ILE ALA SER SER

SEQRES   16  329   ILE VAL SER PHE TYR VAL PRO LEU VAL ILE MET VAL PHE

SEQRES   17  329   VAL TYR SER ARG VAL PHE GLN GLU ALA LYS ARG GLN LEU

SEQRES   18  329   GLN LYS ILE ASP LYS SER GLU GLY PHE CYS LEU LYS GLU

SEQRES   19  329   HIS LYS ALA LEU LYS THR LEU GLY ILE ILE MET GLY THR

SEQRES   20  329   PHE THR LEU CYS TRP LEU PRO PHE PHE ILE VAL ASN ILE

SEQRES   21  329   VAL HIS VAL ILE GLN ASP ASN LEU ILE ARG LYS GLU VAL

SEQRES   22  329   TYR ILE LEU LEU ASN TRP ILE GLY TYR VAL ASN SER GLY

SEQRES   23  329   PHE ASN PRO LEU ILE TYR CYS ARG SER PRO ASP PHE ARG

SEQRES   24  329   ILE ALA PHE GLN GLU LEU LEU CYS LEU ARG ARG SER SER

SEQRES   25  329   LEU LYS ALA TYR GLY ASN GLY TYR SER SER ASN GLY ASN

SEQRES   26  329   THR GLY GLU GLN

SSBOND    1 CYS     97    CYS    175

ATOM      1   N   PHE    1      43.073    0.386  -20.078   0.00   0.00

ATOM      2   CA  PHE    1      41.753   -0.201  -20.163   0.00   0.00

ATOM      3   C   PHE    1      41.414   -0.419  -21.636   0.00   0.00

ATOM      4   O   PHE    1      41.225    0.577  -22.359   0.00   0.00

| ATOM | 5 | CB | PHE | 1 | 40.766 | 0.713 | -19.432 | 0.00 | 0.00 |
| ATOM | 6 | CG | PHE | 1 | 39.626 | -0.008 | -18.812 | 0.00 | 0.00 |
| ATOM | 7 | CD1 | PHE | 1 | 38.307 | 0.247 | -19.185 | 0.00 | 0.00 |
| ATOM | 8 | CD2 | PHE | 1 | 39.869 | -0.865 | -17.741 | 0.00 | 0.00 |
| ATOM | 9 | CE1 | PHE | 1 | 37.253 | -0.332 | -18.502 | 0.00 | 0.00 |
| ATOM | 10 | CE2 | PHE | 1 | 38.817 | -1.448 | -17.050 | 0.00 | 0.00 |
| ATOM | 11 | CZ | PHE | 1 | 37.506 | -1.180 | -17.431 | 0.00 | 0.00 |
| ATOM | 12 | N | LEU | 2 | 40.955 | -1.616 | -21.879 | 0.00 | 0.00 |
| ATOM | 13 | CA | LEU | 2 | 40.600 | -2.066 | -23.178 | 0.00 | 0.00 |
| ATOM | 14 | C | LEU | 2 | 39.522 | -1.130 | -23.782 | 0.00 | 0.00 |
| ATOM | 15 | O | LEU | 2 | 38.456 | -0.903 | -23.189 | 0.00 | 0.00 |
| ATOM | 16 | CB | LEU | 2 | 40.177 | -3.558 | -22.990 | 0.00 | 0.00 |
| ATOM | 17 | CG | LEU | 2 | 40.585 | -4.520 | -24.108 | 0.00 | 0.00 |
| ATOM | 18 | CD1 | LEU | 2 | 39.574 | -5.662 | -24.157 | 0.00 | 0.00 |
| ATOM | 19 | CD2 | LEU | 2 | 40.661 | -3.884 | -25.485 | 0.00 | 0.00 |
| ATOM | 20 | N | LEU | 3 | 39.652 | -1.018 | -25.078 | 0.00 | 0.00 |
| ATOM | 21 | CA | LEU | 3 | 38.777 | -0.250 | -25.976 | 0.00 | 0.00 |
| ATOM | 22 | C | LEU | 3 | 37.698 | -1.167 | -26.669 | 0.00 | 0.00 |
| ATOM | 23 | O | LEU | 3 | 37.353 | -0.941 | -27.843 | 0.00 | 0.00 |
| ATOM | 24 | CB | LEU | 3 | 39.720 | 0.447 | -26.998 | 0.00 | 0.00 |
| ATOM | 25 | CG | LEU | 3 | 39.142 | 1.650 | -27.822 | 0.00 | 0.00 |
| ATOM | 26 | CD1 | LEU | 3 | 39.404 | 1.543 | -29.337 | 0.00 | 0.00 |
| ATOM | 27 | CD2 | LEU | 3 | 37.641 | 1.886 | -27.688 | 0.00 | 0.00 |
| ATOM | 28 | N | ALA | 4 | 37.400 | -2.343 | -26.054 | 0.00 | 0.00 |
| ATOM | 29 | CA | ALA | 4 | 36.477 | -3.272 | -26.597 | 0.00 | 0.00 |

17

| ATOM | 30 | C | ALA | 4 | 35.117 | -2.525 | -26.707 | 0.00 | 0.00 |
|------|----|----|----|----|--------|--------|---------|------|------|
| ATOM | 31 | O | ALA | 4 | 34.699 | -2.282 | -27.891 | 0.00 | 0.00 |
| ATOM | 32 | CB | ALA | 4 | 36.410 | -4.512 | -25.679 | 0.00 | 0.00 |
| ATOM | 33 | N | PRO | 5 | 34.358 | -2.111 | -25.644 | 0.00 | 0.00 |
| ATOM | 34 | CA | PRO | 5 | 33.084 | -1.488 | -25.861 | 0.00 | 0.00 |
| ATOM | 35 | C | PRO | 5 | 33.311 | -0.006 | -26.324 | 0.00 | 0.00 |
| ATOM | 36 | O | PRO | 5 | 32.302 | 0.657 | -26.536 | 0.00 | 0.00 |
| ATOM | 37 | CB | PRO | 5 | 32.239 | -1.431 | -24.582 | 0.00 | 0.00 |
| ATOM | 38 | CG | PRO | 5 | 33.171 | -2.182 | -23.598 | 0.00 | 0.00 |
| ATOM | 39 | CD | PRO | 5 | 34.594 | -2.101 | -24.154 | 0.00 | 0.00 |
| ATOM | 40 | N | ASN | 6 | 34.520 | 0.564 | -26.479 | 0.00 | 0.00 |
| ATOM | 41 | CA | ASN | 6 | 34.653 | 1.953 | -27.011 | 0.00 | 0.00 |
| ATOM | 42 | C | ASN | 6 | 34.068 | 3.074 | -26.086 | 0.00 | 0.00 |
| ATOM | 43 | O | ASN | 6 | 33.839 | 4.201 | -26.540 | 0.00 | 0.00 |
| ATOM | 44 | CB | ASN | 6 | 34.000 | 2.124 | -28.366 | 0.00 | 0.00 |
| ATOM | 45 | CG | ASN | 6 | 33.779 | 1.138 | -29.437 | 0.00 | 0.00 |
| ATOM | 46 | OD1 | ASN | 6 | 32.786 | 1.165 | -30.245 | 0.00 | 0.00 |
| ATOM | 47 | ND2 | ASN | 6 | 34.585 | 0.044 | -29.583 | 0.00 | 0.00 |
| ATOM | 48 | N | ARG | 7 | 34.407 | 2.920 | -24.839 | 0.00 | 0.00 |
| ATOM | 49 | CA | ARG | 7 | 34.120 | 3.835 | -23.733 | 0.00 | 0.00 |
| ATOM | 50 | C | ARG | 7 | 35.410 | 4.653 | -23.343 | 0.00 | 0.00 |
| ATOM | 51 | O | ARG | 7 | 35.233 | 5.799 | -22.940 | 0.00 | 0.00 |
| ATOM | 52 | CB | ARG | 7 | 33.580 | 3.010 | -22.551 | 0.00 | 0.00 |
| ATOM | 53 | CG | ARG | 7 | 33.328 | 3.924 | -21.350 | 0.00 | 0.00 |
| ATOM | 54 | CD | ARG | 7 | 32.202 | 4.900 | -21.560 | 0.00 | 0.00 |

| | | | | | | | | | |
|------|----|-----|-----|----|--------|--------|---------|------|------|
| ATOM | 55 | NE  | ARG | 7  | 30.908 | 4.251  | -21.675 | 0.00 | 0.00 |
| ATOM | 56 | CZ  | ARG | 7  | 29.740 | 4.902  | -21.547 | 0.00 | 0.00 |
| ATOM | 57 | NH1 | ARG | 7  | 29.675 | 6.216  | -21.315 | 0.00 | 0.00 |
| ATOM | 58 | NH2 | ARG | 7  | 28.609 | 4.208  | -21.638 | 0.00 | 0.00 |
| ATOM | 59 | N   | SER | 8  | 36.654 | 4.190  | -23.656 | 0.00 | 0.00 |
| ATOM | 60 | CA  | SER | 8  | 37.904 | 4.876  | -23.437 | 0.00 | 0.00 |
| ATOM | 61 | C   | SER | 8  | 37.932 | 6.234  | -24.183 | 0.00 | 0.00 |
| ATOM | 62 | O   | SER | 8  | 37.958 | 6.283  | -25.421 | 0.00 | 0.00 |
| ATOM | 63 | CB  | SER | 8  | 39.089 | 3.986  | -23.904 | 0.00 | 0.00 |
| ATOM | 64 | OG  | SER | 8  | 40.409 | 4.526  | -23.618 | 0.00 | 0.00 |
| ATOM | 65 | N   | HIS | 9  | 38.165 | 7.300  | -23.402 | 0.00 | 0.00 |
| ATOM | 66 | CA  | HIS | 9  | 38.329 | 8.653  | -23.973 | 0.00 | 0.00 |
| ATOM | 67 | C   | HIS | 9  | 39.745 | 9.163  | -23.460 | 0.00 | 0.00 |
| ATOM | 68 | O   | HIS | 9  | 39.917 | 9.246  | -22.240 | 0.00 | 0.00 |
| ATOM | 69 | CB  | HIS | 9  | 37.146 | 9.564  | -23.685 | 0.00 | 0.00 |
| ATOM | 70 | CG  | HIS | 9  | 37.047 | 10.796 | -24.493 | 0.00 | 0.00 |
| ATOM | 71 | ND1 | HIS | 9  | 36.064 | 11.704 | -24.557 | 0.00 | 0.00 |
| ATOM | 72 | CD2 | HIS | 9  | 37.990 | 11.194 | -25.416 | 0.00 | 0.00 |
| ATOM | 73 | CE1 | HIS | 9  | 36.376 | 12.602 | -25.463 | 0.00 | 0.00 |
| ATOM | 74 | NE2 | HIS | 9  | 37.544 | 12.281 | -25.972 | 0.00 | 0.00 |
| ATOM | 75 | N   | ALA | 10 | 40.613 | 9.714  | -24.336 | 0.00 | 0.00 |
| ATOM | 76 | CA  | ALA | 10 | 41.978 | 10.099 | -23.914 | 0.00 | 0.00 |
| ATOM | 77 | C   | ALA | 10 | 41.939 | 10.930 | -22.615 | 0.00 | 0.00 |
| ATOM | 78 | O   | ALA | 10 | 41.034 | 11.817 | -22.560 | 0.00 | 0.00 |
| ATOM | 79 | CB  | ALA | 10 | 42.624 | 10.929 | -25.068 | 0.00 | 0.00 |

```
ATOM    80  N   PRO   11    42.636  10.598  -21.488  0.00  0.00

ATOM    81  CA  PRO   11    42.443  11.430  -20.339  0.00  0.00

ATOM    82  C   PRO   11    42.462  12.969  -20.636  0.00  0.00

ATOM    83  O   PRO   11    41.726  13.657  -19.918  0.00  0.00

ATOM    84  CB  PRO   11    43.351  11.045  -19.192  0.00  0.00

ATOM    85  CG  PRO   11    44.598  10.601  -20.045  0.00  0.00

ATOM    86  CD  PRO   11    44.017   9.941  -21.287  0.00  0.00

ATOM    87  N   ASP   12    43.185  13.472  -21.606  0.00  0.00

ATOM    88  CA  ASP   12    43.119  14.896  -21.854  0.00  0.00

ATOM    89  C   ASP   12    41.647  15.423  -22.107  0.00  0.00

ATOM    90  O   ASP   12    41.238  16.320  -21.367  0.00  0.00

ATOM    91  CB  ASP   12    44.036  15.208  -23.051  0.00  0.00

ATOM    92  CG  ASP   12    45.467  14.821  -22.771  0.00  0.00

ATOM    93  OD1 ASP   12    46.035  14.195  -23.582  0.00  0.00

ATOM    94  OD2 ASP   12    45.951  15.218  -21.705  0.00  0.00

ATOM    95  N   HIS   13    40.922  14.971  -23.139  0.00  0.00

ATOM    96  CA  HIS   13    39.565  15.432  -23.459  0.00  0.00

ATOM    97  C   HIS   13    38.527  15.140  -22.337  0.00  0.00

ATOM    98  O   HIS   13    37.979  16.107  -21.805  0.00  0.00

ATOM    99  CB  HIS   13    39.144  14.813  -24.774  0.00  0.00

ATOM   100  CG  HIS   13    39.899  15.091  -25.990  0.00  0.00

ATOM   101  ND1 HIS   13    39.346  15.454  -27.190  0.00  0.00

ATOM   102  CD2 HIS   13    41.238  15.064  -26.209  0.00  0.00

ATOM   103  CE1 HIS   13    40.314  15.638  -28.078  0.00  0.00

ATOM   104  NE2 HIS   13    41.467  15.402  -27.511  0.00  0.00
```

| | | | | | | | | | |
|---|---|---|---|---|---|---|---|---|---|
| ATOM | 105 | N | ASP | 14 | 38.258 | 13.862 | -21.957 | 0.00 | 0.00 |
| ATOM | 106 | CA | ASP | 14 | 37.253 | 13.533 | -20.930 | 0.00 | 0.00 |
| ATOM | 107 | C | ASP | 14 | 37.791 | 12.698 | -19.741 | 0.00 | 0.00 |
| ATOM | 108 | O | ASP | 14 | 38.666 | 11.838 | -19.929 | 0.00 | 0.00 |
| ATOM | 109 | CB | ASP | 14 | 35.903 | 13.055 | -21.485 | 0.00 | 0.00 |
| ATOM | 110 | CG | ASP | 14 | 35.199 | 13.882 | -22.507 | 0.00 | 0.00 |
| ATOM | 111 | OD1 | ASP | 14 | 34.360 | 13.247 | -23.276 | 0.00 | 0.00 |
| ATOM | 112 | OD2 | ASP | 14 | 35.353 | 15.131 | -22.637 | 0.00 | 0.00 |
| ATOM | 113 | N | VAL | 15 | 36.898 | 12.656 | -18.710 | 0.00 | 0.00 |
| ATOM | 114 | CA | VAL | 15 | 37.136 | 12.036 | -17.475 | 0.00 | 0.00 |
| ATOM | 115 | C | VAL | 15 | 37.399 | 10.517 | -17.579 | 0.00 | 0.00 |
| ATOM | 116 | O | VAL | 15 | 36.947 | 9.837 | -18.475 | 0.00 | 0.00 |
| ATOM | 117 | CB | VAL | 15 | 36.014 | 12.360 | -16.536 | 0.00 | 0.00 |
| ATOM | 118 | CG1 | VAL | 15 | 35.140 | 13.518 | -16.971 | 0.00 | 0.00 |
| ATOM | 119 | CG2 | VAL | 15 | 35.167 | 11.104 | -16.240 | 0.00 | 0.00 |
| ATOM | 120 | N | THR | 16 | 38.144 | 10.027 | -16.591 | 0.00 | 0.00 |
| ATOM | 121 | CA | THR | 16 | 38.439 | 8.589 | -16.520 | 0.00 | 0.00 |
| ATOM | 122 | C | THR | 16 | 37.305 | 7.828 | -15.707 | 0.00 | 0.00 |
| ATOM | 123 | O | THR | 16 | 37.664 | 6.837 | -15.059 | 0.00 | 0.00 |
| ATOM | 124 | CB | THR | 16 | 39.934 | 8.199 | -16.206 | 0.00 | 0.00 |
| ATOM | 125 | OG1 | THR | 16 | 40.373 | 7.068 | -17.053 | 0.00 | 0.00 |
| ATOM | 126 | CG2 | THR | 16 | 40.471 | 7.943 | -14.793 | 0.00 | 0.00 |
| ATOM | 127 | N | GLN | 17 | 36.092 | 8.409 | -15.467 | 0.00 | 0.00 |
| ATOM | 128 | CA | GLN | 17 | 35.022 | 7.662 | -14.758 | 0.00 | 0.00 |
| ATOM | 129 | C | GLN | 17 | 34.965 | 6.183 | -15.162 | 0.00 | 0.00 |

```
ATOM    130  O    GLN   17    34.657    5.399  -14.270   0.00   0.00

ATOM    131  CB   GLN   17    33.586    8.287  -14.893   0.00   0.00

ATOM    132  CG   GLN   17    33.654    9.770  -14.559   0.00   0.00

ATOM    133  CD   GLN   17    34.068    9.953  -13.109   0.00   0.00

ATOM    134  OE1  GLN   17    33.406    9.484  -12.174   0.00   0.00

ATOM    135  NE2  GLN   17    35.158   10.661  -12.865   0.00   0.00

ATOM    136  N    GLN   18    34.862    5.891  -16.491   0.00   0.00

ATOM    137  CA   GLN   18    34.747    4.512  -16.869   0.00   0.00

ATOM    138  C    GLN   18    35.809    3.595  -16.138   0.00   0.00

ATOM    139  O    GLN   18    35.450    2.468  -15.813   0.00   0.00

ATOM    140  CB   GLN   18    34.920    4.249  -18.388   0.00   0.00

ATOM    141  CG   GLN   18    36.218    4.759  -19.045   0.00   0.00

ATOM    142  CD   GLN   18    37.257    3.731  -19.366   0.00   0.00

ATOM    143  OE1  GLN   18    37.392    2.667  -18.765   0.00   0.00

ATOM    144  NE2  GLN   18    38.080    4.012  -20.401   0.00   0.00

ATOM    145  N    ARG   19    37.083    4.023  -15.906   0.00   0.00

ATOM    146  CA   ARG   19    38.097    3.241  -15.140   0.00   0.00

ATOM    147  C    ARG   19    37.579    3.043  -13.679   0.00   0.00

ATOM    148  O    ARG   19    37.479    1.863  -13.285   0.00   0.00

ATOM    149  CB   ARG   19    39.523    3.759  -15.273   0.00   0.00

ATOM    150  CG   ARG   19    40.143    3.416  -16.595   0.00   0.00

ATOM    151  CD   ARG   19    41.240    4.369  -17.113   0.00   0.00

ATOM    152  NE   ARG   19    41.647    3.953  -18.459   0.00   0.00

ATOM    153  CZ   ARG   19    41.337    4.641  -19.576   0.00   0.00

ATOM    154  NH1  ARG   19    40.636    5.769  -19.498   0.00   0.00
```

| | | | | | | | | | |
|------|-----|-----|-----|----|--------|--------|---------|------|------|
| ATOM | 155 | NH2 | ARG | 19 | 41.662 | 4.113  | -20.774 | 0.00 | 0.00 |
| ATOM | 156 | N   | ASP | 20 | 37.268 | 4.090  | -12.935 | 0.00 | 0.00 |
| ATOM | 157 | CA  | ASP | 20 | 36.690 | 3.915  | -11.600 | 0.00 | 0.00 |
| ATOM | 158 | C   | ASP | 20 | 35.630 | 5.023  | -11.287 | 0.00 | 0.00 |
| ATOM | 159 | O   | ASP | 20 | 35.723 | 6.176  | -11.743 | 0.00 | 0.00 |
| ATOM | 160 | CB  | ASP | 20 | 37.822 | 3.783  | -10.606 | 0.00 | 0.00 |
| ATOM | 161 | CG  | ASP | 20 | 38.595 | 5.053  | -10.375 | 0.00 | 0.00 |
| ATOM | 162 | OD1 | ASP | 20 | 38.044 | 6.107  | -10.114 | 0.00 | 0.00 |
| ATOM | 163 | OD2 | ASP | 20 | 39.837 | 5.007  | -10.540 | 0.00 | 0.00 |
| ATOM | 164 | N   | GLU | 21 | 34.700 | 4.679  | -10.415 | 0.00 | 0.00 |
| ATOM | 165 | CA  | GLU | 21 | 33.606 | 5.526  | -10.071 | 0.00 | 0.00 |
| ATOM | 166 | C   | GLU | 21 | 34.041 | 6.910  | -9.445  | 0.00 | 0.00 |
| ATOM | 167 | O   | GLU | 21 | 35.248 | 7.163  | -9.339  | 0.00 | 0.00 |
| ATOM | 168 | CB  | GLU | 21 | 32.738 | 4.723  | -9.043  | 0.00 | 0.00 |
| ATOM | 169 | CG  | GLU | 21 | 33.123 | 3.256  | -8.825  | 0.00 | 0.00 |
| ATOM | 170 | CD  | GLU | 21 | 32.520 | 2.472  | -7.697  | 0.00 | 0.00 |
| ATOM | 171 | OE1 | GLU | 21 | 33.064 | 1.646  | -6.924  | 0.00 | 0.00 |
| ATOM | 172 | OE2 | GLU | 21 | 31.270 | 2.655  | -7.518  | 0.00 | 0.00 |
| ATOM | 173 | N   | VAL | 22 | 33.169 | 7.904  | -9.663  | 0.00 | 0.00 |
| ATOM | 174 | CA  | VAL | 22 | 33.328 | 9.295  | -9.162  | 0.00 | 0.00 |
| ATOM | 175 | C   | VAL | 22 | 33.760 | 9.406  | -7.664  | 0.00 | 0.00 |
| ATOM | 176 | O   | VAL | 22 | 34.388 | 10.426 | -7.367  | 0.00 | 0.00 |
| ATOM | 177 | CB  | VAL | 22 | 31.998 | 10.004 | -9.510  | 0.00 | 0.00 |
| ATOM | 178 | CG1 | VAL | 22 | 30.860 | 9.381  | -8.711  | 0.00 | 0.00 |
| ATOM | 179 | CG2 | VAL | 22 | 32.112 | 11.487 | -9.103  | 0.00 | 0.00 |

23

| ATOM | 180 | N | TRP | 23 | 33.027 | 8.787 | -6.696 | 0.00 | 0.00 |
|------|-----|-----|-----|----|--------|-------|--------|------|------|
| ATOM | 181 | CA | TRP | 23 | 33.531 | 8.852 | -5.337 | 0.00 | 0.00 |
| ATOM | 182 | C | TRP | 23 | 35.021 | 8.365 | -5.294 | 0.00 | 0.00 |
| ATOM | 183 | O | TRP | 23 | 35.705 | 8.687 | -4.312 | 0.00 | 0.00 |
| ATOM | 184 | CB | TRP | 23 | 32.668 | 8.048 | -4.390 | 0.00 | 0.00 |
| ATOM | 185 | CG | TRP | 23 | 32.763 | 6.567 | -4.496 | 0.00 | 0.00 |
| ATOM | 186 | CD1 | TRP | 23 | 31.994 | 5.798 | -5.290 | 0.00 | 0.00 |
| ATOM | 187 | CD2 | TRP | 23 | 33.717 | 5.714 | -3.855 | 0.00 | 0.00 |
| ATOM | 188 | NE1 | TRP | 23 | 32.382 | 4.481 | -5.168 | 0.00 | 0.00 |
| ATOM | 189 | CE2 | TRP | 23 | 33.417 | 4.433 | -4.338 | 0.00 | 0.00 |
| ATOM | 190 | CE3 | TRP | 23 | 34.777 | 5.905 | -2.952 | 0.00 | 0.00 |
| ATOM | 191 | CZ2 | TRP | 23 | 34.172 | 3.333 | -3.908 | 0.00 | 0.00 |
| ATOM | 192 | CZ3 | TRP | 23 | 35.557 | 4.817 | -2.517 | 0.00 | 0.00 |
| ATOM | 193 | CH2 | TRP | 23 | 35.228 | 3.556 | -3.021 | 0.00 | 0.00 |
| ATOM | 194 | N | VAL | 24 | 35.504 | 7.655 | -6.351 | 0.00 | 0.00 |
| ATOM | 195 | CA | VAL | 24 | 36.836 | 7.229 | -6.418 | 0.00 | 0.00 |
| ATOM | 196 | C | VAL | 24 | 37.702 | 8.493 | -6.828 | 0.00 | 0.00 |
| ATOM | 197 | O | VAL | 24 | 38.714 | 8.260 | -7.443 | 0.00 | 0.00 |
| ATOM | 198 | CB | VAL | 24 | 37.029 | 6.004 | -7.320 | 0.00 | 0.00 |
| ATOM | 199 | CG1 | VAL | 24 | 38.548 | 5.664 | -7.451 | 0.00 | 0.00 |
| ATOM | 200 | CG2 | VAL | 24 | 36.202 | 4.829 | -6.966 | 0.00 | 0.00 |
| ATOM | 201 | N | VAL | 25 | 37.129 | 9.702 | -6.987 | 0.00 | 0.00 |
| ATOM | 202 | CA | VAL | 25 | 38.023 | 10.840 | -7.175 | 0.00 | 0.00 |
| ATOM | 203 | C | VAL | 25 | 38.870 | 10.750 | -5.866 | 0.00 | 0.00 |
| ATOM | 204 | O | VAL | 25 | 39.748 | 9.821 | -5.841 | 0.00 | 0.00 |

24

```
ATOM    205  CB  VAL    25    37.298  12.177  -7.417  0.00  0.00

ATOM    206  CG1 VAL    25    36.635  12.311  -8.764  0.00  0.00

ATOM    207  CG2 VAL    25    36.505  12.774  -6.310  0.00  0.00

ATOM    208  N   GLY    26    38.250  11.098  -4.694  0.00  0.00

ATOM    209  CA  GLY    26    38.795  10.938  -3.307  0.00  0.00

ATOM    210  C   GLY    26    40.361  11.172  -3.168  0.00  0.00

ATOM    211  O   GLY    26    40.894  10.843  -2.115  0.00  0.00

ATOM    212  N   MET    27    41.021  11.449  -4.261  0.00  0.00

ATOM    213  CA  MET    27    42.388  11.791  -4.408  0.00  0.00

ATOM    214  C   MET    27    42.477  13.331  -4.440  0.00  0.00

ATOM    215  O   MET    27    43.599  13.810  -4.420  0.00  0.00

ATOM    216  CB  MET    27    42.822  11.231  -5.750  0.00  0.00

ATOM    217  CG  MET    27    42.927   9.726  -5.779  0.00  0.00

ATOM    218  SD  MET    27    44.503   9.174  -5.069  0.00  0.00

ATOM    219  CE  MET    27    45.560   9.399  -6.527  0.00  0.00

ATOM    220  N   GLY    28    41.416  14.039  -4.820  0.00  0.00

ATOM    221  CA  GLY    28    41.311  15.472  -4.810  0.00  0.00

ATOM    222  C   GLY    28    41.292  15.888  -3.308  0.00  0.00

ATOM    223  O   GLY    28    41.587  17.061  -3.075  0.00  0.00

ATOM    224  N   ILE    29    40.434  15.237  -2.504  0.00  0.00

ATOM    225  CA  ILE    29    40.315  15.392  -1.034  0.00  0.00

ATOM    226  C   ILE    29    41.750  15.108  -0.437  0.00  0.00

ATOM    227  O   ILE    29    42.191  15.893   0.402  0.00  0.00

ATOM    228  CB  ILE    29    39.150  14.517  -0.452  0.00  0.00

ATOM    229  CG1 ILE    29    39.210  14.583   1.105  0.00  0.00
```

```
ATOM   230  CG2 ILE   29    39.307  12.999  -0.848   0.00   0.00

ATOM   231  CD1 ILE   29    38.727  15.958   1.665   0.00   0.00

ATOM   232  N   VAL   30    42.298  13.939  -0.711   0.00   0.00

ATOM   233  CA  VAL   30    43.643  13.534  -0.379   0.00   0.00

ATOM   234  C   VAL   30    44.676  14.603  -0.879   0.00   0.00

ATOM   235  O   VAL   30    45.661  14.801  -0.166   0.00   0.00

ATOM   236  CB  VAL   30    43.919  12.071  -0.804   0.00   0.00

ATOM   237  CG1 VAL   30    42.932  11.054  -0.159   0.00   0.00

ATOM   238  CG2 VAL   30    44.133  11.933  -2.305   0.00   0.00

ATOM   239  N   MET   31    44.600  15.005  -2.173   0.00   0.00

ATOM   240  CA  MET   31    45.440  16.108  -2.693   0.00   0.00

ATOM   241  C   MET   31    45.337  17.387  -1.831   0.00   0.00

ATOM   242  O   MET   31    46.326  18.112  -1.784   0.00   0.00

ATOM   243  CB  MET   31    45.211  16.441  -4.208   0.00   0.00

ATOM   244  CG  MET   31    45.684  15.368  -5.126   0.00   0.00

ATOM   245  SD  MET   31    47.375  14.817  -4.763   0.00   0.00

ATOM   246  CE  MET   31    48.290  16.334  -4.787   0.00   0.00

ATOM   247  N   SER   32    44.088  17.882  -1.686   0.00   0.00

ATOM   248  CA  SER   32    43.770  19.048  -0.888   0.00   0.00

ATOM   249  C   SER   32    44.547  18.975   0.463   0.00   0.00

ATOM   250  O   SER   32    45.061  20.025   0.848   0.00   0.00

ATOM   251  CB  SER   32    42.242  19.126  -0.706   0.00   0.00

ATOM   252  OG  SER   32    41.481  19.637  -1.788   0.00   0.00

ATOM   253  N   LEU   33    44.411  17.917   1.294   0.00   0.00

ATOM   254  CA  LEU   33    45.140  17.701   2.506   0.00   0.00
```

| ATOM | 255 | C | LEU | 33 | 46.690 | 17.860 | 2.286 | 0.00 | 0.00 |
| ATOM | 256 | O | LEU | 33 | 47.300 | 18.439 | 3.186 | 0.00 | 0.00 |
| ATOM | 257 | CB | LEU | 33 | 44.779 | 16.312 | 3.036 | 0.00 | 0.00 |
| ATOM | 258 | CG | LEU | 33 | 43.521 | 16.067 | 3.847 | 0.00 | 0.00 |
| ATOM | 259 | CD1 | LEU | 33 | 42.327 | 16.943 | 3.499 | 0.00 | 0.00 |
| ATOM | 260 | CD2 | LEU | 33 | 43.158 | 14.652 | 3.511 | 0.00 | 0.00 |
| ATOM | 261 | N | ILE | 34 | 47.348 | 17.141 | 1.332 | 0.00 | 0.00 |
| ATOM | 262 | CA | ILE | 34 | 48.774 | 17.315 | 1.001 | 0.00 | 0.00 |
| ATOM | 263 | C | ILE | 34 | 49.064 | 18.836 | 0.762 | 0.00 | 0.00 |
| ATOM | 264 | O | ILE | 34 | 49.780 | 19.368 | 1.583 | 0.00 | 0.00 |
| ATOM | 265 | CB | ILE | 34 | 49.155 | 16.445 | -0.241 | 0.00 | 0.00 |
| ATOM | 266 | CG1 | ILE | 34 | 49.439 | 14.983 | 0.179 | 0.00 | 0.00 |
| ATOM | 267 | CG2 | ILE | 34 | 50.414 | 17.047 | -0.970 | 0.00 | 0.00 |
| ATOM | 268 | CD1 | ILE | 34 | 49.621 | 13.952 | -0.963 | 0.00 | 0.00 |
| ATOM | 269 | N | VAL | 35 | 48.286 | 19.586 | -0.065 | 0.00 | 0.00 |
| ATOM | 270 | CA | VAL | 35 | 48.409 | 21.059 | -0.311 | 0.00 | 0.00 |
| ATOM | 271 | C | VAL | 35 | 48.439 | 21.835 | 1.055 | 0.00 | 0.00 |
| ATOM | 272 | O | VAL | 35 | 49.440 | 22.516 | 1.327 | 0.00 | 0.00 |
| ATOM | 273 | CB | VAL | 35 | 47.406 | 21.632 | -1.382 | 0.00 | 0.00 |
| ATOM | 274 | CG1 | VAL | 35 | 47.302 | 23.184 | -1.439 | 0.00 | 0.00 |
| ATOM | 275 | CG2 | VAL | 35 | 47.778 | 20.968 | -2.758 | 0.00 | 0.00 |
| ATOM | 276 | N | LEU | 36 | 47.425 | 21.716 | 1.896 | 0.00 | 0.00 |
| ATOM | 277 | CA | LEU | 36 | 47.336 | 22.397 | 3.181 | 0.00 | 0.00 |
| ATOM | 278 | C | LEU | 36 | 48.558 | 22.038 | 4.110 | 0.00 | 0.00 |
| ATOM | 279 | O | LEU | 36 | 49.215 | 22.986 | 4.544 | 0.00 | 0.00 |

| ATOM | 280 | CB | LEU | 36 | 45.971 | 22.117 | 3.834 | 0.00 | 0.00 |
|------|-----|-----|-----|-----|--------|--------|-------|------|------|
| ATOM | 281 | CG | LEU | 36 | 45.593 | 22.954 | 5.060 | 0.00 | 0.00 |
| ATOM | 282 | CD1 | LEU | 36 | 45.595 | 24.465 | 4.776 | 0.00 | 0.00 |
| ATOM | 283 | CD2 | LEU | 36 | 44.252 | 22.445 | 5.501 | 0.00 | 0.00 |
| ATOM | 284 | N | ALA | 37 | 48.858 | 20.753 | 4.422 | 0.00 | 0.00 |
| ATOM | 285 | CA | ALA | 37 | 50.031 | 20.359 | 5.217 | 0.00 | 0.00 |
| ATOM | 286 | C | ALA | 37 | 51.432 | 20.733 | 4.571 | 0.00 | 0.00 |
| ATOM | 287 | O | ALA | 37 | 52.292 | 21.121 | 5.351 | 0.00 | 0.00 |
| ATOM | 288 | CB | ALA | 37 | 49.903 | 18.854 | 5.489 | 0.00 | 0.00 |
| ATOM | 289 | N | ILE | 38 | 51.750 | 20.251 | 3.368 | 0.00 | 0.00 |
| ATOM | 290 | CA | ILE | 38 | 52.980 | 20.529 | 2.602 | 0.00 | 0.00 |
| ATOM | 291 | C | ILE | 38 | 53.289 | 22.083 | 2.533 | 0.00 | 0.00 |
| ATOM | 292 | O | ILE | 38 | 54.465 | 22.424 | 2.683 | 0.00 | 0.00 |
| ATOM | 293 | CB | ILE | 38 | 52.911 | 19.954 | 1.168 | 0.00 | 0.00 |
| ATOM | 294 | CG1 | ILE | 38 | 52.062 | 20.725 | 0.185 | 0.00 | 0.00 |
| ATOM | 295 | CG2 | ILE | 38 | 52.649 | 18.434 | 1.186 | 0.00 | 0.00 |
| ATOM | 296 | CD1 | ILE | 38 | 52.006 | 20.193 | -1.262 | 0.00 | 0.00 |
| ATOM | 297 | N | VAL | 39 | 52.315 | 22.992 | 2.240 | 0.00 | 0.00 |
| ATOM | 298 | CA | VAL | 39 | 52.524 | 24.429 | 2.267 | 0.00 | 0.00 |
| ATOM | 299 | C | VAL | 39 | 52.976 | 24.906 | 3.691 | 0.00 | 0.00 |
| ATOM | 300 | O | VAL | 39 | 54.143 | 25.255 | 3.802 | 0.00 | 0.00 |
| ATOM | 301 | CB | VAL | 39 | 51.259 | 25.174 | 1.793 | 0.00 | 0.00 |
| ATOM | 302 | CG1 | VAL | 39 | 51.415 | 26.689 | 2.035 | 0.00 | 0.00 |
| ATOM | 303 | CG2 | VAL | 39 | 50.982 | 24.940 | 0.328 | 0.00 | 0.00 |
| ATOM | 304 | N | PHE | 40 | 52.202 | 24.694 | 4.792 | 0.00 | 0.00 |

| ATOM | 305 | CA | PHE | 40 | 52.564 | 25.112 | 6.152 | 0.00 | 0.00 |
| ATOM | 306 | C | PHE | 40 | 53.850 | 24.412 | 6.718 | 0.00 | 0.00 |
| ATOM | 307 | O | PHE | 40 | 54.665 | 25.121 | 7.324 | 0.00 | 0.00 |
| ATOM | 308 | CB | PHE | 40 | 51.338 | 24.878 | 7.040 | 0.00 | 0.00 |
| ATOM | 309 | CG | PHE | 40 | 50.398 | 26.065 | 7.048 | 0.00 | 0.00 |
| ATOM | 310 | CD1 | PHE | 40 | 50.668 | 27.222 | 6.305 | 0.00 | 0.00 |
| ATOM | 311 | CD2 | PHE | 40 | 49.264 | 26.003 | 7.856 | 0.00 | 0.00 |
| ATOM | 312 | CE1 | PHE | 40 | 49.815 | 28.317 | 6.371 | 0.00 | 0.00 |
| ATOM | 313 | CE2 | PHE | 40 | 48.412 | 27.107 | 7.918 | 0.00 | 0.00 |
| ATOM | 314 | CZ | PHE | 40 | 48.686 | 28.262 | 7.179 | 0.00 | 0.00 |
| ATOM | 315 | N | GLY | 41 | 53.993 | 23.063 | 6.631 | 0.00 | 0.00 |
| ATOM | 316 | CA | GLY | 41 | 55.180 | 22.285 | 7.024 | 0.00 | 0.00 |
| ATOM | 317 | C | GLY | 41 | 56.512 | 22.798 | 6.425 | 0.00 | 0.00 |
| ATOM | 318 | O | GLY | 41 | 57.129 | 23.578 | 7.116 | 0.00 | 0.00 |
| ATOM | 319 | N | ASN | 42 | 56.594 | 22.877 | 5.093 | 0.00 | 0.00 |
| ATOM | 320 | CA | ASN | 42 | 57.792 | 23.265 | 4.293 | 0.00 | 0.00 |
| ATOM | 321 | C | ASN | 42 | 57.958 | 24.798 | 4.372 | 0.00 | 0.00 |
| ATOM | 322 | O | ASN | 42 | 59.012 | 25.199 | 4.835 | 0.00 | 0.00 |
| ATOM | 323 | CB | ASN | 42 | 57.500 | 22.770 | 2.902 | 0.00 | 0.00 |
| ATOM | 324 | CG | ASN | 42 | 57.785 | 21.337 | 2.647 | 0.00 | 0.00 |
| ATOM | 325 | OD1 | ASN | 42 | 58.920 | 20.891 | 2.857 | 0.00 | 0.00 |
| ATOM | 326 | ND2 | ASN | 42 | 56.784 | 20.533 | 2.285 | 0.00 | 0.00 |
| ATOM | 327 | N | VAL | 43 | 56.952 | 25.639 | 4.033 | 0.00 | 0.00 |
| ATOM | 328 | CA | VAL | 43 | 57.030 | 27.100 | 4.193 | 0.00 | 0.00 |
| ATOM | 329 | C | VAL | 43 | 57.552 | 27.464 | 5.619 | 0.00 | 0.00 |

29

| | | | | | | | | | |
|---|---|---|---|---|---|---|---|---|---|
| ATOM | 330 | O | VAL | 43 | 58.544 | 28.204 | 5.657 | 0.00 | 0.00 |
| ATOM | 331 | CB | VAL | 43 | 55.682 | 27.782 | 3.894 | 0.00 | 0.00 |
| ATOM | 332 | CG1 | VAL | 43 | 55.568 | 29.204 | 4.437 | 0.00 | 0.00 |
| ATOM | 333 | CG2 | VAL | 43 | 55.388 | 27.745 | 2.400 | 0.00 | 0.00 |
| ATOM | 334 | N | LEU | 44 | 56.889 | 27.059 | 6.728 | 0.00 | 0.00 |
| ATOM | 335 | CA | LEU | 44 | 57.389 | 27.306 | 8.090 | 0.00 | 0.00 |
| ATOM | 336 | C | LEU | 44 | 58.876 | 26.847 | 8.198 | 0.00 | 0.00 |
| ATOM | 337 | O | LEU | 44 | 59.634 | 27.613 | 8.785 | 0.00 | 0.00 |
| ATOM | 338 | CB | LEU | 44 | 56.481 | 26.610 | 9.133 | 0.00 | 0.00 |
| ATOM | 339 | CG | LEU | 44 | 57.118 | 26.694 | 10.532 | 0.00 | 0.00 |
| ATOM | 340 | CD1 | LEU | 44 | 57.194 | 28.153 | 10.959 | 0.00 | 0.00 |
| ATOM | 341 | CD2 | LEU | 44 | 56.281 | 25.913 | 11.508 | 0.00 | 0.00 |
| ATOM | 342 | N | VAL | 45 | 59.229 | 25.601 | 7.866 | 0.00 | 0.00 |
| ATOM | 343 | CA | VAL | 45 | 60.593 | 25.117 | 7.837 | 0.00 | 0.00 |
| ATOM | 344 | C | VAL | 45 | 61.557 | 26.056 | 7.003 | 0.00 | 0.00 |
| ATOM | 345 | O | VAL | 45 | 62.654 | 26.307 | 7.502 | 0.00 | 0.00 |
| ATOM | 346 | CB | VAL | 45 | 60.640 | 23.661 | 7.422 | 0.00 | 0.00 |
| ATOM | 347 | CG1 | VAL | 45 | 62.036 | 23.062 | 7.418 | 0.00 | 0.00 |
| ATOM | 348 | CG2 | VAL | 45 | 59.620 | 22.786 | 8.143 | 0.00 | 0.00 |
| ATOM | 349 | N | ILE | 46 | 61.217 | 26.503 | 5.771 | 0.00 | 0.00 |
| ATOM | 350 | CA | ILE | 46 | 62.034 | 27.441 | 5.020 | 0.00 | 0.00 |
| ATOM | 351 | C | ILE | 46 | 62.139 | 28.791 | 5.786 | 0.00 | 0.00 |
| ATOM | 352 | O | ILE | 46 | 63.279 | 29.249 | 5.902 | 0.00 | 0.00 |
| ATOM | 353 | CB | ILE | 46 | 61.552 | 27.527 | 3.540 | 0.00 | 0.00 |
| ATOM | 354 | CG1 | ILE | 46 | 61.918 | 28.714 | 2.740 | 0.00 | 0.00 |

| ATOM | 355 | CG2 | ILE | 46 | 60.083 | 27.254 | 3.380 | 0.00 | 0.00 |
| ATOM | 356 | CD1 | ILE | 46 | 61.887 | 28.442 | 1.233 | 0.00 | 0.00 |
| ATOM | 357 | N | THR | 47 | 61.034 | 29.522 | 6.094 | 0.00 | 0.00 |
| ATOM | 358 | CA | THR | 47 | 61.131 | 30.729 | 6.902 | 0.00 | 0.00 |
| ATOM | 359 | C | THR | 47 | 62.028 | 30.554 | 8.196 | 0.00 | 0.00 |
| ATOM | 360 | O | THR | 47 | 62.634 | 31.550 | 8.590 | 0.00 | 0.00 |
| ATOM | 361 | CB | THR | 47 | 59.727 | 31.284 | 7.299 | 0.00 | 0.00 |
| ATOM | 362 | OG1 | THR | 47 | 58.778 | 31.267 | 6.200 | 0.00 | 0.00 |
| ATOM | 363 | CG2 | THR | 47 | 59.736 | 32.715 | 7.921 | 0.00 | 0.00 |
| ATOM | 364 | N | ALA | 48 | 61.918 | 29.467 | 8.987 | 0.00 | 0.00 |
| ATOM | 365 | CA | ALA | 48 | 62.757 | 29.154 | 10.148 | 0.00 | 0.00 |
| ATOM | 366 | C | ALA | 48 | 64.295 | 29.072 | 9.829 | 0.00 | 0.00 |
| ATOM | 367 | O | ALA | 48 | 65.030 | 29.710 | 10.590 | 0.00 | 0.00 |
| ATOM | 368 | CB | ALA | 48 | 62.185 | 27.881 | 10.783 | 0.00 | 0.00 |
| ATOM | 369 | N | ILE | 49 | 64.779 | 28.257 | 8.845 | 0.00 | 0.00 |
| ATOM | 370 | CA | ILE | 49 | 66.248 | 28.277 | 8.554 | 0.00 | 0.00 |
| ATOM | 371 | C | ILE | 49 | 66.712 | 29.683 | 8.023 | 0.00 | 0.00 |
| ATOM | 372 | O | ILE | 49 | 67.720 | 30.165 | 8.559 | 0.00 | 0.00 |
| ATOM | 373 | CB | ILE | 49 | 66.736 | 27.113 | 7.671 | 0.00 | 0.00 |
| ATOM | 374 | CG1 | ILE | 49 | 66.148 | 25.754 | 8.022 | 0.00 | 0.00 |
| ATOM | 375 | CG2 | ILE | 49 | 68.304 | 27.045 | 7.582 | 0.00 | 0.00 |
| ATOM | 376 | CD1 | ILE | 49 | 66.548 | 24.575 | 7.114 | 0.00 | 0.00 |
| ATOM | 377 | N | ALA | 50 | 66.029 | 30.333 | 7.071 | 0.00 | 0.00 |
| ATOM | 378 | CA | ALA | 50 | 66.343 | 31.691 | 6.590 | 0.00 | 0.00 |
| ATOM | 379 | C | ALA | 50 | 66.416 | 32.737 | 7.756 | 0.00 | 0.00 |

| ATOM | 380 | O | ALA | 50 | 67.465 | 33.403 | 7.836 | 0.00 | 0.00 |
|------|-----|-----|-----|-----|--------|--------|--------|------|------|
| ATOM | 381 | CB | ALA | 50 | 65.279 | 32.050 | 5.523 | 0.00 | 0.00 |
| ATOM | 382 | N | LYS | 51 | 65.372 | 32.949 | 8.560 | 0.00 | 0.00 |
| ATOM | 383 | CA | LYS | 51 | 65.443 | 33.901 | 9.677 | 0.00 | 0.00 |
| ATOM | 384 | C | LYS | 51 | 66.449 | 33.509 | 10.824 | 0.00 | 0.00 |
| ATOM | 385 | O | LYS | 51 | 67.030 | 34.455 | 11.366 | 0.00 | 0.00 |
| ATOM | 386 | CB | LYS | 51 | 64.027 | 34.061 | 10.235 | 0.00 | 0.00 |
| ATOM | 387 | CG | LYS | 51 | 63.747 | 35.082 | 11.344 | 0.00 | 0.00 |
| ATOM | 388 | CD | LYS | 51 | 62.155 | 35.241 | 11.442 | 0.00 | 0.00 |
| ATOM | 389 | CE | LYS | 51 | 61.659 | 35.425 | 10.051 | 0.00 | 0.00 |
| ATOM | 390 | NZ | LYS | 51 | 61.721 | 36.763 | 9.595 | 0.00 | 0.00 |
| ATOM | 391 | N | PHE | 52 | 66.819 | 32.218 | 11.035 | 0.00 | 0.00 |
| ATOM | 392 | CA | PHE | 52 | 67.673 | 31.889 | 12.184 | 0.00 | 0.00 |
| ATOM | 393 | C | PHE | 52 | 69.182 | 32.007 | 11.745 | 0.00 | 0.00 |
| ATOM | 394 | O | PHE | 52 | 69.649 | 31.199 | 10.924 | 0.00 | 0.00 |
| ATOM | 395 | CB | PHE | 52 | 67.169 | 30.460 | 12.505 | 0.00 | 0.00 |
| ATOM | 396 | CG | PHE | 52 | 66.297 | 30.478 | 13.789 | 0.00 | 0.00 |
| ATOM | 397 | CD1 | PHE | 52 | 65.357 | 31.493 | 13.904 | 0.00 | 0.00 |
| ATOM | 398 | CD2 | PHE | 52 | 66.379 | 29.479 | 14.760 | 0.00 | 0.00 |
| ATOM | 399 | CE1 | PHE | 52 | 64.499 | 31.551 | 14.998 | 0.00 | 0.00 |
| ATOM | 400 | CE2 | PHE | 52 | 65.499 | 29.512 | 15.863 | 0.00 | 0.00 |
| ATOM | 401 | CZ | PHE | 52 | 64.559 | 30.535 | 15.950 | 0.00 | 0.00 |
| ATOM | 402 | N | GLU | 53 | 69.838 | 33.121 | 12.141 | 0.00 | 0.00 |
| ATOM | 403 | CA | GLU | 53 | 71.224 | 33.483 | 11.772 | 0.00 | 0.00 |
| ATOM | 404 | C | GLU | 53 | 72.253 | 32.300 | 11.846 | 0.00 | 0.00 |

| | | | | | | | | | |
|------|-----|-----|-----|----|--------|--------|--------|------|------|
| ATOM | 405 | O   | GLU | 53 | 72.905 | 32.097 | 10.813 | 0.00 | 0.00 |
| ATOM | 406 | CB  | GLU | 53 | 71.714 | 34.639 | 12.639 | 0.00 | 0.00 |
| ATOM | 407 | CG  | GLU | 53 | 71.258 | 35.992 | 12.140 | 0.00 | 0.00 |
| ATOM | 408 | CD  | GLU | 53 | 71.530 | 37.134 | 13.113 | 0.00 | 0.00 |
| ATOM | 409 | OE1 | GLU | 53 | 72.631 | 37.187 | 13.681 | 0.00 | 0.00 |
| ATOM | 410 | OE2 | GLU | 53 | 70.624 | 37.956 | 13.302 | 0.00 | 0.00 |
| ATOM | 411 | N   | ARG | 54 | 72.279 | 31.449 | 12.896 | 0.00 | 0.00 |
| ATOM | 412 | CA  | ARG | 54 | 73.320 | 30.406 | 12.978 | 0.00 | 0.00 |
| ATOM | 413 | C   | ARG | 54 | 72.856 | 28.970 | 12.536 | 0.00 | 0.00 |
| ATOM | 414 | O   | ARG | 54 | 73.621 | 28.032 | 12.746 | 0.00 | 0.00 |
| ATOM | 415 | CB  | ARG | 54 | 73.838 | 30.307 | 14.395 | 0.00 | 0.00 |
| ATOM | 416 | CG  | ARG | 54 | 74.320 | 31.547 | 15.050 | 0.00 | 0.00 |
| ATOM | 417 | CD  | ARG | 54 | 75.345 | 32.183 | 14.170 | 0.00 | 0.00 |
| ATOM | 418 | NE  | ARG | 54 | 76.577 | 31.436 | 13.999 | 0.00 | 0.00 |
| ATOM | 419 | CZ  | ARG | 54 | 77.478 | 31.159 | 14.947 | 0.00 | 0.00 |
| ATOM | 420 | NH1 | ARG | 54 | 77.308 | 31.512 | 16.210 | 0.00 | 0.00 |
| ATOM | 421 | NH2 | ARG | 54 | 78.551 | 30.420 | 14.622 | 0.00 | 0.00 |
| ATOM | 422 | N   | LEU | 55 | 71.690 | 28.775 | 11.887 | 0.00 | 0.00 |
| ATOM | 423 | CA  | LEU | 55 | 71.224 | 27.467 | 11.342 | 0.00 | 0.00 |
| ATOM | 424 | C   | LEU | 55 | 71.903 | 27.331 |  9.952 | 0.00 | 0.00 |
| ATOM | 425 | O   | LEU | 55 | 71.219 | 27.473 |  8.931 | 0.00 | 0.00 |
| ATOM | 426 | CB  | LEU | 55 | 69.687 | 27.446 | 11.266 | 0.00 | 0.00 |
| ATOM | 427 | CG  | LEU | 55 | 69.112 | 26.484 | 12.343 | 0.00 | 0.00 |
| ATOM | 428 | CD1 | LEU | 55 | 69.750 | 26.636 | 13.709 | 0.00 | 0.00 |
| ATOM | 429 | CD2 | LEU | 55 | 67.618 | 26.568 | 12.475 | 0.00 | 0.00 |

| | | | | | | | | | |
|---|---|---|---|---|---|---|---|---|---|
| ATOM | 430 | N | GLN | 56 | 73.210 | 27.002 | 9.883 | 0.00 | 0.00 |
| ATOM | 431 | CA | GLN | 56 | 74.010 | 26.942 | 8.626 | 0.00 | 0.00 |
| ATOM | 432 | C | GLN | 56 | 74.605 | 25.556 | 8.299 | 0.00 | 0.00 |
| ATOM | 433 | O | GLN | 56 | 75.197 | 25.468 | 7.220 | 0.00 | 0.00 |
| ATOM | 434 | CB | GLN | 56 | 75.130 | 27.966 | 8.827 | 0.00 | 0.00 |
| ATOM | 435 | CG | GLN | 56 | 74.724 | 29.415 | 8.955 | 0.00 | 0.00 |
| ATOM | 436 | CD | GLN | 56 | 73.896 | 29.994 | 7.840 | 0.00 | 0.00 |
| ATOM | 437 | OE1 | GLN | 56 | 74.087 | 29.682 | 6.659 | 0.00 | 0.00 |
| ATOM | 438 | NE2 | GLN | 56 | 72.948 | 30.872 | 8.162 | 0.00 | 0.00 |
| ATOM | 439 | N | THR | 57 | 74.518 | 24.524 | 9.178 | 0.00 | 0.00 |
| ATOM | 440 | CA | THR | 57 | 75.128 | 23.264 | 8.924 | 0.00 | 0.00 |
| ATOM | 441 | C | THR | 57 | 74.657 | 22.707 | 7.557 | 0.00 | 0.00 |
| ATOM | 442 | O | THR | 57 | 73.564 | 23.065 | 7.093 | 0.00 | 0.00 |
| ATOM | 443 | CB | THR | 57 | 74.815 | 22.261 | 10.075 | 0.00 | 0.00 |
| ATOM | 444 | OG1 | THR | 57 | 73.485 | 21.652 | 9.991 | 0.00 | 0.00 |
| ATOM | 445 | CG2 | THR | 57 | 74.956 | 22.914 | 11.495 | 0.00 | 0.00 |
| ATOM | 446 | N | VAL | 58 | 75.524 | 22.016 | 6.819 | 0.00 | 0.00 |
| ATOM | 447 | CA | VAL | 58 | 75.086 | 21.382 | 5.576 | 0.00 | 0.00 |
| ATOM | 448 | C | VAL | 58 | 73.712 | 20.657 | 5.759 | 0.00 | 0.00 |
| ATOM | 449 | O | VAL | 58 | 72.947 | 20.662 | 4.811 | 0.00 | 0.00 |
| ATOM | 450 | CB | VAL | 58 | 76.245 | 20.473 | 5.141 | 0.00 | 0.00 |
| ATOM | 451 | CG1 | VAL | 58 | 75.900 | 19.381 | 4.113 | 0.00 | 0.00 |
| ATOM | 452 | CG2 | VAL | 58 | 77.380 | 21.381 | 4.563 | 0.00 | 0.00 |
| ATOM | 453 | N | THR | 59 | 73.454 | 19.942 | 6.890 | 0.00 | 0.00 |
| ATOM | 454 | CA | THR | 59 | 72.179 | 19.288 | 7.241 | 0.00 | 0.00 |

| ATOM | 455 | C | THR | 59 | 71.018 | 20.323 | 7.216 | 0.00 | 0.00 |
| ATOM | 456 | O | THR | 59 | 69.941 | 19.931 | 6.724 | 0.00 | 0.00 |
| ATOM | 457 | CB | THR | 59 | 72.290 | 18.586 | 8.638 | 0.00 | 0.00 |
| ATOM | 458 | OG1 | THR | 59 | 73.500 | 17.829 | 8.813 | 0.00 | 0.00 |
| ATOM | 459 | CG2 | THR | 59 | 71.066 | 17.647 | 8.949 | 0.00 | 0.00 |
| ATOM | 460 | N | ASN | 60 | 71.046 | 21.394 | 8.015 | 0.00 | 0.00 |
| ATOM | 461 | CA | ASN | 60 | 70.020 | 22.454 | 7.954 | 0.00 | 0.00 |
| ATOM | 462 | C | ASN | 60 | 69.914 | 22.946 | 6.495 | 0.00 | 0.00 |
| ATOM | 463 | O | ASN | 60 | 68.772 | 23.194 | 6.091 | 0.00 | 0.00 |
| ATOM | 464 | CB | ASN | 60 | 70.343 | 23.575 | 8.951 | 0.00 | 0.00 |
| ATOM | 465 | CG | ASN | 60 | 70.133 | 23.181 | 10.410 | 0.00 | 0.00 |
| ATOM | 466 | OD1 | ASN | 60 | 70.419 | 23.854 | 11.414 | 0.00 | 0.00 |
| ATOM | 467 | ND2 | ASN | 60 | 69.588 | 21.981 | 10.594 | 0.00 | 0.00 |
| ATOM | 468 | N | TYR | 61 | 71.008 | 23.287 | 5.784 | 0.00 | 0.00 |
| ATOM | 469 | CA | TYR | 61 | 70.860 | 23.638 | 4.386 | 0.00 | 0.00 |
| ATOM | 470 | C | TYR | 61 | 70.143 | 22.593 | 3.532 | 0.00 | 0.00 |
| ATOM | 471 | O | TYR | 61 | 69.377 | 23.026 | 2.671 | 0.00 | 0.00 |
| ATOM | 472 | CB | TYR | 61 | 72.201 | 23.958 | 3.716 | 0.00 | 0.00 |
| ATOM | 473 | CG | TYR | 61 | 72.672 | 25.362 | 3.823 | 0.00 | 0.00 |
| ATOM | 474 | CD1 | TYR | 61 | 71.959 | 26.362 | 3.188 | 0.00 | 0.00 |
| ATOM | 475 | CD2 | TYR | 61 | 73.823 | 25.649 | 4.536 | 0.00 | 0.00 |
| ATOM | 476 | CE1 | TYR | 61 | 72.399 | 27.664 | 3.268 | 0.00 | 0.00 |
| ATOM | 477 | CE2 | TYR | 61 | 74.264 | 26.946 | 4.619 | 0.00 | 0.00 |
| ATOM | 478 | CZ | TYR | 61 | 73.548 | 27.931 | 3.981 | 0.00 | 0.00 |
| ATOM | 479 | OH | TYR | 61 | 73.994 | 29.232 | 4.034 | 0.00 | 0.00 |

```
ATOM    480  N   PHE   62      70.659  21.371  3.469  0.00  0.00

ATOM    481  CA  PHE   62      70.031  20.299  2.783  0.00  0.00

ATOM    482  C   PHE   62      68.487  20.433  2.997  0.00  0.00

ATOM    483  O   PHE   62      67.706  20.195  2.077  0.00  0.00

ATOM    484  CB  PHE   62      70.484  19.071  3.571  0.00  0.00

ATOM    485  CG  PHE   62      71.608  18.266  3.087  0.00  0.00

ATOM    486  CD1 PHE   62      71.725  17.825  1.770  0.00  0.00

ATOM    487  CD2 PHE   62      72.631  18.033  3.983  0.00  0.00

ATOM    488  CE1 PHE   62      72.882  17.156  1.343  0.00  0.00

ATOM    489  CE2 PHE   62      73.793  17.367  3.599  0.00  0.00

ATOM    490  CZ  PHE   62      73.899  16.939  2.265  0.00  0.00

ATOM    491  N   ILE   63      68.162  20.467  4.318  0.00  0.00

ATOM    492  CA  ILE   63      66.816  20.627  4.871  0.00  0.00

ATOM    493  C   ILE   63      66.065  21.792  4.176  0.00  0.00

ATOM    494  O   ILE   63      64.907  21.553  3.835  0.00  0.00

ATOM    495  CB  ILE   63      66.760  20.684  6.437  0.00  0.00

ATOM    496  CG1 ILE   63      66.410  19.331  7.006  0.00  0.00

ATOM    497  CG2 ILE   63      65.718  21.758  6.976  0.00  0.00

ATOM    498  CD1 ILE   63      67.215  18.884  8.242  0.00  0.00

ATOM    499  N   THR   64      66.622  23.038  4.071  0.00  0.00

ATOM    500  CA  THR   64      65.923  24.159  3.360  0.00  0.00

ATOM    501  C   THR   64      65.602  23.756  1.888  0.00  0.00

ATOM    502  O   THR   64      64.622  24.298  1.389  0.00  0.00

ATOM    503  CB  THR   64      66.630  25.566  3.308  0.00  0.00

ATOM    504  OG1 THR   64      67.414  25.876  4.440  0.00  0.00
```

| | | | | | | | | | |
|---|---|---|---|---|---|---|---|---|---|
| ATOM | 505 | CG2 | THR | 64 | 65.519 | 26.672 | 3.144 | 0.00 | 0.00 |
| ATOM | 506 | N | SER | 65 | 66.620 | 23.365 | 1.105 | 0.00 | 0.00 |
| ATOM | 507 | CA | SER | 65 | 66.515 | 22.882 | -0.285 | 0.00 | 0.00 |
| ATOM | 508 | C | SER | 65 | 65.246 | 21.985 | -0.468 | 0.00 | 0.00 |
| ATOM | 509 | O | SER | 65 | 64.694 | 22.045 | -1.574 | 0.00 | 0.00 |
| ATOM | 510 | CB | SER | 65 | 67.814 | 22.182 | -0.704 | 0.00 | 0.00 |
| ATOM | 511 | OG | SER | 65 | 67.936 | 21.886 | -2.083 | 0.00 | 0.00 |
| ATOM | 512 | N | LEU | 66 | 65.101 | 20.903 | 0.304 | 0.00 | 0.00 |
| ATOM | 513 | CA | LEU | 66 | 63.927 | 20.083 | 0.279 | 0.00 | 0.00 |
| ATOM | 514 | C | LEU | 66 | 62.685 | 20.936 | 0.643 | 0.00 | 0.00 |
| ATOM | 515 | O | LEU | 66 | 61.646 | 20.570 | 0.167 | 0.00 | 0.00 |
| ATOM | 516 | CB | LEU | 66 | 64.146 | 18.983 | 1.319 | 0.00 | 0.00 |
| ATOM | 517 | CG | LEU | 66 | 65.340 | 18.064 | 1.164 | 0.00 | 0.00 |
| ATOM | 518 | CD1 | LEU | 66 | 65.317 | 17.154 | 2.376 | 0.00 | 0.00 |
| ATOM | 519 | CD2 | LEU | 66 | 65.376 | 17.397 | -0.202 | 0.00 | 0.00 |
| ATOM | 520 | N | ALA | 67 | 62.755 | 21.902 | 1.593 | 0.00 | 0.00 |
| ATOM | 521 | CA | ALA | 67 | 61.619 | 22.777 | 1.871 | 0.00 | 0.00 |
| ATOM | 522 | C | ALA | 67 | 61.197 | 23.477 | 0.532 | 0.00 | 0.00 |
| ATOM | 523 | O | ALA | 67 | 59.998 | 23.677 | 0.367 | 0.00 | 0.00 |
| ATOM | 524 | CB | ALA | 67 | 61.984 | 23.728 | 3.022 | 0.00 | 0.00 |
| ATOM | 525 | N | CYS | 68 | 62.125 | 24.149 | -0.184 | 0.00 | 0.00 |
| ATOM | 526 | CA | CYS | 68 | 61.902 | 24.758 | -1.483 | 0.00 | 0.00 |
| ATOM | 527 | C | CYS | 68 | 61.383 | 23.682 | -2.493 | 0.00 | 0.00 |
| ATOM | 528 | O | CYS | 68 | 60.549 | 24.078 | -3.305 | 0.00 | 0.00 |
| ATOM | 529 | CB | CYS | 68 | 63.246 | 25.371 | -1.948 | 0.00 | 0.00 |

| ATOM | 530 | SG | CYS | 68 | 63.781 | 26.812 | -0.973 | 0.00 | 0.00 |
|------|-----|----|----|-----|--------|--------|--------|------|------|
| ATOM | 531 | N | ALA | 69 | 62.118 | 22.565 | -2.742 | 0.00 | 0.00 |
| ATOM | 532 | CA | ALA | 69 | 61.679 | 21.451 | -3.598 | 0.00 | 0.00 |
| ATOM | 533 | C | ALA | 69 | 60.227 | 21.037 | -3.248 | 0.00 | 0.00 |
| ATOM | 534 | O | ALA | 69 | 59.416 | 21.135 | -4.143 | 0.00 | 0.00 |
| ATOM | 535 | CB | ALA | 69 | 62.683 | 20.283 | -3.494 | 0.00 | 0.00 |
| ATOM | 536 | N | ASP | 70 | 59.915 | 20.905 | -1.984 | 0.00 | 0.00 |
| ATOM | 537 | CA | ASP | 70 | 58.576 | 20.568 | -1.425 | 0.00 | 0.00 |
| ATOM | 538 | C | ASP | 70 | 57.528 | 21.674 | -1.822 | 0.00 | 0.00 |
| ATOM | 539 | O | ASP | 70 | 56.351 | 21.324 | -1.907 | 0.00 | 0.00 |
| ATOM | 540 | CB | ASP | 70 | 58.804 | 20.403 | 0.045 | 0.00 | 0.00 |
| ATOM | 541 | CG | ASP | 70 | 59.473 | 19.109 | 0.404 | 0.00 | 0.00 |
| ATOM | 542 | OD1 | ASP | 70 | 59.912 | 18.352 | -0.517 | 0.00 | 0.00 |
| ATOM | 543 | OD2 | ASP | 70 | 59.766 | 18.842 | 1.626 | 0.00 | 0.00 |
| ATOM | 544 | N | LEU | 71 | 57.841 | 22.978 | -1.696 | 0.00 | 0.00 |
| ATOM | 545 | CA | LEU | 71 | 56.974 | 24.056 | -2.136 | 0.00 | 0.00 |
| ATOM | 546 | C | LEU | 71 | 56.684 | 23.864 | -3.659 | 0.00 | 0.00 |
| ATOM | 547 | O | LEU | 71 | 55.557 | 24.164 | -4.027 | 0.00 | 0.00 |
| ATOM | 548 | CB | LEU | 71 | 57.564 | 25.426 | -1.840 | 0.00 | 0.00 |
| ATOM | 549 | CG | LEU | 71 | 57.746 | 25.748 | -0.374 | 0.00 | 0.00 |
| ATOM | 550 | CD1 | LEU | 71 | 58.330 | 27.165 | -0.266 | 0.00 | 0.00 |
| ATOM | 551 | CD2 | LEU | 71 | 56.360 | 25.708 | 0.283 | 0.00 | 0.00 |
| ATOM | 552 | N | VAL | 72 | 57.730 | 23.837 | -4.517 | 0.00 | 0.00 |
| ATOM | 553 | CA | VAL | 72 | 57.679 | 23.569 | -5.962 | 0.00 | 0.00 |
| ATOM | 554 | C | VAL | 72 | 56.673 | 22.374 | -6.180 | 0.00 | 0.00 |

| ATOM | 555 | O   | VAL | 72 | 55.878 | 22.483 | -7.085 | 0.00 | 0.00 |
|------|-----|-----|-----|----|--------|--------|--------|------|------|
| ATOM | 556 | CB  | VAL | 72 | 59.134 | 23.230 | -6.448 | 0.00 | 0.00 |
| ATOM | 557 | CG1 | VAL | 72 | 59.102 | 22.586 | -7.880 | 0.00 | 0.00 |
| ATOM | 558 | CG2 | VAL | 72 | 59.986 | 24.450 | -6.432 | 0.00 | 0.00 |
| ATOM | 559 | N   | MET | 73 | 56.793 | 21.254 | -5.430 | 0.00 | 0.00 |
| ATOM | 560 | CA  | MET | 73 | 55.899 | 20.123 | -5.437 | 0.00 | 0.00 |
| ATOM | 561 | C   | MET | 73 | 54.406 | 20.583 | -5.354 | 0.00 | 0.00 |
| ATOM | 562 | O   | MET | 73 | 53.852 | 20.797 | -6.427 | 0.00 | 0.00 |
| ATOM | 563 | CB  | MET | 73 | 56.274 | 19.313 | -4.151 | 0.00 | 0.00 |
| ATOM | 564 | CG  | MET | 73 | 57.737 | 18.821 | -4.289 | 0.00 | 0.00 |
| ATOM | 565 | SD  | MET | 73 | 58.046 | 17.715 | -2.884 | 0.00 | 0.00 |
| ATOM | 566 | CE  | MET | 73 | 57.288 | 16.214 | -3.402 | 0.00 | 0.00 |
| ATOM | 567 | N   | GLY | 74 | 54.096 | 21.264 | -4.258 | 0.00 | 0.00 |
| ATOM | 568 | CA  | GLY | 74 | 52.791 | 21.725 | -3.868 | 0.00 | 0.00 |
| ATOM | 569 | C   | GLY | 74 | 52.077 | 22.650 | -4.840 | 0.00 | 0.00 |
| ATOM | 570 | O   | GLY | 74 | 50.873 | 22.741 | -4.686 | 0.00 | 0.00 |
| ATOM | 571 | N   | LEU | 75 | 52.775 | 23.789 | -5.096 | 0.00 | 0.00 |
| ATOM | 572 | CA  | LEU | 75 | 52.356 | 24.815 | -6.051 | 0.00 | 0.00 |
| ATOM | 573 | C   | LEU | 75 | 52.496 | 24.411 | -7.563 | 0.00 | 0.00 |
| ATOM | 574 | O   | LEU | 75 | 51.766 | 25.018 | -8.370 | 0.00 | 0.00 |
| ATOM | 575 | CB  | LEU | 75 | 53.205 | 26.082 | -5.784 | 0.00 | 0.00 |
| ATOM | 576 | CG  | LEU | 75 | 52.992 | 26.791 | -4.437 | 0.00 | 0.00 |
| ATOM | 577 | CD1 | LEU | 75 | 53.866 | 28.026 | -4.320 | 0.00 | 0.00 |
| ATOM | 578 | CD2 | LEU | 75 | 51.540 | 27.182 | -4.219 | 0.00 | 0.00 |
| ATOM | 579 | N   | ALA | 76 | 53.634 | 23.835 | -8.017 | 0.00 | 0.00 |

| ATOM | 580 | CA | ALA | 76 | 53.818 | 23.540 | -9.422 | 0.00 | 0.00 |
| ATOM | 581 | C | ALA | 76 | 53.054 | 22.245 | -9.915 | 0.00 | 0.00 |
| ATOM | 582 | O | ALA | 76 | 52.456 | 22.310 | -10.991 | 0.00 | 0.00 |
| ATOM | 583 | CB | ALA | 76 | 55.296 | 23.583 | -9.759 | 0.00 | 0.00 |
| ATOM | 584 | N | VAL | 77 | 53.247 | 21.068 | -9.337 | 0.00 | 0.00 |
| ATOM | 585 | CA | VAL | 77 | 52.512 | 19.849 | -9.655 | 0.00 | 0.00 |
| ATOM | 586 | C | VAL | 77 | 51.286 | 19.674 | -8.775 | 0.00 | 0.00 |
| ATOM | 587 | O | VAL | 77 | 50.241 | 19.408 | -9.348 | 0.00 | 0.00 |
| ATOM | 588 | CB | VAL | 77 | 53.415 | 18.625 | -9.625 | 0.00 | 0.00 |
| ATOM | 589 | CG1 | VAL | 77 | 54.599 | 18.651 | -10.571 | 0.00 | 0.00 |
| ATOM | 590 | CG2 | VAL | 77 | 53.996 | 18.357 | -8.233 | 0.00 | 0.00 |
| ATOM | 591 | N | VAL | 78 | 51.388 | 20.001 | -7.529 | 0.00 | 0.00 |
| ATOM | 592 | CA | VAL | 78 | 50.328 | 19.783 | -6.625 | 0.00 | 0.00 |
| ATOM | 593 | C | VAL | 78 | 49.017 | 20.507 | -7.078 | 0.00 | 0.00 |
| ATOM | 594 | O | VAL | 78 | 48.057 | 19.725 | -7.315 | 0.00 | 0.00 |
| ATOM | 595 | CB | VAL | 78 | 50.717 | 19.856 | -5.170 | 0.00 | 0.00 |
| ATOM | 596 | CG1 | VAL | 78 | 49.527 | 19.543 | -4.202 | 0.00 | 0.00 |
| ATOM | 597 | CG2 | VAL | 78 | 51.960 | 19.158 | -4.787 | 0.00 | 0.00 |
| ATOM | 598 | N | PRO | 79 | 48.813 | 21.853 | -7.222 | 0.00 | 0.00 |
| ATOM | 599 | CA | PRO | 79 | 47.516 | 22.255 | -7.587 | 0.00 | 0.00 |
| ATOM | 600 | C | PRO | 79 | 47.038 | 21.553 | -8.890 | 0.00 | 0.00 |
| ATOM | 601 | O | PRO | 79 | 45.834 | 21.535 | -9.108 | 0.00 | 0.00 |
| ATOM | 602 | CB | PRO | 79 | 47.378 | 23.815 | -7.791 | 0.00 | 0.00 |
| ATOM | 603 | CG | PRO | 79 | 48.830 | 24.251 | -7.527 | 0.00 | 0.00 |
| ATOM | 604 | CD | PRO | 79 | 49.721 | 23.085 | -7.224 | 0.00 | 0.00 |

40

| | | | | | | | | | |
|---|---|---|---|---|---|---|---|---|---|
| ATOM | 605 | N | PHE | 80 | 47.929 | 21.268 | -9.810 | 0.00 | 0.00 |
| ATOM | 606 | CA | PHE | 80 | 47.668 | 20.504 | -11.022 | 0.00 | 0.00 |
| ATOM | 607 | C | PHE | 80 | 46.922 | 19.188 | -10.797 | 0.00 | 0.00 |
| ATOM | 608 | O | PHE | 80 | 45.723 | 19.187 | -11.069 | 0.00 | 0.00 |
| ATOM | 609 | CB | PHE | 80 | 48.990 | 20.376 | -11.801 | 0.00 | 0.00 |
| ATOM | 610 | CG | PHE | 80 | 49.405 | 21.419 | -12.763 | 0.00 | 0.00 |
| ATOM | 611 | CD1 | PHE | 80 | 50.305 | 21.070 | -13.793 | 0.00 | 0.00 |
| ATOM | 612 | CD2 | PHE | 80 | 48.907 | 22.720 | -12.714 | 0.00 | 0.00 |
| ATOM | 613 | CE1 | PHE | 80 | 50.746 | 22.039 | -14.671 | 0.00 | 0.00 |
| ATOM | 614 | CE2 | PHE | 80 | 49.323 | 23.694 | -13.621 | 0.00 | 0.00 |
| ATOM | 615 | CZ | PHE | 80 | 50.228 | 23.329 | -14.629 | 0.00 | 0.00 |
| ATOM | 616 | N | GLY | 81 | 47.479 | 18.285 | -10.045 | 0.00 | 0.00 |
| ATOM | 617 | CA | GLY | 81 | 46.890 | 17.015 | -9.659 | 0.00 | 0.00 |
| ATOM | 618 | C | GLY | 81 | 45.477 | 17.246 | -8.974 | 0.00 | 0.00 |
| ATOM | 619 | O | GLY | 81 | 44.649 | 16.387 | -9.158 | 0.00 | 0.00 |
| ATOM | 620 | N | ALA | 82 | 45.337 | 18.201 | -8.017 | 0.00 | 0.00 |
| ATOM | 621 | CA | ALA | 82 | 44.100 | 18.608 | -7.364 | 0.00 | 0.00 |
| ATOM | 622 | C | ALA | 82 | 42.976 | 18.981 | -8.373 | 0.00 | 0.00 |
| ATOM | 623 | O | ALA | 82 | 41.993 | 18.260 | -8.360 | 0.00 | 0.00 |
| ATOM | 624 | CB | ALA | 82 | 44.435 | 19.755 | -6.399 | 0.00 | 0.00 |
| ATOM | 625 | N | ALA | 83 | 43.136 | 19.965 | -9.268 | 0.00 | 0.00 |
| ATOM | 626 | CA | ALA | 83 | 42.141 | 20.304 | -10.312 | 0.00 | 0.00 |
| ATOM | 627 | C | ALA | 83 | 41.828 | 19.077 | -11.268 | 0.00 | 0.00 |
| ATOM | 628 | O | ALA | 83 | 40.811 | 19.103 | -11.971 | 0.00 | 0.00 |
| ATOM | 629 | CB | ALA | 83 | 42.650 | 21.550 | -11.054 | 0.00 | 0.00 |

| ATOM | 630 | N | HIS | 84 | 42.891 | 18.376 | -11.576 | 0.00 | 0.00 |
|------|-----|-----|-----|-----|--------|--------|---------|------|------|
| ATOM | 631 | CA | HIS | 84 | 42.958 | 17.165 | -12.398 | 0.00 | 0.00 |
| ATOM | 632 | C | HIS | 84 | 42.028 | 16.030 | -11.809 | 0.00 | 0.00 |
| ATOM | 633 | O | HIS | 84 | 41.235 | 15.500 | -12.565 | 0.00 | 0.00 |
| ATOM | 634 | CB | HIS | 84 | 44.382 | 16.640 | -12.463 | 0.00 | 0.00 |
| ATOM | 635 | CG | HIS | 84 | 44.760 | 15.548 | -13.463 | 0.00 | 0.00 |
| ATOM | 636 | ND1 | HIS | 84 | 44.935 | 15.646 | -14.803 | 0.00 | 0.00 |
| ATOM | 637 | CD2 | HIS | 84 | 44.994 | 14.210 | -13.119 | 0.00 | 0.00 |
| ATOM | 638 | CE1 | HIS | 84 | 45.264 | 14.440 | -15.259 | 0.00 | 0.00 |
| ATOM | 639 | NE2 | HIS | 84 | 45.296 | 13.589 | -14.240 | 0.00 | 0.00 |
| ATOM | 640 | N | ILE | 85 | 42.346 | 15.531 | -10.644 | 0.00 | 0.00 |
| ATOM | 641 | CA | ILE | 85 | 41.509 | 14.569 | -9.975 | 0.00 | 0.00 |
| ATOM | 642 | C | ILE | 85 | 40.053 | 15.091 | -9.816 | 0.00 | 0.00 |
| ATOM | 643 | O | ILE | 85 | 39.108 | 14.292 | -9.698 | 0.00 | 0.00 |
| ATOM | 644 | CB | ILE | 85 | 42.022 | 14.229 | -8.542 | 0.00 | 0.00 |
| ATOM | 645 | CG1 | ILE | 85 | 43.554 | 13.922 | -8.531 | 0.00 | 0.00 |
| ATOM | 646 | CG2 | ILE | 85 | 41.268 | 12.935 | -7.991 | 0.00 | 0.00 |
| ATOM | 647 | CD1 | ILE | 85 | 44.279 | 13.738 | -7.202 | 0.00 | 0.00 |
| ATOM | 648 | N | LEU | 86 | 39.926 | 16.424 | -9.611 | 0.00 | 0.00 |
| ATOM | 649 | CA | LEU | 86 | 38.609 | 17.112 | -9.540 | 0.00 | 0.00 |
| ATOM | 650 | C | LEU | 86 | 37.823 | 16.861 | -10.867 | 0.00 | 0.00 |
| ATOM | 651 | O | LEU | 86 | 36.620 | 16.648 | -10.742 | 0.00 | 0.00 |
| ATOM | 652 | CB | LEU | 86 | 38.735 | 18.596 | -9.181 | 0.00 | 0.00 |
| ATOM | 653 | CG | LEU | 86 | 39.164 | 18.771 | -7.733 | 0.00 | 0.00 |
| ATOM | 654 | CD1 | LEU | 86 | 39.621 | 20.240 | -7.600 | 0.00 | 0.00 |

```
ATOM    655  CD2 LEU  86      37.928  18.518   -6.870  0.00  0.00

ATOM    656  N   MET  87      38.436  16.928  -12.084  0.00  0.00

ATOM    657  CA  MET  87      37.655  16.568  -13.299  0.00  0.00

ATOM    658  C   MET  87      37.911  15.103  -13.854  0.00  0.00

ATOM    659  O   MET  87      37.087  14.667  -14.659  0.00  0.00

ATOM    660  CB  MET  87      37.960  17.651  -14.309  0.00  0.00

ATOM    661  CG  MET  87      37.282  18.980  -13.996  0.00  0.00

ATOM    662  SD  MET  87      35.620  18.906  -13.432  0.00  0.00

ATOM    663  CE  MET  87      34.771  18.066  -14.859  0.00  0.00

ATOM    664  N   LYS  88      38.614  14.263  -13.045  0.00  0.00

ATOM    665  CA  LYS  88      39.049  12.872  -13.345  0.00  0.00

ATOM    666  C   LYS  88      39.857  12.716  -14.618  0.00  0.00

ATOM    667  O   LYS  88      40.062  11.530  -15.008  0.00  0.00

ATOM    668  CB  LYS  88      37.959  11.882  -13.158  0.00  0.00

ATOM    669  CG  LYS  88      38.262  10.416  -13.094  0.00  0.00

ATOM    670  CD  LYS  88      38.756  10.031  -11.699  0.00  0.00

ATOM    671  CE  LYS  88      37.640  10.036  -10.635  0.00  0.00

ATOM    672  NZ  LYS  88      36.697   8.934  -10.803  0.00  0.00

ATOM    673  N   MET  89      40.835  13.607  -14.726  0.00  0.00

ATOM    674  CA  MET  89      41.588  13.659  -15.977  0.00  0.00

ATOM    675  C   MET  89      42.569  14.781  -16.113  0.00  0.00

ATOM    676  O   MET  89      42.388  15.689  -15.315  0.00  0.00

ATOM    677  CB  MET  89      40.620  14.185  -16.906  0.00  0.00

ATOM    678  CG  MET  89      39.791  15.253  -17.061  0.00  0.00

ATOM    679  SD  MET  89      38.239  15.215  -18.089  0.00  0.00
```

```
ATOM   680  CE  MET   89   37.447  16.632 -17.336  0.00  0.00

ATOM   681  N   TRP   90   43.046  15.075 -17.322  0.00  0.00

ATOM   682  CA  TRP   90   43.870  16.271 -17.376  0.00  0.00

ATOM   683  C   TRP   90   43.157  17.473 -18.127  0.00  0.00

ATOM   684  O   TRP   90   43.256  17.525 -19.355  0.00  0.00

ATOM   685  CB  TRP   90   45.035  15.810 -18.252  0.00  0.00

ATOM   686  CG  TRP   90   46.280  15.330 -17.500  0.00  0.00

ATOM   687  CD1 TRP   90   46.622  14.011 -17.620  0.00  0.00

ATOM   688  CD2 TRP   90   47.086  15.997 -16.588  0.00  0.00

ATOM   689  NE1 TRP   90   47.629  13.833 -16.787  0.00  0.00

ATOM   690  CE2 TRP   90   47.934  14.985 -16.163  0.00  0.00

ATOM   691  CE3 TRP   90   47.243  17.267 -16.067  0.00  0.00

ATOM   692  CZ2 TRP   90   48.911  15.211 -15.222  0.00  0.00

ATOM   693  CZ3 TRP   90   48.227  17.498 -15.122  0.00  0.00

ATOM   694  CH2 TRP   90   49.061  16.482 -14.708  0.00  0.00

ATOM   695  N   THR   91   42.186  18.199 -17.532  0.00  0.00

ATOM   696  CA  THR   91   41.525  19.409 -18.104  0.00  0.00

ATOM   697  C   THR   91   42.543  20.439 -18.731  0.00  0.00

ATOM   698  O   THR   91   42.223  20.924 -19.822  0.00  0.00

ATOM   699  CB  THR   91   40.683  20.109 -16.980  0.00  0.00

ATOM   700  OG1 THR   91   39.722  19.201 -16.374  0.00  0.00

ATOM   701  CG2 THR   91   40.033  21.465 -17.357  0.00  0.00

ATOM   702  N   PHE   92   43.801  20.601 -18.218  0.00  0.00

ATOM   703  CA  PHE   92   44.763  21.604 -18.739  0.00  0.00

ATOM   704  C   PHE   92   45.739  21.075 -19.868  0.00  0.00
```

ATOM    705  O    PHE    92    46.684   21.818  -20.202   0.00   0.00

ATOM    706  CB   PHE    92    45.489   22.310  -17.588   0.00   0.00

ATOM    707  CG   PHE    92    44.619   22.895  -16.507   0.00   0.00

ATOM    708  CD1  PHE    92    43.531   23.683  -16.857   0.00   0.00

ATOM    709  CD2  PHE    92    44.911   22.684  -15.182   0.00   0.00

ATOM    710  CE1  PHE    92    42.767   24.242  -15.861   0.00   0.00

ATOM    711  CE2  PHE    92    44.140   23.247  -14.196   0.00   0.00

ATOM    712  CZ   PHE    92    43.063   24.031  -14.529   0.00   0.00

ATOM    713  N    GLY    93    45.474   19.943  -20.558   0.00   0.00

ATOM    714  CA   GLY    93    46.323   19.501  -21.684   0.00   0.00

ATOM    715  C    GLY    93    47.591   18.760  -21.176   0.00   0.00

ATOM    716  O    GLY    93    47.600   18.306  -20.027   0.00   0.00

ATOM    717  N    ASN    94    48.385   18.193  -22.085   0.00   0.00

ATOM    718  CA   ASN    94    49.652   17.570  -21.646   0.00   0.00

ATOM    719  C    ASN    94    50.538   18.566  -20.791   0.00   0.00

ATOM    720  O    ASN    94    51.581   18.103  -20.350   0.00   0.00

ATOM    721  CB   ASN    94    50.435   17.120  -22.880   0.00   0.00

ATOM    722  CG   ASN    94    49.988   15.808  -23.443   0.00   0.00

ATOM    723  OD1  ASN    94    50.879   15.087  -23.971   0.00   0.00

ATOM    724  ND2  ASN    94    48.838   15.363  -23.064   0.00   0.00

ATOM    725  N    PHE    95    50.495   19.898  -21.086   0.00   0.00

ATOM    726  CA   PHE    95    51.204   20.883  -20.275   0.00   0.00

ATOM    727  C    PHE    95    50.808   20.740  -18.792   0.00   0.00

ATOM    728  O    PHE    95    51.649   21.040  -17.959   0.00   0.00

ATOM    729  CB   PHE    95    50.854   22.312  -20.793   0.00   0.00

| ATOM | 730 | CG  | PHE | 95 | 51.129 | 22.563 | -22.264 | 0.00 | 0.00 |
| ATOM | 731 | CD1 | PHE | 95 | 52.366 | 22.194 | -22.828 | 0.00 | 0.00 |
| ATOM | 732 | CD2 | PHE | 95 | 50.213 | 23.229 | -23.065 | 0.00 | 0.00 |
| ATOM | 733 | CE1 | PHE | 95 | 52.598 | 22.522 | -24.146 | 0.00 | 0.00 |
| ATOM | 734 | CE2 | PHE | 95 | 50.470 | 23.565 | -24.370 | 0.00 | 0.00 |
| ATOM | 735 | CZ  | PHE | 95 | 51.678 | 23.174 | -24.937 | 0.00 | 0.00 |
| ATOM | 736 | N   | TRP | 96 | 49.605 | 20.216 | -18.469 | 0.00 | 0.00 |
| ATOM | 737 | CA  | TRP | 96 | 49.185 | 20.011 | -17.147 | 0.00 | 0.00 |
| ATOM | 738 | C   | TRP | 96 | 49.967 | 18.697 | -16.740 | 0.00 | 0.00 |
| ATOM | 739 | O   | TRP | 96 | 50.722 | 18.721 | -15.744 | 0.00 | 0.00 |
| ATOM | 740 | CB  | TRP | 96 | 47.644 | 20.024 | -17.218 | 0.00 | 0.00 |
| ATOM | 741 | CG  | TRP | 96 | 47.053 | 20.008 | -15.764 | 0.00 | 0.00 |
| ATOM | 742 | CD1 | TRP | 96 | 47.715 | 20.551 | -14.701 | 0.00 | 0.00 |
| ATOM | 743 | CD2 | TRP | 96 | 45.770 | 19.628 | -15.445 | 0.00 | 0.00 |
| ATOM | 744 | NE1 | TRP | 96 | 46.862 | 20.533 | -13.727 | 0.00 | 0.00 |
| ATOM | 745 | CE2 | TRP | 96 | 45.692 | 19.997 | -14.112 | 0.00 | 0.00 |
| ATOM | 746 | CE3 | TRP | 96 | 44.686 | 19.042 | -16.056 | 0.00 | 0.00 |
| ATOM | 747 | CZ2 | TRP | 96 | 44.544 | 19.797 | -13.368 | 0.00 | 0.00 |
| ATOM | 748 | CZ3 | TRP | 96 | 43.534 | 18.843 | -15.308 | 0.00 | 0.00 |
| ATOM | 749 | CH2 | TRP | 96 | 43.461 | 19.220 | -13.971 | 0.00 | 0.00 |
| ATOM | 750 | N   | CYS | 97 | 49.987 | 17.655 | -17.604 | 0.00 | 0.00 |
| ATOM | 751 | CA  | CYS | 97 | 50.725 | 16.429 | -17.401 | 0.00 | 0.00 |
| ATOM | 752 | C   | CYS | 97 | 52.245 | 16.736 | -17.168 | 0.00 | 0.00 |
| ATOM | 753 | O   | CYS | 97 | 52.633 | 16.626 | -16.014 | 0.00 | 0.00 |
| ATOM | 754 | CB  | CYS | 97 | 50.428 | 15.492 | -18.525 | 0.00 | 0.00 |

| ATOM | 755 | SG | CYS | 97 | 51.381 | 13.975 | -18.578 | 0.00 | 0.00 |
| ATOM | 756 | N | GLU | 98 | 52.879 | 17.602 | -17.987 | 0.00 | 0.00 |
| ATOM | 757 | CA | GLU | 98 | 54.245 | 18.070 | -17.865 | 0.00 | 0.00 |
| ATOM | 758 | C | GLU | 98 | 54.545 | 18.732 | -16.505 | 0.00 | 0.00 |
| ATOM | 759 | O | GLU | 98 | 55.623 | 18.429 | -15.996 | 0.00 | 0.00 |
| ATOM | 760 | CB | GLU | 98 | 54.557 | 19.032 | -19.023 | 0.00 | 0.00 |
| ATOM | 761 | CG | GLU | 98 | 54.864 | 18.271 | -20.301 | 0.00 | 0.00 |
| ATOM | 762 | CD | GLU | 98 | 54.979 | 19.029 | -21.579 | 0.00 | 0.00 |
| ATOM | 763 | OE1 | GLU | 98 | 55.744 | 18.638 | -22.463 | 0.00 | 0.00 |
| ATOM | 764 | OE2 | GLU | 98 | 54.173 | 19.993 | -21.663 | 0.00 | 0.00 |
| ATOM | 765 | N | PHE | 99 | 53.811 | 19.759 | -16.056 | 0.00 | 0.00 |
| ATOM | 766 | CA | PHE | 99 | 54.094 | 20.249 | -14.742 | 0.00 | 0.00 |
| ATOM | 767 | C | PHE | 99 | 53.884 | 19.218 | -13.604 | 0.00 | 0.00 |
| ATOM | 768 | O | PHE | 99 | 54.907 | 18.768 | -13.170 | 0.00 | 0.00 |
| ATOM | 769 | CB | PHE | 99 | 53.285 | 21.488 | -14.493 | 0.00 | 0.00 |
| ATOM | 770 | CG | PHE | 99 | 53.606 | 22.791 | -15.057 | 0.00 | 0.00 |
| ATOM | 771 | CD1 | PHE | 99 | 52.961 | 23.287 | -16.189 | 0.00 | 0.00 |
| ATOM | 772 | CD2 | PHE | 99 | 54.631 | 23.594 | -14.505 | 0.00 | 0.00 |
| ATOM | 773 | CE1 | PHE | 99 | 53.274 | 24.513 | -16.781 | 0.00 | 0.00 |
| ATOM | 774 | CE2 | PHE | 99 | 54.965 | 24.803 | -15.088 | 0.00 | 0.00 |
| ATOM | 775 | CZ | PHE | 99 | 54.280 | 25.277 | -16.208 | 0.00 | 0.00 |
| ATOM | 776 | N | TRP | 100 | 52.784 | 18.419 | -13.607 | 0.00 | 0.00 |
| ATOM | 777 | CA | TRP | 100 | 52.538 | 17.527 | -12.479 | 0.00 | 0.00 |
| ATOM | 778 | C | TRP | 100 | 53.638 | 16.415 | -12.443 | 0.00 | 0.00 |
| ATOM | 779 | O | TRP | 100 | 53.840 | 15.885 | -11.364 | 0.00 | 0.00 |

| | | | | | | | | | |
|---|---|---|---|---|---|---|---|---|---|
| ATOM | 780 | CB | TRP | 100 | 51.061 | 17.129 | -12.291 | 0.00 | 0.00 |
| ATOM | 781 | CG | TRP | 100 | 50.775 | 15.985 | -11.326 | 0.00 | 0.00 |
| ATOM | 782 | CD1 | TRP | 100 | 50.257 | 14.801 | -11.764 | 0.00 | 0.00 |
| ATOM | 783 | CD2 | TRP | 100 | 50.829 | 16.054 | -9.952 | 0.00 | 0.00 |
| ATOM | 784 | NE1 | TRP | 100 | 49.964 | 14.125 | -10.688 | 0.00 | 0.00 |
| ATOM | 785 | CE2 | TRP | 100 | 50.280 | 14.818 | -9.587 | 0.00 | 0.00 |
| ATOM | 786 | CE3 | TRP | 100 | 51.240 | 16.960 | -8.964 | 0.00 | 0.00 |
| ATOM | 787 | CZ2 | TRP | 100 | 50.135 | 14.476 | -8.242 | 0.00 | 0.00 |
| ATOM | 788 | CZ3 | TRP | 100 | 51.096 | 16.612 | -7.617 | 0.00 | 0.00 |
| ATOM | 789 | CH2 | TRP | 100 | 50.547 | 15.379 | -7.259 | 0.00 | 0.00 |
| ATOM | 790 | N | THR | 101 | 54.096 | 15.831 | -13.586 | 0.00 | 0.00 |
| ATOM | 791 | CA | THR | 101 | 55.199 | 14.829 | -13.645 | 0.00 | 0.00 |
| ATOM | 792 | C | THR | 101 | 56.541 | 15.514 | -13.136 | 0.00 | 0.00 |
| ATOM | 793 | O | THR | 101 | 57.270 | 14.858 | -12.411 | 0.00 | 0.00 |
| ATOM | 794 | CB | THR | 101 | 55.487 | 14.330 | -15.114 | 0.00 | 0.00 |
| ATOM | 795 | OG1 | THR | 101 | 56.617 | 14.807 | -15.774 | 0.00 | 0.00 |
| ATOM | 796 | CG2 | THR | 101 | 54.249 | 14.399 | -16.034 | 0.00 | 0.00 |
| ATOM | 797 | N | SER | 102 | 56.977 | 16.562 | -13.891 | 0.00 | 0.00 |
| ATOM | 798 | CA | SER | 102 | 58.230 | 17.288 | -13.695 | 0.00 | 0.00 |
| ATOM | 799 | C | SER | 102 | 58.493 | 17.726 | -12.252 | 0.00 | 0.00 |
| ATOM | 800 | O | SER | 102 | 59.643 | 18.049 | -12.004 | 0.00 | 0.00 |
| ATOM | 801 | CB | SER | 102 | 58.290 | 18.541 | -14.597 | 0.00 | 0.00 |
| ATOM | 802 | OG | SER | 102 | 57.326 | 19.604 | -14.314 | 0.00 | 0.00 |
| ATOM | 803 | N | ILE | 103 | 57.498 | 18.194 | -11.504 | 0.00 | 0.00 |
| ATOM | 804 | CA | ILE | 103 | 57.805 | 18.592 | -10.129 | 0.00 | 0.00 |

```
ATOM    805  C    ILE   103     57.642  17.333   -9.192  0.00  0.00

ATOM    806  O    ILE   103     58.307  17.340   -8.200  0.00  0.00

ATOM    807  CB   ILE   103     56.927  19.793   -9.660  0.00  0.00

ATOM    808  CG1  ILE   103     56.867  21.005  -10.559  0.00  0.00

ATOM    809  CG2  ILE   103     57.038  20.140   -8.128  0.00  0.00

ATOM    810  CD1  ILE   103     58.118  21.867  -10.467  0.00  0.00

ATOM    811  N    ASP   104     56.814  16.307   -9.549  0.00  0.00

ATOM    812  CA   ASP   104     56.778  15.101   -8.699  0.00  0.00

ATOM    813  C    ASP   104     58.226  14.467   -8.795  0.00  0.00

ATOM    814  O    ASP   104     58.541  13.694   -7.856  0.00  0.00

ATOM    815  CB   ASP   104     55.674  14.064   -9.083  0.00  0.00

ATOM    816  CG   ASP   104     56.166  12.599   -9.100  0.00  0.00

ATOM    817  OD1  ASP   104     57.343  12.261   -8.980  0.00  0.00

ATOM    818  OD2  ASP   104     56.699  12.249  -10.146  0.00  0.00

ATOM    819  N    VAL   105     58.786  14.256   -9.999  0.00  0.00

ATOM    820  CA   VAL   105     60.153  13.771  -10.254  0.00  0.00

ATOM    821  C    VAL   105     61.193  14.809   -9.731  0.00  0.00

ATOM    822  O    VAL   105     62.197  14.345   -9.219  0.00  0.00

ATOM    823  CB   VAL   105     60.394  13.461  -11.753  0.00  0.00

ATOM    824  CG1  VAL   105     61.719  12.731  -12.116  0.00  0.00

ATOM    825  CG2  VAL   105     59.213  12.710  -12.348  0.00  0.00

ATOM    826  N    LEU   106     61.182  16.101  -10.177  0.00  0.00

ATOM    827  CA   LEU   106     62.138  17.132   -9.695  0.00  0.00

ATOM    828  C    LEU   106     62.276  17.070   -8.133  0.00  0.00

ATOM    829  O    LEU   106     63.369  17.421   -7.669  0.00  0.00
```

```
ATOM    830  CB   LEU   106      61.697  18.542 -10.136   0.00   0.00

ATOM    831  CG   LEU   106      62.609  19.695  -9.718   0.00   0.00

ATOM    832  CD1  LEU   106      63.952  19.564 -10.420   0.00   0.00

ATOM    833  CD2  LEU   106      61.976  21.041 -10.005   0.00   0.00

ATOM    834  N    CYS   107      61.177  17.064  -7.378   0.00   0.00

ATOM    835  CA   CYS   107      61.166  16.951  -5.934   0.00   0.00

ATOM    836  C    CYS   107      61.858  15.619  -5.442   0.00   0.00

ATOM    837  O    CYS   107      62.426  15.683  -4.358   0.00   0.00

ATOM    838  CB   CYS   107      59.715  17.074  -5.552   0.00   0.00

ATOM    839  SG   CYS   107      58.552  15.718  -5.932   0.00   0.00

ATOM    840  N    VAL   108      61.441  14.446  -5.908   0.00   0.00

ATOM    841  CA   VAL   108      61.990  13.169  -5.539   0.00   0.00

ATOM    842  C    VAL   108      63.511  13.138  -5.808   0.00   0.00

ATOM    843  O    VAL   108      64.173  12.308  -5.153   0.00   0.00

ATOM    844  CB   VAL   108      61.216  12.105  -6.342   0.00   0.00

ATOM    845  CG1  VAL   108      61.931  10.740  -6.173   0.00   0.00

ATOM    846  CG2  VAL   108      59.787  11.961  -5.804   0.00   0.00

ATOM    847  N    THR   109      63.825  13.360  -7.095   0.00   0.00

ATOM    848  CA   THR   109      65.172  13.475  -7.545   0.00   0.00

ATOM    849  C    THR   109      65.960  14.396  -6.661   0.00   0.00

ATOM    850  O    THR   109      67.131  14.080  -6.469   0.00   0.00

ATOM    851  CB   THR   109      65.313  13.934  -9.044   0.00   0.00

ATOM    852  OG1  THR   109      64.849  15.322  -9.254   0.00   0.00

ATOM    853  CG2  THR   109      64.839  12.860 -10.004   0.00   0.00

ATOM    854  N    ALA   110      65.516  15.665  -6.497   0.00   0.00
```

ATOM    855  CA   ALA   110    66.155  16.591  -5.576  0.00  0.00

ATOM    856  C    ALA   110    66.255  15.873  -4.194  0.00  0.00

ATOM    857  O    ALA   110    67.276  16.076  -3.556  0.00  0.00

ATOM    858  CB   ALA   110    65.408  17.935  -5.515  0.00  0.00

ATOM    859  N    SER   111    65.236  15.077  -3.746  0.00  0.00

ATOM    860  CA   SER   111    65.227  14.269  -2.529  0.00  0.00

ATOM    861  C    SER   111    66.295  13.114  -2.570  0.00  0.00

ATOM    862  O    SER   111    66.920  12.929  -1.525  0.00  0.00

ATOM    863  CB   SER   111    63.818  13.726  -2.274  0.00  0.00

ATOM    864  OG   SER   111    62.863  14.656  -1.786  0.00  0.00

ATOM    865  N    ILE   112    66.285  12.165  -3.551  0.00  0.00

ATOM    866  CA   ILE   112    67.334  11.114  -3.636  0.00  0.00

ATOM    867  C    ILE   112    68.779  11.694  -3.829  0.00  0.00

ATOM    868  O    ILE   112    69.693  11.056  -3.308  0.00  0.00

ATOM    869  CB   ILE   112    66.898  10.050  -4.695  0.00  0.00

ATOM    870  CG1  ILE   112    67.645   8.707  -4.422  0.00  0.00

ATOM    871  CG2  ILE   112    67.059  10.589  -6.130  0.00  0.00

ATOM    872  CD1  ILE   112    67.444   7.643  -5.511  0.00  0.00

ATOM    873  N    GLU   113    69.000  12.773  -4.601  0.00  0.00

ATOM    874  CA   GLU   113    70.311  13.372  -4.746  0.00  0.00

ATOM    875  C    GLU   113    70.751  14.076  -3.406  0.00  0.00

ATOM    876  O    GLU   113    71.967  14.174  -3.217  0.00  0.00

ATOM    877  CB   GLU   113    70.229  14.416  -5.878  0.00  0.00

ATOM    878  CG   GLU   113    70.477  13.968  -7.305  0.00  0.00

ATOM    879  CD   GLU   113    71.163  12.635  -7.313  0.00  0.00

| ATOM | 880 | OE1 | GLU | 113 | 70.737 | 11.633 | -7.846 | 0.00 | 0.00 |
|------|-----|-----|-----|-----|--------|--------|--------|------|------|
| ATOM | 881 | OE2 | GLU | 113 | 72.203 | 12.723 | -6.630 | 0.00 | 0.00 |
| ATOM | 882 | N   | THR | 114 | 69.871 | 14.816 | -2.700 | 0.00 | 0.00 |
| ATOM | 883 | CA  | THR | 114 | 70.150 | 15.420 | -1.399 | 0.00 | 0.00 |
| ATOM | 884 | C   | THR | 114 | 70.643 | 14.297 | -0.442 | 0.00 | 0.00 |
| ATOM | 885 | O   | THR | 114 | 71.465 | 14.642 | 0.413  | 0.00 | 0.00 |
| ATOM | 886 | CB  | THR | 114 | 68.951 | 16.274 | -0.884 | 0.00 | 0.00 |
| ATOM | 887 | OG1 | THR | 114 | 69.270 | 17.185 | 0.202  | 0.00 | 0.00 |
| ATOM | 888 | CG2 | THR | 114 | 67.600 | 15.671 | -0.696 | 0.00 | 0.00 |
| ATOM | 889 | N   | LEU | 115 | 69.983 | 13.126 | -0.388 | 0.00 | 0.00 |
| ATOM | 890 | CA  | LEU | 115 | 70.445 | 11.994 | 0.402  | 0.00 | 0.00 |
| ATOM | 891 | C   | LEU | 115 | 71.899 | 11.624 | -0.004 | 0.00 | 0.00 |
| ATOM | 892 | O   | LEU | 115 | 72.579 | 11.041 | 0.858  | 0.00 | 0.00 |
| ATOM | 893 | CB  | LEU | 115 | 69.493 | 10.830 | 0.166  | 0.00 | 0.00 |
| ATOM | 894 | CG  | LEU | 115 | 68.152 | 10.983 | 0.836  | 0.00 | 0.00 |
| ATOM | 895 | CD1 | LEU | 115 | 67.237 | 9.771  | 0.651  | 0.00 | 0.00 |
| ATOM | 896 | CD2 | LEU | 115 | 68.250 | 11.198 | 2.346  | 0.00 | 0.00 |
| ATOM | 897 | N   | CYS | 116 | 72.286 | 11.660 | -1.293 | 0.00 | 0.00 |
| ATOM | 898 | CA  | CYS | 116 | 73.668 | 11.408 | -1.755 | 0.00 | 0.00 |
| ATOM | 899 | C   | CYS | 116 | 74.596 | 12.440 | -1.068 | 0.00 | 0.00 |
| ATOM | 900 | O   | CYS | 116 | 75.490 | 11.995 | -0.365 | 0.00 | 0.00 |
| ATOM | 901 | CB  | CYS | 116 | 73.797 | 11.419 | -3.275 | 0.00 | 0.00 |
| ATOM | 902 | SG  | CYS | 116 | 75.530 | 11.348 | -3.777 | 0.00 | 0.00 |
| ATOM | 903 | N   | VAL | 117 | 74.299 | 13.684 | -1.141 | 0.00 | 0.00 |
| ATOM | 904 | CA  | VAL | 117 | 75.167 | 14.619 | -0.418 | 0.00 | 0.00 |

| | | | | | | | | | |
|---|---|---|---|---|---|---|---|---|---|
| ATOM | 905 | C | VAL | 117 | 75.291 | 14.292 | 1.060 | 0.00 | 0.00 |
| ATOM | 906 | O | VAL | 117 | 76.414 | 14.462 | 1.571 | 0.00 | 0.00 |
| ATOM | 907 | CB | VAL | 117 | 74.769 | 16.096 | -0.607 | 0.00 | 0.00 |
| ATOM | 908 | CG1 | VAL | 117 | 75.598 | 17.039 | 0.227 | 0.00 | 0.00 |
| ATOM | 909 | CG2 | VAL | 117 | 74.706 | 16.504 | -2.058 | 0.00 | 0.00 |
| ATOM | 910 | N | ILE | 118 | 74.148 | 14.231 | 1.752 | 0.00 | 0.00 |
| ATOM | 911 | CA | ILE | 118 | 74.120 | 13.901 | 3.157 | 0.00 | 0.00 |
| ATOM | 912 | C | ILE | 118 | 74.972 | 12.690 | 3.547 | 0.00 | 0.00 |
| ATOM | 913 | O | ILE | 118 | 75.595 | 12.812 | 4.610 | 0.00 | 0.00 |
| ATOM | 914 | CB | ILE | 118 | 72.633 | 13.622 | 3.493 | 0.00 | 0.00 |
| ATOM | 915 | CG1 | ILE | 118 | 71.823 | 14.882 | 3.577 | 0.00 | 0.00 |
| ATOM | 916 | CG2 | ILE | 118 | 72.533 | 12.995 | 4.913 | 0.00 | 0.00 |
| ATOM | 917 | CD1 | ILE | 118 | 70.275 | 14.737 | 3.570 | 0.00 | 0.00 |
| ATOM | 918 | N | ALA | 119 | 74.753 | 11.501 | 2.984 | 0.00 | 0.00 |
| ATOM | 919 | CA | ALA | 119 | 75.521 | 10.310 | 3.193 | 0.00 | 0.00 |
| ATOM | 920 | C | ALA | 119 | 77.043 | 10.591 | 3.206 | 0.00 | 0.00 |
| ATOM | 921 | O | ALA | 119 | 77.679 | 10.162 | 4.163 | 0.00 | 0.00 |
| ATOM | 922 | CB | ALA | 119 | 75.108 | 9.254 | 2.142 | 0.00 | 0.00 |
| ATOM | 923 | N | VAL | 120 | 77.635 | 11.167 | 2.145 | 0.00 | 0.00 |
| ATOM | 924 | CA | VAL | 120 | 79.070 | 11.568 | 2.079 | 0.00 | 0.00 |
| ATOM | 925 | C | VAL | 120 | 79.413 | 12.579 | 3.207 | 0.00 | 0.00 |
| ATOM | 926 | O | VAL | 120 | 80.571 | 12.565 | 3.614 | 0.00 | 0.00 |
| ATOM | 927 | CB | VAL | 120 | 79.427 | 12.188 | 0.717 | 0.00 | 0.00 |
| ATOM | 928 | CG1 | VAL | 120 | 80.872 | 12.662 | 0.544 | 0.00 | 0.00 |
| ATOM | 929 | CG2 | VAL | 120 | 79.086 | 11.320 | -0.468 | 0.00 | 0.00 |

| ATOM | 930 | N | ASP | 121 | 78.549 | 13.579 | 3.461 | 0.00 | 0.00 |
| ATOM | 931 | CA | ASP | 121 | 78.766 | 14.500 | 4.557 | 0.00 | 0.00 |
| ATOM | 932 | C | ASP | 121 | 78.988 | 13.688 | 5.883 | 0.00 | 0.00 |
| ATOM | 933 | O | ASP | 121 | 79.942 | 14.049 | 6.576 | 0.00 | 0.00 |
| ATOM | 934 | CB | ASP | 121 | 77.635 | 15.519 | 4.591 | 0.00 | 0.00 |
| ATOM | 935 | CG | ASP | 121 | 77.951 | 16.731 | 5.370 | 0.00 | 0.00 |
| ATOM | 936 | OD1 | ASP | 121 | 79.163 | 16.803 | 5.650 | 0.00 | 0.00 |
| ATOM | 937 | OD2 | ASP | 121 | 77.082 | 17.535 | 5.712 | 0.00 | 0.00 |
| ATOM | 938 | N | ARG | 122 | 78.040 | 12.839 | 6.355 | 0.00 | 0.00 |
| ATOM | 939 | CA | ARG | 122 | 78.207 | 11.978 | 7.529 | 0.00 | 0.00 |
| ATOM | 940 | C | ARG | 122 | 79.440 | 11.028 | 7.420 | 0.00 | 0.00 |
| ATOM | 941 | O | ARG | 122 | 79.798 | 10.489 | 8.459 | 0.00 | 0.00 |
| ATOM | 942 | CB | ARG | 122 | 76.924 | 11.348 | 8.039 | 0.00 | 0.00 |
| ATOM | 943 | CG | ARG | 122 | 75.687 | 12.159 | 8.261 | 0.00 | 0.00 |
| ATOM | 944 | CD | ARG | 122 | 75.843 | 13.430 | 9.133 | 0.00 | 0.00 |
| ATOM | 945 | NE | ARG | 122 | 76.220 | 14.653 | 8.409 | 0.00 | 0.00 |
| ATOM | 946 | CZ | ARG | 122 | 75.826 | 15.885 | 8.799 | 0.00 | 0.00 |
| ATOM | 947 | NH1 | ARG | 122 | 75.074 | 16.078 | 9.884 | 0.00 | 0.00 |
| ATOM | 948 | NH2 | ARG | 122 | 76.189 | 16.966 | 8.101 | 0.00 | 0.00 |
| ATOM | 949 | N | TYR | 123 | 79.859 | 10.558 | 6.204 | 0.00 | 0.00 |
| ATOM | 950 | CA | TYR | 123 | 81.049 | 9.753 | 5.968 | 0.00 | 0.00 |
| ATOM | 951 | C | TYR | 123 | 82.288 | 10.614 | 6.317 | 0.00 | 0.00 |
| ATOM | 952 | O | TYR | 123 | 82.879 | 10.316 | 7.360 | 0.00 | 0.00 |
| ATOM | 953 | CB | TYR | 123 | 81.120 | 9.261 | 4.533 | 0.00 | 0.00 |
| ATOM | 954 | CG | TYR | 123 | 82.321 | 8.407 | 4.234 | 0.00 | 0.00 |

| ATOM | 955 | CD1 | TYR | 123 | 82.330 | 7.060 | 4.602 | 0.00 | 0.00 |
|------|-----|-----|-----|-----|--------|-------|-------|------|------|
| ATOM | 956 | CD2 | TYR | 123 | 83.456 | 8.936 | 3.622 | 0.00 | 0.00 |
| ATOM | 957 | CE1 | TYR | 123 | 83.431 | 6.260 | 4.379 | 0.00 | 0.00 |
| ATOM | 958 | CE2 | TYR | 123 | 84.564 | 8.142 | 3.390 | 0.00 | 0.00 |
| ATOM | 959 | CZ | TYR | 123 | 84.543 | 6.802 | 3.773 | 0.00 | 0.00 |
| ATOM | 960 | OH | TYR | 123 | 85.632 | 5.992 | 3.549 | 0.00 | 0.00 |
| ATOM | 961 | N | PHE | 124 | 82.526 | 11.773 | 5.665 | 0.00 | 0.00 |
| ATOM | 962 | CA | PHE | 124 | 83.617 | 12.690 | 5.992 | 0.00 | 0.00 |
| ATOM | 963 | C | PHE | 124 | 83.653 | 13.056 | 7.515 | 0.00 | 0.00 |
| ATOM | 964 | O | PHE | 124 | 84.752 | 12.987 | 8.077 | 0.00 | 0.00 |
| ATOM | 965 | CB | PHE | 124 | 83.501 | 13.938 | 5.138 | 0.00 | 0.00 |
| ATOM | 966 | CG | PHE | 124 | 83.784 | 13.818 | 3.671 | 0.00 | 0.00 |
| ATOM | 967 | CD1 | PHE | 124 | 84.853 | 13.053 | 3.210 | 0.00 | 0.00 |
| ATOM | 968 | CD2 | PHE | 124 | 83.013 | 14.534 | 2.770 | 0.00 | 0.00 |
| ATOM | 969 | CE1 | PHE | 124 | 85.154 | 12.994 | 1.841 | 0.00 | 0.00 |
| ATOM | 970 | CE2 | PHE | 124 | 83.269 | 14.475 | 1.386 | 0.00 | 0.00 |
| ATOM | 971 | CZ | PHE | 124 | 84.334 | 13.673 | 0.940 | 0.00 | 0.00 |
| ATOM | 972 | N | ALA | 125 | 82.551 | 13.485 | 8.129 | 0.00 | 0.00 |
| ATOM | 973 | CA | ALA | 125 | 82.450 | 13.854 | 9.541 | 0.00 | 0.00 |
| ATOM | 974 | C | ALA | 125 | 82.577 | 12.638 | 10.541 | 0.00 | 0.00 |
| ATOM | 975 | O | ALA | 125 | 83.512 | 12.706 | 11.351 | 0.00 | 0.00 |
| ATOM | 976 | CB | ALA | 125 | 81.124 | 14.613 | 9.708 | 0.00 | 0.00 |
| ATOM | 977 | N | ILE | 126 | 81.724 | 11.612 | 10.503 | 0.00 | 0.00 |
| ATOM | 978 | CA | ILE | 126 | 81.675 | 10.489 | 11.428 | 0.00 | 0.00 |
| ATOM | 979 | C | ILE | 126 | 82.649 | 9.309 | 11.082 | 0.00 | 0.00 |

```
ATOM    980   O    ILE   126      83.173    8.760   12.042   0.00   0.00

ATOM    981   CB   ILE   126      80.185   10.023   11.506   0.00   0.00

ATOM    982   CG1  ILE   126      79.238   11.203   11.707   0.00   0.00

ATOM    983   CG2  ILE   126      79.967    8.876   12.534   0.00   0.00

ATOM    984   CD1  ILE   126      77.798   10.883   11.185   0.00   0.00

ATOM    985   N    THR   127      82.737    8.759    9.833   0.00   0.00

ATOM    986   CA   THR   127      83.734    7.704    9.468   0.00   0.00

ATOM    987   C    THR   127      85.221    8.238    9.633   0.00   0.00

ATOM    988   O    THR   127      86.114    7.399    9.803   0.00   0.00

ATOM    989   CB   THR   127      83.404    7.037    8.085   0.00   0.00

ATOM    990   OG1  THR   127      82.073    6.422    8.089   0.00   0.00

ATOM    991   CG2  THR   127      84.462    5.941    7.748   0.00   0.00

ATOM    992   N    SER   128      85.346    9.526   10.025   0.00   0.00

ATOM    993   CA   SER   128      86.559   10.230   10.268   0.00   0.00

ATOM    994   C    SER   128      87.536   10.270    9.077   0.00   0.00

ATOM    995   O    SER   128      88.728   10.428    9.357   0.00   0.00

ATOM    996   CB   SER   128      87.230    9.596   11.492   0.00   0.00

ATOM    997   OG   SER   128      86.518    9.491   12.695   0.00   0.00

ATOM    998   N    PRO   129      87.124   10.335    7.774   0.00   0.00

ATOM    999   CA   PRO   129      88.092   10.475    6.707   0.00   0.00

ATOM   1000   C    PRO   129      88.773   11.905    6.768   0.00   0.00

ATOM   1001   O    PRO   129      88.502   12.723    7.646   0.00   0.00

ATOM   1002   CB   PRO   129      87.433   10.181    5.307   0.00   0.00

ATOM   1003   CG   PRO   129      85.933   10.303    5.665   0.00   0.00

ATOM   1004   CD   PRO   129      85.739   10.164    7.155   0.00   0.00
```

```
ATOM   1005  N   PHE   130      89.412  12.196  5.637  0.00  0.00

ATOM   1006  CA  PHE   130      90.233  13.323  5.403  0.00  0.00

ATOM   1007  C   PHE   130      91.324  13.284  6.513  0.00  0.00

ATOM   1008  O   PHE   130      91.857  12.160  6.716  0.00  0.00

ATOM   1009  CB  PHE   130      89.441  14.572  5.319  0.00  0.00

ATOM   1010  CG  PHE   130      89.016  15.047  4.005  0.00  0.00

ATOM   1011  CD1 PHE   130      88.232  14.217  3.216  0.00  0.00

ATOM   1012  CD2 PHE   130      89.337  16.313  3.521  0.00  0.00

ATOM   1013  CE1 PHE   130      87.765  14.601  1.970  0.00  0.00

ATOM   1014  CE2 PHE   130      88.886  16.741  2.273  0.00  0.00

ATOM   1015  CZ  PHE   130      88.103  15.876  1.515  0.00  0.00

ATOM   1016  N   LYS   131      91.942  14.333  6.873  0.00  0.00

ATOM   1017  CA  LYS   131      92.906  14.276  7.987  0.00  0.00

ATOM   1018  C   LYS   131      93.203  15.687  8.526  0.00  0.00

ATOM   1019  O   LYS   131      93.553  15.750  9.718  0.00  0.00

ATOM   1020  CB  LYS   131      94.165  13.458  7.631  0.00  0.00

ATOM   1021  CG  LYS   131      95.046  13.905  6.493  0.00  0.00

ATOM   1022  CD  LYS   131      96.315  13.128  6.784  0.00  0.00

ATOM   1023  CE  LYS   131      97.259  13.103  5.596  0.00  0.00

ATOM   1024  NZ  LYS   131      98.543  12.573  6.028  0.00  0.00

ATOM   1025  N   TYR   132      93.659  16.525  7.543  0.00  0.00

ATOM   1026  CA  TYR   132      94.073  17.831  7.924  0.00  0.00

ATOM   1027  C   TYR   132      92.842  18.705  8.212  0.00  0.00

ATOM   1028  O   TYR   132      92.453  19.571  7.419  0.00  0.00

ATOM   1029  CB  TYR   132      94.985  18.438  6.842  0.00  0.00
```

| ATOM | 1030 | CG | TYR | 132 | 95.949 | 19.482 | 7.410 | 0.00 | 0.00 |
| ATOM | 1031 | CD1 | TYR | 132 | 95.872 | 20.813 | 6.970 | 0.00 | 0.00 |
| ATOM | 1032 | CD2 | TYR | 132 | 96.943 | 19.210 | 8.365 | 0.00 | 0.00 |
| ATOM | 1033 | CE1 | TYR | 132 | 96.770 | 21.807 | 7.381 | 0.00 | 0.00 |
| ATOM | 1034 | CE2 | TYR | 132 | 97.853 | 20.179 | 8.801 | 0.00 | 0.00 |
| ATOM | 1035 | CZ | TYR | 132 | 97.779 | 21.491 | 8.302 | 0.00 | 0.00 |
| ATOM | 1036 | OH | TYR | 132 | 98.676 | 22.453 | 8.660 | 0.00 | 0.00 |
| ATOM | 1037 | N | GLN | 133 | 92.138 | 18.248 | 9.240 | 0.00 | 0.00 |
| ATOM | 1038 | CA | GLN | 133 | 91.018 | 18.854 | 9.828 | 0.00 | 0.00 |
| ATOM | 1039 | C | GLN | 133 | 89.976 | 19.469 | 8.797 | 0.00 | 0.00 |
| ATOM | 1040 | O | GLN | 133 | 89.541 | 20.605 | 9.064 | 0.00 | 0.00 |
| ATOM | 1041 | CB | GLN | 133 | 91.574 | 19.853 | 10.849 | 0.00 | 0.00 |
| ATOM | 1042 | CG | GLN | 133 | 92.221 | 21.074 | 10.257 | 0.00 | 0.00 |
| ATOM | 1043 | CD | GLN | 133 | 92.996 | 21.792 | 11.345 | 0.00 | 0.00 |
| ATOM | 1044 | OE1 | GLN | 133 | 93.344 | 22.968 | 11.218 | 0.00 | 0.00 |
| ATOM | 1045 | NE2 | GLN | 133 | 93.336 | 21.157 | 12.454 | 0.00 | 0.00 |
| ATOM | 1046 | N | SER | 134 | 89.390 | 18.713 | 7.853 | 0.00 | 0.00 |
| ATOM | 1047 | CA | SER | 134 | 88.367 | 19.342 | 6.961 | 0.00 | 0.00 |
| ATOM | 1048 | C | SER | 134 | 86.948 | 19.406 | 7.645 | 0.00 | 0.00 |
| ATOM | 1049 | O | SER | 134 | 86.437 | 18.338 | 8.020 | 0.00 | 0.00 |
| ATOM | 1050 | CB | SER | 134 | 88.185 | 18.534 | 5.679 | 0.00 | 0.00 |
| ATOM | 1051 | OG | SER | 134 | 87.903 | 17.158 | 5.817 | 0.00 | 0.00 |
| ATOM | 1052 | N | LEU | 135 | 86.343 | 20.593 | 7.908 | 0.00 | 0.00 |
| ATOM | 1053 | CA | LEU | 135 | 84.986 | 20.686 | 8.449 | 0.00 | 0.00 |
| ATOM | 1054 | C | LEU | 135 | 83.978 | 21.632 | 7.663 | 0.00 | 0.00 |

| ATOM | 1055 | O | LEU | 135 | 84.002 | 22.856 | 7.858 | 0.00 | 0.00 |
| ATOM | 1056 | CB | LEU | 135 | 85.059 | 21.148 | 9.908 | 0.00 | 0.00 |
| ATOM | 1057 | CG | LEU | 135 | 83.661 | 21.190 | 10.628 | 0.00 | 0.00 |
| ATOM | 1058 | CD1 | LEU | 135 | 83.021 | 19.795 | 10.637 | 0.00 | 0.00 |
| ATOM | 1059 | CD2 | LEU | 135 | 83.842 | 21.695 | 12.048 | 0.00 | 0.00 |
| ATOM | 1060 | N | LEU | 136 | 83.400 | 21.126 | 6.543 | 0.00 | 0.00 |
| ATOM | 1061 | CA | LEU | 136 | 82.356 | 21.714 | 5.654 | 0.00 | 0.00 |
| ATOM | 1062 | C | LEU | 136 | 82.240 | 23.262 | 5.735 | 0.00 | 0.00 |
| ATOM | 1063 | O | LEU | 136 | 83.253 | 23.882 | 5.435 | 0.00 | 0.00 |
| ATOM | 1064 | CB | LEU | 136 | 81.075 | 21.036 | 5.993 | 0.00 | 0.00 |
| ATOM | 1065 | CG | LEU | 136 | 80.619 | 19.641 | 5.823 | 0.00 | 0.00 |
| ATOM | 1066 | CD1 | LEU | 136 | 79.383 | 19.419 | 6.657 | 0.00 | 0.00 |
| ATOM | 1067 | CD2 | LEU | 136 | 80.358 | 19.272 | 4.384 | 0.00 | 0.00 |
| ATOM | 1068 | N | THR | 137 | 81.030 | 23.867 | 5.532 | 0.00 | 0.00 |
| ATOM | 1069 | CA | THR | 137 | 80.694 | 25.318 | 5.666 | 0.00 | 0.00 |
| ATOM | 1070 | C | THR | 137 | 79.420 | 25.517 | 4.839 | 0.00 | 0.00 |
| ATOM | 1071 | O | THR | 137 | 78.557 | 24.637 | 4.730 | 0.00 | 0.00 |
| ATOM | 1072 | CB | THR | 137 | 81.795 | 26.387 | 5.306 | 0.00 | 0.00 |
| ATOM | 1073 | OG1 | THR | 137 | 82.122 | 26.317 | 3.857 | 0.00 | 0.00 |
| ATOM | 1074 | CG2 | THR | 137 | 82.912 | 26.327 | 6.306 | 0.00 | 0.00 |
| ATOM | 1075 | N | LYS | 138 | 79.086 | 26.774 | 4.709 | 0.00 | 0.00 |
| ATOM | 1076 | CA | LYS | 138 | 77.993 | 27.296 | 3.904 | 0.00 | 0.00 |
| ATOM | 1077 | C | LYS | 138 | 78.325 | 26.967 | 2.391 | 0.00 | 0.00 |
| ATOM | 1078 | O | LYS | 138 | 77.375 | 26.719 | 1.664 | 0.00 | 0.00 |
| ATOM | 1079 | CB | LYS | 138 | 78.023 | 28.822 | 4.135 | 0.00 | 0.00 |

| ATOM | 1080 | CG | LYS | 138 | 77.173 | 29.184 | 5.353 | 0.00 | 0.00 |
|------|------|-----|-----|-----|--------|--------|--------|------|------|
| ATOM | 1081 | CD | LYS | 138 | 76.874 | 30.674 | 5.437 | 0.00 | 0.00 |
| ATOM | 1082 | CE | LYS | 138 | 76.812 | 31.202 | 6.901 | 0.00 | 0.00 |
| ATOM | 1083 | NZ | LYS | 138 | 78.150 | 31.653 | 7.456 | 0.00 | 0.00 |
| ATOM | 1084 | N | ASN | 139 | 79.511 | 27.430 | 1.899 | 0.00 | 0.00 |
| ATOM | 1085 | CA | ASN | 139 | 79.969 | 27.231 | 0.550 | 0.00 | 0.00 |
| ATOM | 1086 | C | ASN | 139 | 79.905 | 25.729 | 0.037 | 0.00 | 0.00 |
| ATOM | 1087 | O | ASN | 139 | 79.597 | 25.547 | -1.139 | 0.00 | 0.00 |
| ATOM | 1088 | CB | ASN | 139 | 81.236 | 28.015 | 0.183 | 0.00 | 0.00 |
| ATOM | 1089 | CG | ASN | 139 | 82.349 | 27.691 | 1.208 | 0.00 | 0.00 |
| ATOM | 1090 | OD1 | ASN | 139 | 82.352 | 28.376 | 2.259 | 0.00 | 0.00 |
| ATOM | 1091 | ND2 | ASN | 139 | 83.199 | 26.710 | 0.999 | 0.00 | 0.00 |
| ATOM | 1092 | N | LYS | 140 | 80.445 | 24.709 | 0.754 | 0.00 | 0.00 |
| ATOM | 1093 | CA | LYS | 140 | 80.326 | 23.249 | 0.435 | 0.00 | 0.00 |
| ATOM | 1094 | C | LYS | 140 | 78.793 | 22.868 | 0.384 | 0.00 | 0.00 |
| ATOM | 1095 | O | LYS | 140 | 78.457 | 22.067 | -0.465 | 0.00 | 0.00 |
| ATOM | 1096 | CB | LYS | 140 | 81.089 | 22.373 | 1.426 | 0.00 | 0.00 |
| ATOM | 1097 | CG | LYS | 140 | 82.495 | 22.623 | 1.885 | 0.00 | 0.00 |
| ATOM | 1098 | CD | LYS | 140 | 83.473 | 21.961 | 0.898 | 0.00 | 0.00 |
| ATOM | 1099 | CE | LYS | 140 | 84.897 | 22.455 | 1.072 | 0.00 | 0.00 |
| ATOM | 1100 | NZ | LYS | 140 | 85.704 | 21.656 | 0.070 | 0.00 | 0.00 |
| ATOM | 1101 | N | ALA | 141 | 77.998 | 23.281 | 1.381 | 0.00 | 0.00 |
| ATOM | 1102 | CA | ALA | 141 | 76.555 | 23.120 | 1.464 | 0.00 | 0.00 |
| ATOM | 1103 | C | ALA | 141 | 75.877 | 23.676 | 0.146 | 0.00 | 0.00 |
| ATOM | 1104 | O | ALA | 141 | 74.754 | 23.247 | -0.092 | 0.00 | 0.00 |

```
ATOM   1105  CB   ALA   141      76.040  23.850   2.716  0.00  0.00

ATOM   1106  N    ARG   142      76.258  24.862  -0.403  0.00  0.00

ATOM   1107  CA   ARG   142      75.758  25.401  -1.671  0.00  0.00

ATOM   1108  C    ARG   142      76.288  24.480  -2.833  0.00  0.00

ATOM   1109  O    ARG   142      75.710  24.586  -3.910  0.00  0.00

ATOM   1110  CB   ARG   142      76.189  26.877  -1.904  0.00  0.00

ATOM   1111  CG   ARG   142      75.685  27.464  -3.272  0.00  0.00

ATOM   1112  CD   ARG   142      74.172  27.451  -3.325  0.00  0.00

ATOM   1113  NE   ARG   142      73.773  28.183  -4.523  0.00  0.00

ATOM   1114  CZ   ARG   142      73.578  29.502  -4.551  0.00  0.00

ATOM   1115  NH1  ARG   142      73.721  30.238  -3.451  0.00  0.00

ATOM   1116  NH2  ARG   142      73.242  30.048  -5.718  0.00  0.00

ATOM   1117  N    VAL   143      77.539  23.956  -2.810  0.00  0.00

ATOM   1118  CA   VAL   143      78.058  23.010  -3.803  0.00  0.00

ATOM   1119  C    VAL   143      77.146  21.756  -3.867  0.00  0.00

ATOM   1120  O    VAL   143      77.005  21.265  -4.966  0.00  0.00

ATOM   1121  CB   VAL   143      79.529  22.650  -3.522  0.00  0.00

ATOM   1122  CG1  VAL   143      79.992  21.496  -4.403  0.00  0.00

ATOM   1123  CG2  VAL   143      80.444  23.863  -3.618  0.00  0.00

ATOM   1124  N    ILE   144      76.784  21.121  -2.753  0.00  0.00

ATOM   1125  CA   ILE   144      75.871  20.018  -2.668  0.00  0.00

ATOM   1126  C    ILE   144      74.396  20.377  -3.069  0.00  0.00

ATOM   1127  O    ILE   144      73.710  19.462  -3.507  0.00  0.00

ATOM   1128  CB   ILE   144      76.030  19.338  -1.262  0.00  0.00

ATOM   1129  CG1  ILE   144      75.955  20.321  -0.084  0.00  0.00
```

61

```
ATOM   1130  CG2 ILE   144      77.319  18.476  -1.197  0.00  0.00

ATOM   1131  CD1 ILE   144      75.655  19.620   1.286  0.00  0.00

ATOM   1132  N   ILE   145      73.790  21.504  -2.598  0.00  0.00

ATOM   1133  CA  ILE   145      72.468  21.974  -2.985  0.00  0.00

ATOM   1134  C   ILE   145      72.537  22.281  -4.534  0.00  0.00

ATOM   1135  O   ILE   145      71.513  22.105  -5.192  0.00  0.00

ATOM   1136  CB  ILE   145      71.867  23.141  -2.122  0.00  0.00

ATOM   1137  CG1 ILE   145      71.196  22.653  -0.784  0.00  0.00

ATOM   1138  CG2 ILE   145      70.718  23.933  -2.859  0.00  0.00

ATOM   1139  CD1 ILE   145      70.965  23.783   0.260  0.00  0.00

ATOM   1140  N   LEU   146      73.496  23.088  -5.044  0.00  0.00

ATOM   1141  CA  LEU   146      73.700  23.348  -6.479  0.00  0.00

ATOM   1142  C   LEU   146      73.816  21.999  -7.245  0.00  0.00

ATOM   1143  O   LEU   146      73.073  21.828  -8.213  0.00  0.00

ATOM   1144  CB  LEU   146      74.906  24.256  -6.738  0.00  0.00

ATOM   1145  CG  LEU   146      75.209  24.584  -8.196  0.00  0.00

ATOM   1146  CD1 LEU   146      74.077  25.426  -8.764  0.00  0.00

ATOM   1147  CD2 LEU   146      76.540  25.290  -8.348  0.00  0.00

ATOM   1148  N   MET   147      74.654  21.048  -6.790  0.00  0.00

ATOM   1149  CA  MET   147      74.786  19.722  -7.338  0.00  0.00

ATOM   1150  C   MET   147      73.445  18.924  -7.294  0.00  0.00

ATOM   1151  O   MET   147      73.185  18.247  -8.295  0.00  0.00

ATOM   1152  CB  MET   147      75.928  19.018  -6.589  0.00  0.00

ATOM   1153  CG  MET   147      75.850  17.518  -6.809  0.00  0.00

ATOM   1154  SD  MET   147      77.118  16.743  -5.821  0.00  0.00
```

```
ATOM   1155  CE   MET   147      78.101  16.256   -7.187   0.00   0.00

ATOM   1156  N    VAL   148      72.669  18.911   -6.190   0.00   0.00

ATOM   1157  CA   VAL   148      71.389  18.229   -6.189   0.00   0.00

ATOM   1158  C    VAL   148      70.479  18.726   -7.366   0.00   0.00

ATOM   1159  O    VAL   148      69.728  17.883   -7.844   0.00   0.00

ATOM   1160  CB   VAL   148      70.720  18.239   -4.802   0.00   0.00

ATOM   1161  CG1  VAL   148      71.533  17.505   -3.740   0.00   0.00

ATOM   1162  CG2  VAL   148      70.359  19.674   -4.394   0.00   0.00

ATOM   1163  N    TRP   149      70.187  20.036   -7.496   0.00   0.00

ATOM   1164  CA   TRP   149      69.448  20.603   -8.599   0.00   0.00

ATOM   1165  C    TRP   149      69.992  20.141   -9.985   0.00   0.00

ATOM   1166  O    TRP   149      69.160  20.098  -10.899   0.00   0.00

ATOM   1167  CB   TRP   149      69.479  22.113   -8.499   0.00   0.00

ATOM   1168  CG   TRP   149      68.602  22.778   -7.514   0.00   0.00

ATOM   1169  CD1  TRP   149      68.840  22.953   -6.178   0.00   0.00

ATOM   1170  CD2  TRP   149      67.319  23.365   -7.777   0.00   0.00

ATOM   1171  NE1  TRP   149      67.784  23.629   -5.572   0.00   0.00

ATOM   1172  CE2  TRP   149      66.848  23.888   -6.545   0.00   0.00

ATOM   1173  CE3  TRP   149      66.547  23.515   -8.924   0.00   0.00

ATOM   1174  CZ2  TRP   149      65.624  24.541   -6.444   0.00   0.00

ATOM   1175  CZ3  TRP   149      65.324  24.146   -8.825   0.00   0.00

ATOM   1176  CH2  TRP   149      64.878  24.659   -7.615   0.00   0.00

ATOM   1177  N    ILE   150      71.311  20.225  -10.275   0.00   0.00

ATOM   1178  CA   ILE   150      71.825  19.705  -11.512   0.00   0.00

ATOM   1179  C    ILE   150      71.385  18.232  -11.749   0.00   0.00
```

| ATOM | 1180 | O | ILE | 150 | 71.025 | 17.944 | -12.889 | 0.00 | 0.00 |
|------|------|------|------|------|--------|--------|---------|------|------|
| ATOM | 1181 | CB | ILE | 150 | 73.385 | 19.823 | -11.568 | 0.00 | 0.00 |
| ATOM | 1182 | CG1 | ILE | 150 | 73.924 | 21.225 | -11.346 | 0.00 | 0.00 |
| ATOM | 1183 | CG2 | ILE | 150 | 73.951 | 19.323 | -12.935 | 0.00 | 0.00 |
| ATOM | 1184 | CD1 | ILE | 150 | 75.439 | 21.137 | -11.044 | 0.00 | 0.00 |
| ATOM | 1185 | N | VAL | 151 | 71.712 | 17.257 | -10.882 | 0.00 | 0.00 |
| ATOM | 1186 | CA | VAL | 151 | 71.310 | 15.871 | -11.121 | 0.00 | 0.00 |
| ATOM | 1187 | C | VAL | 151 | 69.751 | 15.692 | -11.153 | 0.00 | 0.00 |
| ATOM | 1188 | O | VAL | 151 | 69.326 | 14.763 | -11.854 | 0.00 | 0.00 |
| ATOM | 1189 | CB | VAL | 151 | 71.990 | 14.872 | -10.198 | 0.00 | 0.00 |
| ATOM | 1190 | CG1 | VAL | 151 | 71.490 | 13.431 | -10.275 | 0.00 | 0.00 |
| ATOM | 1191 | CG2 | VAL | 151 | 73.478 | 15.016 | -10.190 | 0.00 | 0.00 |
| ATOM | 1192 | N | SER | 152 | 68.958 | 16.244 | -10.213 | 0.00 | 0.00 |
| ATOM | 1193 | CA | SER | 152 | 67.514 | 16.173 | -10.293 | 0.00 | 0.00 |
| ATOM | 1194 | C | SER | 152 | 66.999 | 16.703 | -11.659 | 0.00 | 0.00 |
| ATOM | 1195 | O | SER | 152 | 65.934 | 16.247 | -12.084 | 0.00 | 0.00 |
| ATOM | 1196 | CB | SER | 152 | 67.021 | 17.108 | -9.211 | 0.00 | 0.00 |
| ATOM | 1197 | OG | SER | 152 | 67.112 | 18.506 | -9.380 | 0.00 | 0.00 |
| ATOM | 1198 | N | GLY | 153 | 67.488 | 17.903 | -12.070 | 0.00 | 0.00 |
| ATOM | 1199 | CA | GLY | 153 | 67.223 | 18.518 | -13.331 | 0.00 | 0.00 |
| ATOM | 1200 | C | GLY | 153 | 67.565 | 17.555 | -14.478 | 0.00 | 0.00 |
| ATOM | 1201 | O | GLY | 153 | 66.837 | 17.561 | -15.460 | 0.00 | 0.00 |
| ATOM | 1202 | N | LEU | 154 | 68.752 | 16.944 | -14.485 | 0.00 | 0.00 |
| ATOM | 1203 | CA | LEU | 154 | 69.164 | 15.935 | -15.451 | 0.00 | 0.00 |
| ATOM | 1204 | C | LEU | 154 | 68.039 | 14.851 | -15.634 | 0.00 | 0.00 |

```
ATOM    1205   O    LEU   154      67.363  14.957 -16.641   0.00   0.00

ATOM    1206   CB   LEU   154      70.432  15.388 -14.907   0.00   0.00

ATOM    1207   CG   LEU   154      71.298  14.293 -15.421   0.00   0.00

ATOM    1208   CD1  LEU   154      70.537  12.958 -15.552   0.00   0.00

ATOM    1209   CD2  LEU   154      71.873  14.658 -16.799   0.00   0.00

ATOM    1210   N    THR   155      67.541  14.182 -14.559   0.00   0.00

ATOM    1211   CA   THR   155      66.568  13.105 -14.633   0.00   0.00

ATOM    1212   C    THR   155      65.176  13.583 -15.212   0.00   0.00

ATOM    1213   O    THR   155      64.808  13.101 -16.278   0.00   0.00

ATOM    1214   CB   THR   155      66.472  12.515 -13.196   0.00   0.00

ATOM    1215   OG1  THR   155      67.702  11.884 -12.752   0.00   0.00

ATOM    1216   CG2  THR   155      65.282  11.510 -13.166   0.00   0.00

ATOM    1217   N    SER   156      64.526  14.641 -14.694   0.00   0.00

ATOM    1218   CA   SER   156      63.173  15.097 -15.101   0.00   0.00

ATOM    1219   C    SER   156      63.145  16.097 -16.300   0.00   0.00

ATOM    1220   O    SER   156      62.032  16.247 -16.850   0.00   0.00

ATOM    1221   CB   SER   156      62.543  15.725 -13.848   0.00   0.00

ATOM    1222   OG   SER   156      63.112  16.913 -13.318   0.00   0.00

ATOM    1223   N    PHE   157      64.010  17.121 -16.337   0.00   0.00

ATOM    1224   CA   PHE   157      64.070  18.017 -17.525   0.00   0.00

ATOM    1225   C    PHE   157      63.996  17.212 -18.847   0.00   0.00

ATOM    1226   O    PHE   157      63.479  17.799 -19.820   0.00   0.00

ATOM    1227   CB   PHE   157      65.284  18.948 -17.417   0.00   0.00

ATOM    1228   CG   PHE   157      65.486  19.799 -18.667   0.00   0.00

ATOM    1229   CD1  PHE   157      64.673  20.896 -18.875   0.00   0.00
```

| ATOM | 1230 | CD2 | PHE | 157 | 66.464 | 19.489 | -19.591 | 0.00 | 0.00 |
|------|------|-----|-----|-----|--------|--------|---------|------|------|
| ATOM | 1231 | CE1 | PHE | 157 | 64.842 | 21.664 | -20.000 | 0.00 | 0.00 |
| ATOM | 1232 | CE2 | PHE | 157 | 66.625 | 20.269 | -20.720 | 0.00 | 0.00 |
| ATOM | 1233 | CZ | PHE | 157 | 65.810 | 21.357 | -20.926 | 0.00 | 0.00 |
| ATOM | 1234 | N | LEU | 158 | 64.568 | 16.006 | -18.947 | 0.00 | 0.00 |
| ATOM | 1235 | CA | LEU | 158 | 64.627 | 15.178 | -20.116 | 0.00 | 0.00 |
| ATOM | 1236 | C | LEU | 158 | 63.267 | 14.919 | -20.769 | 0.00 | 0.00 |
| ATOM | 1237 | O | LEU | 158 | 63.162 | 15.293 | -21.931 | 0.00 | 0.00 |
| ATOM | 1238 | CB | LEU | 158 | 65.386 | 13.916 | -19.870 | 0.00 | 0.00 |
| ATOM | 1239 | CG | LEU | 158 | 66.636 | 13.880 | -19.076 | 0.00 | 0.00 |
| ATOM | 1240 | CD1 | LEU | 158 | 66.946 | 12.438 | -18.637 | 0.00 | 0.00 |
| ATOM | 1241 | CD2 | LEU | 158 | 67.724 | 14.481 | -19.912 | 0.00 | 0.00 |
| ATOM | 1242 | N | PRO | 159 | 62.236 | 14.264 | -20.152 | 0.00 | 0.00 |
| ATOM | 1243 | CA | PRO | 159 | 61.055 | 14.057 | -20.917 | 0.00 | 0.00 |
| ATOM | 1244 | C | PRO | 159 | 60.487 | 15.351 | -21.586 | 0.00 | 0.00 |
| ATOM | 1245 | O | PRO | 159 | 59.764 | 15.170 | -22.537 | 0.00 | 0.00 |
| ATOM | 1246 | CB | PRO | 159 | 60.030 | 13.299 | -20.015 | 0.00 | 0.00 |
| ATOM | 1247 | CG | PRO | 159 | 60.466 | 13.762 | -18.573 | 0.00 | 0.00 |
| ATOM | 1248 | CD | PRO | 159 | 61.922 | 14.162 | -18.675 | 0.00 | 0.00 |
| ATOM | 1249 | N | ILE | 160 | 60.340 | 16.503 | -20.887 | 0.00 | 0.00 |
| ATOM | 1250 | CA | ILE | 160 | 59.812 | 17.704 | -21.609 | 0.00 | 0.00 |
| ATOM | 1251 | C | ILE | 160 | 60.605 | 17.968 | -22.926 | 0.00 | 0.00 |
| ATOM | 1252 | O | ILE | 160 | 59.955 | 17.977 | -23.969 | 0.00 | 0.00 |
| ATOM | 1253 | CB | ILE | 160 | 59.797 | 19.021 | -20.762 | 0.00 | 0.00 |
| ATOM | 1254 | CG1 | ILE | 160 | 58.789 | 18.889 | -19.608 | 0.00 | 0.00 |

```
ATOM  1255  CG2  ILE  160      59.482  20.267  -21.672  0.00  0.00

ATOM  1256  CD1  ILE  160      58.618  20.116  -18.691  0.00  0.00

ATOM  1257  N    GLN  161      61.950  18.049  -22.922  0.00  0.00

ATOM  1258  CA   GLN  161      62.810  18.298  -24.090  0.00  0.00

ATOM  1259  C    GLN  161      63.031  17.005  -24.918  0.00  0.00

ATOM  1260  O    GLN  161      63.983  16.256  -24.683  0.00  0.00

ATOM  1261  CB   GLN  161      64.119  18.937  -23.632  0.00  0.00

ATOM  1262  CG   GLN  161      64.938  19.448  -24.835  0.00  0.00

ATOM  1263  CD   GLN  161      66.422  19.469  -24.639  0.00  0.00

ATOM  1264  OE1  GLN  161      66.997  19.469  -23.554  0.00  0.00

ATOM  1265  NE2  GLN  161      67.121  19.459  -25.807  0.00  0.00

ATOM  1266  N    MET  162      62.348  17.028  -26.053  0.00  0.00

ATOM  1267  CA   MET  162      62.245  15.948  -27.040  0.00  0.00

ATOM  1268  C    MET  162      61.839  14.598  -26.367  0.00  0.00

ATOM  1269  O    MET  162      61.861  13.576  -27.070  0.00  0.00

ATOM  1270  CB   MET  162      63.552  15.847  -27.838  0.00  0.00

ATOM  1271  CG   MET  162      63.986  17.239  -28.345  0.00  0.00

ATOM  1272  SD   MET  162      63.029  17.571  -29.840  0.00  0.00

ATOM  1273  CE   MET  162      63.690  19.114  -30.489  0.00  0.00

ATOM  1274  N    HIS  163      61.170  14.678  -25.195  0.00  0.00

ATOM  1275  CA   HIS  163      60.629  13.543  -24.484  0.00  0.00

ATOM  1276  C    HIS  163      61.816  12.526  -24.347  0.00  0.00

ATOM  1277  O    HIS  163      61.816  11.656  -25.276  0.00  0.00

ATOM  1278  CB   HIS  163      59.452  13.084  -25.324  0.00  0.00

ATOM  1279  CG   HIS  163      58.194  13.821  -24.989  0.00  0.00
```

```
ATOM    1280  ND1 HIS    163        57.029  13.256 -24.501  0.00  0.00

ATOM    1281  CD2 HIS    163        57.950  15.161 -25.091  0.00  0.00

ATOM    1282  CE1 HIS    163        56.120  14.244 -24.400  0.00  0.00

ATOM    1283  NE2 HIS    163        56.639  15.400 -24.679  0.00  0.00

ATOM    1284  N   TRP    164        63.022  12.908 -23.827  0.00  0.00

ATOM    1285  CA  TRP    164        63.883  11.795 -23.609  0.00  0.00

ATOM    1286  C   TRP    164        62.859  10.988 -22.729  0.00  0.00

ATOM    1287  O   TRP    164        62.382  11.526 -21.720  0.00  0.00

ATOM    1288  CB  TRP    164        65.221  12.146 -22.981  0.00  0.00

ATOM    1289  CG  TRP    164        66.215  11.049 -23.091  0.00  0.00

ATOM    1290  CD1 TRP    164        66.175   9.966 -23.933  0.00  0.00

ATOM    1291  CD2 TRP    164        67.394  10.895 -22.291  0.00  0.00

ATOM    1292  NE1 TRP    164        67.249   9.139 -23.689  0.00  0.00

ATOM    1293  CE2 TRP    164        68.013   9.684 -22.688  0.00  0.00

ATOM    1294  CE3 TRP    164        67.982  11.653 -21.269  0.00  0.00

ATOM    1295  CZ2 TRP    164        69.189   9.216 -22.094  0.00  0.00

ATOM    1296  CZ3 TRP    164        69.142  11.188 -20.681  0.00  0.00

ATOM    1297  CH2 TRP    164        69.737   9.978 -21.094  0.00  0.00

ATOM    1298  N   TYR    165        62.251   9.990 -23.297  0.00  0.00

ATOM    1299  CA  TYR    165        61.150   9.255 -22.749  0.00  0.00

ATOM    1300  C   TYR    165        59.969  10.319 -22.761  0.00  0.00

ATOM    1301  O   TYR    165        59.694  10.799 -23.849  0.00  0.00

ATOM    1302  CB  TYR    165        61.378   8.508 -21.415  0.00  0.00

ATOM    1303  CG  TYR    165        61.070   9.315 -20.200  0.00  0.00

ATOM    1304  CD1 TYR    165        59.816   9.147 -19.601  0.00  0.00
```

```
ATOM   1305  CD2 TYR   165      62.084  10.139 -19.637  0.00   0.00

ATOM   1306  CE1 TYR   165      59.522   9.856 -18.427  0.00   0.00

ATOM   1307  CE2 TYR   165      61.758  10.818 -18.415  0.00   0.00

ATOM   1308  CZ  TYR   165      60.502  10.672 -17.872  0.00   0.00

ATOM   1309  OH  TYR   165      60.189  11.329 -16.710  0.00   0.00

ATOM   1310  N   ARG   166      59.031  10.400 -21.769  0.00   0.00

ATOM   1311  CA  ARG   166      57.887  11.357 -21.841  0.00   0.00

ATOM   1312  C   ARG   166      57.049  11.484 -20.508  0.00   0.00

ATOM   1313  O   ARG   166      56.975  10.518 -19.742  0.00   0.00

ATOM   1314  CB  ARG   166      56.910  10.805 -22.914  0.00   0.00

ATOM   1315  CG  ARG   166      56.518   9.394 -22.681  0.00   0.00

ATOM   1316  CD  ARG   166      55.152   8.979 -23.067  0.00   0.00

ATOM   1317  NE  ARG   166      55.003   8.503 -24.402  0.00   0.00

ATOM   1318  CZ  ARG   166      54.167   7.507 -24.678  0.00   0.00

ATOM   1319  NH1 ARG   166      53.443   6.865 -23.764  0.00   0.00

ATOM   1320  NH2 ARG   166      53.933   7.240 -25.955  0.00   0.00

ATOM   1321  N   ALA   167      56.192  12.498 -20.476  0.00   0.00

ATOM   1322  CA  ALA   167      55.243  12.749 -19.394  0.00   0.00

ATOM   1323  C   ALA   167      53.833  12.230 -19.848  0.00   0.00

ATOM   1324  O   ALA   167      53.296  12.701 -20.854  0.00   0.00

ATOM   1325  CB  ALA   167      55.233  14.257 -19.132  0.00   0.00

ATOM   1326  N   THR   168      53.444  11.045 -19.340  0.00   0.00

ATOM   1327  CA  THR   168      52.130  10.421 -19.544  0.00   0.00

ATOM   1328  C   THR   168      51.461  10.222 -18.126  0.00   0.00

ATOM   1329  O   THR   168      52.117   9.675 -17.232  0.00   0.00
```

69

| ATOM | 1330 | CB | THR | 168 | 52.268 | 9.125 | -20.420 | 0.00 | 0.00 |
| ATOM | 1331 | OG1 | THR | 168 | 51.005 | 8.631 | -20.938 | 0.00 | 0.00 |
| ATOM | 1332 | CG2 | THR | 168 | 53.002 | 7.973 | -19.687 | 0.00 | 0.00 |
| ATOM | 1333 | N | HIS | 169 | 50.156 | 10.039 | -18.162 | 0.00 | 0.00 |
| ATOM | 1334 | CA | HIS | 169 | 49.234 | 9.927 | -16.992 | 0.00 | 0.00 |
| ATOM | 1335 | C | HIS | 169 | 49.137 | 8.493 | -16.322 | 0.00 | 0.00 |
| ATOM | 1336 | O | HIS | 169 | 49.429 | 7.520 | -17.025 | 0.00 | 0.00 |
| ATOM | 1337 | CB | HIS | 169 | 47.835 | 10.257 | -17.561 | 0.00 | 0.00 |
| ATOM | 1338 | CG | HIS | 169 | 47.359 | 9.347 | -18.666 | 0.00 | 0.00 |
| ATOM | 1339 | ND1 | HIS | 169 | 47.572 | 9.655 | -20.008 | 0.00 | 0.00 |
| ATOM | 1340 | CD2 | HIS | 169 | 46.724 | 8.148 | -18.647 | 0.00 | 0.00 |
| ATOM | 1341 | CE1 | HIS | 169 | 47.077 | 8.671 | -20.738 | 0.00 | 0.00 |
| ATOM | 1342 | NE2 | HIS | 169 | 46.569 | 7.764 | -19.940 | 0.00 | 0.00 |
| ATOM | 1343 | N | GLN | 170 | 48.562 | 8.340 | -15.097 | 0.00 | 0.00 |
| ATOM | 1344 | CA | GLN | 170 | 48.363 | 6.995 | -14.497 | 0.00 | 0.00 |
| ATOM | 1345 | C | GLN | 170 | 46.822 | 6.648 | -14.459 | 0.00 | 0.00 |
| ATOM | 1346 | O | GLN | 170 | 46.415 | 5.923 | -15.362 | 0.00 | 0.00 |
| ATOM | 1347 | CB | GLN | 170 | 49.011 | 6.969 | -13.160 | 0.00 | 0.00 |
| ATOM | 1348 | CG | GLN | 170 | 48.706 | 8.057 | -12.229 | 0.00 | 0.00 |
| ATOM | 1349 | CD | GLN | 170 | 49.500 | 7.898 | -10.966 | 0.00 | 0.00 |
| ATOM | 1350 | OE1 | GLN | 170 | 50.636 | 8.378 | -10.871 | 0.00 | 0.00 |
| ATOM | 1351 | NE2 | GLN | 170 | 48.938 | 7.170 | -9.997 | 0.00 | 0.00 |
| ATOM | 1352 | N | GLU | 171 | 45.994 | 6.953 | -13.416 | 0.00 | 0.00 |
| ATOM | 1353 | CA | GLU | 171 | 44.504 | 6.819 | -13.444 | 0.00 | 0.00 |
| ATOM | 1354 | C | GLU | 171 | 43.985 | 8.168 | -14.008 | 0.00 | 0.00 |

| ATOM | 1355 | O | GLU | 171 | 42.909 | 8.639 | -13.617 | 0.00 | 0.00 |
| ATOM | 1356 | CB | GLU | 171 | 43.995 | 6.773 | -12.015 | 0.00 | 0.00 |
| ATOM | 1357 | CG | GLU | 171 | 44.527 | 7.725 | -10.986 | 0.00 | 0.00 |
| ATOM | 1358 | CD | GLU | 171 | 43.952 | 7.627 | -9.671 | 0.00 | 0.00 |
| ATOM | 1359 | OE1 | GLU | 171 | 44.714 | 8.195 | -8.888 | 0.00 | 0.00 |
| ATOM | 1360 | OE2 | GLU | 171 | 42.809 | 7.181 | -9.196 | 0.00 | 0.00 |
| ATOM | 1361 | N | ALA | 172 | 44.476 | 8.513 | -15.167 | 0.00 | 0.00 |
| ATOM | 1362 | CA | ALA | 172 | 44.180 | 9.811 | -15.750 | 0.00 | 0.00 |
| ATOM | 1363 | C | ALA | 172 | 44.234 | 10.923 | -14.615 | 0.00 | 0.00 |
| ATOM | 1364 | O | ALA | 172 | 43.353 | 11.810 | -14.677 | 0.00 | 0.00 |
| ATOM | 1365 | CB | ALA | 172 | 42.820 | 9.727 | -16.461 | 0.00 | 0.00 |
| ATOM | 1366 | N | ILE | 173 | 45.089 | 10.783 | -13.624 | 0.00 | 0.00 |
| ATOM | 1367 | CA | ILE | 173 | 45.277 | 11.664 | -12.455 | 0.00 | 0.00 |
| ATOM | 1368 | C | ILE | 173 | 46.743 | 12.104 | -12.232 | 0.00 | 0.00 |
| ATOM | 1369 | O | ILE | 173 | 46.971 | 13.200 | -11.640 | 0.00 | 0.00 |
| ATOM | 1370 | CB | ILE | 173 | 44.658 | 11.116 | -11.140 | 0.00 | 0.00 |
| ATOM | 1371 | CG1 | ILE | 173 | 43.103 | 11.105 | -11.296 | 0.00 | 0.00 |
| ATOM | 1372 | CG2 | ILE | 173 | 45.045 | 11.857 | -9.849 | 0.00 | 0.00 |
| ATOM | 1373 | CD1 | ILE | 173 | 42.433 | 10.015 | -10.434 | 0.00 | 0.00 |
| ATOM | 1374 | N | ASN | 174 | 47.753 | 11.415 | -12.843 | 0.00 | 0.00 |
| ATOM | 1375 | CA | ASN | 174 | 49.105 | 11.798 | -12.562 | 0.00 | 0.00 |
| ATOM | 1376 | C | ASN | 174 | 50.016 | 11.334 | -13.707 | 0.00 | 0.00 |
| ATOM | 1377 | O | ASN | 174 | 50.269 | 10.139 | -13.819 | 0.00 | 0.00 |
| ATOM | 1378 | CB | ASN | 174 | 49.544 | 11.002 | -11.302 | 0.00 | 0.00 |
| ATOM | 1379 | CG | ASN | 174 | 50.988 | 10.972 | -10.923 | 0.00 | 0.00 |

```
ATOM   1380   OD1 ASN   174      51.828  10.527 -11.731  0.00   0.00

ATOM   1381   ND2 ASN   174      51.368  11.437  -9.752  0.00   0.00

ATOM   1382   N   CYS   175      50.950  12.176 -13.938  0.00   0.00

ATOM   1383   CA  CYS   175      52.003  11.899 -14.937  0.00   0.00

ATOM   1384   C   CYS   175      53.397  11.625 -14.346  0.00   0.00

ATOM   1385   O   CYS   175      53.787  12.104 -13.275  0.00   0.00

ATOM   1386   CB  CYS   175      52.018  12.948 -16.035  0.00   0.00

ATOM   1387   SG  CYS   175      50.563  13.014 -17.024  0.00   0.00

ATOM   1388   N   TYR   176      54.137  10.840 -15.158  0.00   0.00

ATOM   1389   CA  TYR   176      55.496  10.410 -14.930  0.00   0.00

ATOM   1390   C   TYR   176      55.920   9.579 -16.213  0.00   0.00

ATOM   1391   O   TYR   176      55.703  10.032 -17.355  0.00   0.00

ATOM   1392   CB  TYR   176      55.671   9.762 -13.508  0.00   0.00

ATOM   1393   CG  TYR   176      57.129   9.219 -13.443  0.00   0.00

ATOM   1394   CD1 TYR   176      58.136  10.146 -13.197  0.00   0.00

ATOM   1395   CD2 TYR   176      57.445   7.863 -13.509  0.00   0.00

ATOM   1396   CE1 TYR   176      59.455   9.730 -13.015  0.00   0.00

ATOM   1397   CE2 TYR   176      58.766   7.439 -13.331  0.00   0.00

ATOM   1398   CZ  TYR   176      59.774   8.373 -13.078  0.00   0.00

ATOM   1399   OH  TYR   176      61.088   7.975 -12.850  0.00   0.00

ATOM   1400   N   ALA   177      56.821   8.590 -16.032  0.00   0.00

ATOM   1401   CA  ALA   177      57.395   7.772 -17.032  0.00   0.00

ATOM   1402   C   ALA   177      56.322   7.156 -18.000  0.00   0.00

ATOM   1403   O   ALA   177      55.290   6.672 -17.536  0.00   0.00

ATOM   1404   CB  ALA   177      58.252   6.729 -16.295  0.00   0.00
```

72

```
ATOM  1405  N   ASN  178    56.870   6.642 -19.090  0.00  0.00

ATOM  1406  CA  ASN  178    56.211   6.151 -20.336  0.00  0.00

ATOM  1407  C   ASN  178    55.139   4.979 -20.379  0.00  0.00

ATOM  1408  O   ASN  178    53.979   5.333 -20.553  0.00  0.00

ATOM  1409  CB  ASN  178    57.336   5.907 -21.279  0.00  0.00

ATOM  1410  CG  ASN  178    57.867   4.418 -21.201  0.00  0.00

ATOM  1411  OD1 ASN  178    57.617   3.541 -22.027  0.00  0.00

ATOM  1412  ND2 ASN  178    58.600   4.106 -20.140  0.00  0.00

ATOM  1413  N   GLU  179    55.461   3.669 -20.530  0.00  0.00

ATOM  1414  CA  GLU  179    54.462   2.575 -20.747  0.00  0.00

ATOM  1415  C   GLU  179    54.183   2.215 -22.255  0.00  0.00

ATOM  1416  O   GLU  179    53.412   1.262 -22.476  0.00  0.00

ATOM  1417  CB  GLU  179    53.138   2.788 -19.965  0.00  0.00

ATOM  1418  CG  GLU  179    53.288   2.758 -18.428  0.00  0.00

ATOM  1419  CD  GLU  179    51.951   2.966 -17.833  0.00  0.00

ATOM  1420  OE1 GLU  179    50.905   3.254 -18.403  0.00  0.00

ATOM  1421  OE2 GLU  179    51.914   2.573 -16.610  0.00  0.00

ATOM  1422  N   THR  180    54.961   2.704 -23.239  0.00  0.00

ATOM  1423  CA  THR  180    54.877   2.372 -24.659  0.00  0.00

ATOM  1424  C   THR  180    56.327   2.461 -25.191  0.00  0.00

ATOM  1425  O   THR  180    57.014   3.468 -24.913  0.00  0.00

ATOM  1426  CB  THR  180    53.925   3.295 -25.476  0.00  0.00

ATOM  1427  OG1 THR  180    54.490   4.644 -25.636  0.00  0.00

ATOM  1428  CG2 THR  180    52.513   3.389 -24.894  0.00  0.00

ATOM  1429  N   CYS  181    56.792   1.423 -25.905  0.00  0.00
```

```
ATOM   1430   CA   CYS   181    58.126   1.502 -26.519  0.00  0.00

ATOM   1431   C    CYS   181    58.362   2.935 -27.116  0.00  0.00

ATOM   1432   O    CYS   181    59.325   3.554 -26.652  0.00  0.00

ATOM   1433   CB   CYS   181    58.313   0.370 -27.547  0.00  0.00

ATOM   1434   SG   CYS   181    59.819   0.712 -28.515  0.00  0.00

ATOM   1435   N    CYS   182    57.463   3.513 -27.919  0.00  0.00

ATOM   1436   CA   CYS   182    57.591   4.869 -28.411  0.00  0.00

ATOM   1437   C    CYS   182    58.968   5.101 -29.103  0.00  0.00

ATOM   1438   O    CYS   182    59.784   5.844 -28.541  0.00  0.00

ATOM   1439   CB   CYS   182    57.261   5.896 -27.337  0.00  0.00

ATOM   1440   SG   CYS   182    56.750   7.500 -28.058  0.00  0.00

ATOM   1441   N    ASP   183    59.361   4.224 -30.073  0.00  0.00

ATOM   1442   CA   ASP   183    60.652   4.296 -30.767  0.00  0.00

ATOM   1443   C    ASP   183    61.065   5.756 -31.153  0.00  0.00

ATOM   1444   O    ASP   183    62.276   5.992 -31.116  0.00  0.00

ATOM   1445   CB   ASP   183    60.520   3.343 -31.918  0.00  0.00

ATOM   1446   CG   ASP   183    60.336   3.577 -33.367  0.00  0.00

ATOM   1447   OD1  ASP   183    60.764   2.631 -34.200  0.00  0.00

ATOM   1448   OD2  ASP   183    59.795   4.594 -33.942  0.00  0.00

ATOM   1449   N    PHE   184    60.180   6.619 -31.722  0.00  0.00

ATOM   1450   CA   PHE   184    60.452   8.026 -32.021  0.00  0.00

ATOM   1451   C    PHE   184    61.160   8.739 -30.823  0.00  0.00

ATOM   1452   O    PHE   184    62.276   9.227 -31.038  0.00  0.00

ATOM   1453   CB   PHE   184    59.147   8.722 -32.442  0.00  0.00

ATOM   1454   CG   PHE   184    59.347  10.072 -33.102  0.00  0.00
```

```
ATOM    1455   CD1  PHE    184      60.114  10.115 -34.265  0.00  0.00

ATOM    1456   CD2  PHE    184      58.773  11.234 -32.488  0.00  0.00

ATOM    1457   CE1  PHE    184      60.293  11.374 -34.869  0.00  0.00

ATOM    1458   CE2  PHE    184      59.053  12.437 -33.105  0.00  0.00

ATOM    1459   CZ   PHE    184      59.741  12.580 -34.265  0.00  0.00

ATOM    1460   N    PHE    185      60.626   8.715 -29.587  0.00  0.00

ATOM    1461   CA   PHE    185      61.187   9.437 -28.413  0.00  0.00

ATOM    1462   C    PHE    185      62.133   8.581 -27.498  0.00  0.00

ATOM    1463   O    PHE    185      62.673   9.155 -26.543  0.00  0.00

ATOM    1464   CB   PHE    185      60.015   9.997 -27.612  0.00  0.00

ATOM    1465   CG   PHE    185      59.121  10.914 -28.391  0.00  0.00

ATOM    1466   CD1  PHE    185      59.597  12.241 -28.559  0.00  0.00

ATOM    1467   CD2  PHE    185      57.928  10.510 -28.993  0.00  0.00

ATOM    1468   CE1  PHE    185      58.793  13.119 -29.350  0.00  0.00

ATOM    1469   CE2  PHE    185      57.167  11.396 -29.763  0.00  0.00

ATOM    1470   CZ   PHE    185      57.603  12.719 -29.943  0.00  0.00

ATOM    1471   N    THR    186      62.465   7.332 -27.833  0.00  0.00

ATOM    1472   CA   THR    186      63.421   6.452 -27.121  0.00  0.00

ATOM    1473   C    THR    186      63.129   6.250 -25.598  0.00  0.00

ATOM    1474   O    THR    186      63.852   6.830 -24.779  0.00  0.00

ATOM    1475   CB   THR    186      64.930   6.695 -27.510  0.00  0.00

ATOM    1476   OG1  THR    186      65.970   6.484 -26.535  0.00  0.00

ATOM    1477   CG2  THR    186      65.151   7.860 -28.424  0.00  0.00

ATOM    1478   N    ASN    187      61.915   5.863 -25.257  0.00  0.00

ATOM    1479   CA   ASN    187      61.395   5.692 -23.887  0.00  0.00
```

```
ATOM   1480  C    ASN   187     62.252    4.623  -23.104   0.00   0.00

ATOM   1481  O    ASN   187     62.358    4.788  -21.892   0.00   0.00

ATOM   1482  CB   ASN   187     59.949    5.375  -24.110   0.00   0.00

ATOM   1483  CG   ASN   187     59.050    6.548  -24.023   0.00   0.00

ATOM   1484  OD1  ASN   187     58.435    6.871  -25.018   0.00   0.00

ATOM   1485  ND2  ASN  ·187     59.027    7.259  -22.908   0.00   0.00

ATOM   1486  N    GLN   188     62.464    3.380  -23.641   0.00   0.00

ATOM   1487  CA   GLN   188     63.324    2.342  -23.058   0.00   0.00

ATOM   1488  C    GLN   188     64.761    2.883  -22.757   0.00   0.00

ATOM   1489  O    GLN   188     65.273    2.536  -21.698   0.00   0.00

ATOM   1490  CB   GLN   188     63.319    1.130  -24.006   0.00   0.00

ATOM   1491  CG   GLN   188     64.136   -0.035  -23.399   0.00   0.00

ATOM   1492  CD   GLN   188     64.113   -1.202  -24.371   0.00   0.00

ATOM   1493  OE1  GLN   188     64.145   -0.998  -25.583   0.00   0.00

ATOM   1494  NE2  GLN   188     64.034   -2.400  -23.811   0.00   0.00

ATOM   1495  N    ALA   189     65.487    3.471  -23.744   0.00   0.00

ATOM   1496  CA   ALA   189     66.797    4.079  -23.483   0.00   0.00

ATOM   1497  C    ALA   189     66.644    5.078  -22.301   0.00   0.00

ATOM   1498  O    ALA   189     67.593    5.130  -21.527   0.00   0.00

ATOM   1499  CB   ALA   189     67.475    4.650  -24.719   0.00   0.00

ATOM   1500  N    TYR   190     65.634    5.986  -22.264   0.00   0.00

ATOM   1501  CA   TYR   190     65.543    6.800  -21.038   0.00   0.00

ATOM   1502  C    TYR   190     65.364    5.930  -19.762   0.00   0.00

ATOM   1503  O    TYR   190     65.923    6.341  -18.766   0.00   0.00

ATOM   1504  CB   TYR   190     64.426    7.798  -21.030   0.00   0.00
```

```
ATOM   1505  CG   TYR   190    64.314   8.646 -19.817   0.00   0.00

ATOM   1506  CD1  TYR   190    63.557   8.064 -18.795   0.00   0.00

ATOM   1507  CD2  TYR   190    64.992   9.866 -19.627   0.00   0.00

ATOM   1508  CE1  TYR   190    63.418   8.717 -17.572   0.00   0.00

ATOM   1509  CE2  TYR   190    64.872  10.496 -18.397   0.00   0.00

ATOM   1510  CZ   TYR   190    64.073   9.938 -17.403   0.00   0.00

ATOM   1511  OH   TYR   190    63.892  10.585 -16.167   0.00   0.00

ATOM   1512  N    ALA   191    64.291   5.111 -19.669   0.00   0.00

ATOM   1513  CA   ALA   191    63.971   4.236 -18.553   0.00   0.00

ATOM   1514  C    ALA   191    65.233   3.535 -17.982   0.00   0.00

ATOM   1515  O    ALA   191    65.266   3.395 -16.761   0.00   0.00

ATOM   1516  CB   ALA   191    62.889   3.247 -19.015   0.00   0.00

ATOM   1517  N    ILE   192    66.094   2.889 -18.783   0.00   0.00

ATOM   1518  CA   ILE   192    67.349   2.290 -18.353   0.00   0.00

ATOM   1519  C    ILE   192    68.275   3.404 -17.802   0.00   0.00

ATOM   1520  O    ILE   192    68.830   3.175 -16.713   0.00   0.00

ATOM   1521  CB   ILE   192    67.941   1.506 -19.537   0.00   0.00

ATOM   1522  CG1  ILE   192    67.026   0.387 -20.006   0.00   0.00

ATOM   1523  CG2  ILE   192    69.320   0.927 -19.109   0.00   0.00

ATOM   1524  CD1  ILE   192    66.973  -0.924 -19.203   0.00   0.00

ATOM   1525  N    ALA   193    68.621   4.442 -18.589   0.00   0.00

ATOM   1526  CA   ALA   193    69.397   5.573 -18.105   0.00   0.00

ATOM   1527  C    ALA   193    68.837   6.064 -16.710   0.00   0.00

ATOM   1528  O    ALA   193    69.674   6.273 -15.827   0.00   0.00

ATOM   1529  CB   ALA   193    69.401   6.670 -19.178   0.00   0.00
```

| ATOM | 1530 | N   | SER | 194 | 67.523 | 6.339 | -16.532 | 0.00 | 0.00 |
| ATOM | 1531 | CA  | SER | 194 | 66.933 | 6.820 | -15.270 | 0.00 | 0.00 |
| ATOM | 1532 | C   | SER | 194 | 67.095 | 5.801 | -14.097 | 0.00 | 0.00 |
| ATOM | 1533 | O   | SER | 194 | 67.325 | 6.287 | -12.982 | 0.00 | 0.00 |
| ATOM | 1534 | CB  | SER | 194 | 65.437 | 7.169 | -15.518 | 0.00 | 0.00 |
| ATOM | 1535 | OG  | SER | 194 | 64.562 | 6.019 | -15.438 | 0.00 | 0.00 |
| ATOM | 1536 | N   | SER | 195 | 66.618 | 4.542 | -14.211 | 0.00 | 0.00 |
| ATOM | 1537 | CA  | SER | 195 | 66.850 | 3.552 | -13.175 | 0.00 | 0.00 |
| ATOM | 1538 | C   | SER | 195 | 68.365 | 3.454 | -12.836 | 0.00 | 0.00 |
| ATOM | 1539 | O   | SER | 195 | 68.726 | 4.016 | -11.820 | 0.00 | 0.00 |
| ATOM | 1540 | CB  | SER | 195 | 66.251 | 2.189 | -13.572 | 0.00 | 0.00 |
| ATOM | 1541 | OG  | SER | 195 | 64.837 | 2.058 | -13.615 | 0.00 | 0.00 |
| ATOM | 1542 | N   | ILE | 196 | 69.251 | 3.077 | -13.806 | 0.00 | 0.00 |
| ATOM | 1543 | CA  | ILE | 196 | 70.663 | 2.938 | -13.423 | 0.00 | 0.00 |
| ATOM | 1544 | C   | ILE | 196 | 71.288 | 4.228 | -12.826 | 0.00 | 0.00 |
| ATOM | 1545 | O   | ILE | 196 | 71.397 | 4.245 | -11.627 | 0.00 | 0.00 |
| ATOM | 1546 | CB  | ILE | 196 | 71.490 | 2.383 | -14.598 | 0.00 | 0.00 |
| ATOM | 1547 | CG1 | ILE | 196 | 71.042 | 1.022 | -15.008 | 0.00 | 0.00 |
| ATOM | 1548 | CG2 | ILE | 196 | 72.966 | 2.291 | -14.248 | 0.00 | 0.00 |
| ATOM | 1549 | CD1 | ILE | 196 | 71.199 | 0.687 | -16.493 | 0.00 | 0.00 |
| ATOM | 1550 | N   | VAL | 197 | 71.349 | 5.358 | -13.574 | 0.00 | 0.00 |
| ATOM | 1551 | CA  | VAL | 197 | 71.986 | 6.535 | -12.982 | 0.00 | 0.00 |
| ATOM | 1552 | C   | VAL | 197 | 71.254 | 7.108 | -11.698 | 0.00 | 0.00 |
| ATOM | 1553 | O   | VAL | 197 | 71.990 | 7.589 | -10.831 | 0.00 | 0.00 |
| ATOM | 1554 | CB  | VAL | 197 | 72.100 | 7.610 | -14.077 | 0.00 | 0.00 |

| ATOM | 1555 | CG1 | VAL | 197 | 72.870 | 8.834 | -13.555 | 0.00 | 0.00 |
|------|------|-----|-----|-----|--------|-------|---------|------|------|
| ATOM | 1556 | CG2 | VAL | 197 | 72.739 | 7.091 | -15.352 | 0.00 | 0.00 |
| ATOM | 1557 | N | SER | 198 | 69.952 | 7.510 | -11.746 | 0.00 | 0.00 |
| ATOM | 1558 | CA | SER | 198 | 69.241 | 8.064 | -10.608 | 0.00 | 0.00 |
| ATOM | 1559 | C | SER | 198 | 68.981 | 7.012 | -9.470 | 0.00 | 0.00 |
| ATOM | 1560 | O | SER | 198 | 69.046 | 7.442 | -8.321 | 0.00 | 0.00 |
| ATOM | 1561 | CB | SER | 198 | 67.951 | 8.737 | -11.117 | 0.00 | 0.00 |
| ATOM | 1562 | OG | SER | 198 | 67.105 | 9.256 | -10.086 | 0.00 | 0.00 |
| ATOM | 1563 | N | PHE | 199 | 68.175 | 5.976 | -9.759 | 0.00 | 0.00 |
| ATOM | 1564 | CA | PHE | 199 | 67.726 | 4.953 | -8.827 | 0.00 | 0.00 |
| ATOM | 1565 | C | PHE | 199 | 68.837 | 4.031 | -8.287 | 0.00 | 0.00 |
| ATOM | 1566 | O | PHE | 199 | 69.305 | 4.341 | -7.198 | 0.00 | 0.00 |
| ATOM | 1567 | CB | PHE | 199 | 66.576 | 4.224 | -9.526 | 0.00 | 0.00 |
| ATOM | 1568 | CG | PHE | 199 | 65.813 | 3.155 | -8.857 | 0.00 | 0.00 |
| ATOM | 1569 | CD1 | PHE | 199 | 65.366 | 3.283 | -7.537 | 0.00 | 0.00 |
| ATOM | 1570 | CD2 | PHE | 199 | 65.478 | 1.977 | -9.545 | 0.00 | 0.00 |
| ATOM | 1571 | CE1 | PHE | 199 | 64.590 | 2.292 | -6.929 | 0.00 | 0.00 |
| ATOM | 1572 | CE2 | PHE | 199 | 64.734 | 0.993 | -8.956 | 0.00 | 0.00 |
| ATOM | 1573 | CZ | PHE | 199 | 64.291 | 1.129 | -7.635 | 0.00 | 0.00 |
| ATOM | 1574 | N | TYR | 200 | 69.474 | 3.203 | -9.142 | 0.00 | 0.00 |
| ATOM | 1575 | CA | TYR | 200 | 70.564 | 2.308 | -8.735 | 0.00 | 0.00 |
| ATOM | 1576 | C | TYR | 200 | 71.787 | 3.103 | -8.234 | 0.00 | 0.00 |
| ATOM | 1577 | O | TYR | 200 | 72.272 | 2.749 | -7.158 | 0.00 | 0.00 |
| ATOM | 1578 | CB | TYR | 200 | 70.997 | 1.337 | -9.842 | 0.00 | 0.00 |
| ATOM | 1579 | CG | TYR | 200 | 69.870 | 0.408 | -10.215 | 0.00 | 0.00 |

| ATOM | 1580 | CD1 | TYR | 200 | 68.774 | 0.835 | -10.976 | 0.00 | 0.00 |
|------|------|-----|-----|-----|--------|-------|---------|------|------|
| ATOM | 1581 | CD2 | TYR | 200 | 69.886 | -0.909 | -9.751 | 0.00 | 0.00 |
| ATOM | 1582 | CE1 | TYR | 200 | 67.733 | -0.060 | -11.296 | 0.00 | 0.00 |
| ATOM | 1583 | CE2 | TYR | 200 | 68.876 | -1.792 | -10.081 | 0.00 | 0.00 |
| ATOM | 1584 | CZ | TYR | 200 | 67.788 | -1.361 | -10.822 | 0.00 | 0.00 |
| ATOM | 1585 | OH | TYR | 200 | 66.837 | -2.309 | -11.092 | 0.00 | 0.00 |
| ATOM | 1586 | N | VAL | 201 | 72.168 | 4.255 | -8.837 | 0.00 | 0.00 |
| ATOM | 1587 | CA | VAL | 201 | 73.396 | 4.995 | -8.449 | 0.00 | 0.00 |
| ATOM | 1588 | C | VAL | 201 | 73.404 | 5.665 | -7.086 | 0.00 | 0.00 |
| ATOM | 1589 | O | VAL | 201 | 74.041 | 5.039 | -6.227 | 0.00 | 0.00 |
| ATOM | 1590 | CB | VAL | 201 | 73.935 | 5.897 | -9.518 | 0.00 | 0.00 |
| ATOM | 1591 | CG1 | VAL | 201 | 75.323 | 6.414 | -9.212 | 0.00 | 0.00 |
| ATOM | 1592 | CG2 | VAL | 201 | 73.890 | 5.318 | -10.913 | 0.00 | 0.00 |
| ATOM | 1593 | N | PRO | 202 | 72.822 | 6.862 | -6.773 | 0.00 | 0.00 |
| ATOM | 1594 | CA | PRO | 202 | 72.894 | 7.226 | -5.411 | 0.00 | 0.00 |
| ATOM | 1595 | C | PRO | 202 | 72.394 | 6.118 | -4.403 | 0.00 | 0.00 |
| ATOM | 1596 | O | PRO | 202 | 72.895 | 6.154 | -3.286 | 0.00 | 0.00 |
| ATOM | 1597 | CB | PRO | 202 | 72.194 | 8.565 | -5.125 | 0.00 | 0.00 |
| ATOM | 1598 | CG | PRO | 202 | 71.388 | 8.800 | -6.412 | 0.00 | 0.00 |
| ATOM | 1599 | CD | PRO | 202 | 71.990 | 7.913 | -7.499 | 0.00 | 0.00 |
| ATOM | 1600 | N | LEU | 203 | 71.350 | 5.315 | -4.702 | 0.00 | 0.00 |
| ATOM | 1601 | CA | LEU | 203 | 70.983 | 4.268 | -3.724 | 0.00 | 0.00 |
| ATOM | 1602 | C | LEU | 203 | 72.237 | 3.507 | -3.178 | 0.00 | 0.00 |
| ATOM | 1603 | O | LEU | 203 | 72.267 | 3.308 | -1.979 | 0.00 | 0.00 |
| ATOM | 1604 | CB | LEU | 203 | 69.921 | 3.300 | -4.251 | 0.00 | 0.00 |

| ATOM | 1605 | CG | LEU | 203 | 68.509 | 3.843 | -4.341 | 0.00 | 0.00 |
|------|------|-----|-----|-----|--------|-------|--------|------|------|
| ATOM | 1606 | CD1 | LEU | 203 | 67.589 | 2.759 | -4.922 | 0.00 | 0.00 |
| ATOM | 1607 | CD2 | LEU | 203 | 68.012 | 4.320 | -2.977 | 0.00 | 0.00 |
| ATOM | 1608 | N | VAL | 204 | 73.033 | 2.873 | -4.038 | 0.00 | 0.00 |
| ATOM | 1609 | CA | VAL | 204 | 74.277 | 2.167 | -3.716 | 0.00 | 0.00 |
| ATOM | 1610 | C | VAL | 204 | 75.342 | 3.152 | -3.125 | 0.00 | 0.00 |
| ATOM | 1611 | O | VAL | 204 | 76.155 | 2.654 | -2.355 | 0.00 | 0.00 |
| ATOM | 1612 | CB | VAL | 204 | 74.623 | 1.416 | -4.946 | 0.00 | 0.00 |
| ATOM | 1613 | CG1 | VAL | 204 | 75.915 | 1.545 | -5.539 | 0.00 | 0.00 |
| ATOM | 1614 | CG2 | VAL | 204 | 73.895 | 0.127 | -5.163 | 0.00 | 0.00 |
| ATOM | 1615 | N | ILE | 205 | 75.546 | 4.379 | -3.659 | 0.00 | 0.00 |
| ATOM | 1616 | CA | ILE | 205 | 76.480 | 5.352 | -3.028 | 0.00 | 0.00 |
| ATOM | 1617 | C | ILE | 205 | 76.015 | 5.548 | -1.535 | 0.00 | 0.00 |
| ATOM | 1618 | O | ILE | 205 | 76.881 | 5.466 | -0.669 | 0.00 | 0.00 |
| ATOM | 1619 | CB | ILE | 205 | 76.524 | 6.686 | -3.862 | 0.00 | 0.00 |
| ATOM | 1620 | CG1 | ILE | 205 | 77.243 | 6.488 | -5.232 | 0.00 | 0.00 |
| ATOM | 1621 | CG2 | ILE | 205 | 77.120 | 7.895 | -3.067 | 0.00 | 0.00 |
| ATOM | 1622 | CD1 | ILE | 205 | 77.130 | 7.859 | -6.035 | 0.00 | 0.00 |
| ATOM | 1623 | N | MET | 206 | 74.706 | 5.797 | -1.220 | 0.00 | 0.00 |
| ATOM | 1624 | CA | MET | 206 | 74.139 | 5.898 | 0.118 | 0.00 | 0.00 |
| ATOM | 1625 | C | MET | 206 | 74.392 | 4.610 | 0.971 | 0.00 | 0.00 |
| ATOM | 1626 | O | MET | 206 | 74.759 | 4.786 | 2.142 | 0.00 | 0.00 |
| ATOM | 1627 | CB | MET | 206 | 72.626 | 6.115 | -0.067 | 0.00 | 0.00 |
| ATOM | 1628 | CG | MET | 206 | 72.303 | 7.434 | -0.695 | 0.00 | 0.00 |
| ATOM | 1629 | SD | MET | 206 | 70.583 | 7.581 | -1.269 | 0.00 | 0.00 |

| ATOM | 1630 | CE | MET | 206 | 69.725 | 7.523 | 0.310 | 0.00 | 0.00 |
|------|------|-----|-----|-----|--------|-------|-------|------|------|
| ATOM | 1631 | N | VAL | 207 | 73.937 | 3.402 | 0.535 | 0.00 | 0.00 |
| ATOM | 1632 | CA | VAL | 207 | 74.154 | 2.102 | 1.196 | 0.00 | 0.00 |
| ATOM | 1633 | C | VAL | 207 | 75.676 | 1.848 | 1.486 | 0.00 | 0.00 |
| ATOM | 1634 | O | VAL | 207 | 75.919 | 1.207 | 2.504 | 0.00 | 0.00 |
| ATOM | 1635 | CB | VAL | 207 | 73.489 | 1.019 | 0.345 | 0.00 | 0.00 |
| ATOM | 1636 | CG1 | VAL | 207 | 73.640 | -0.377 | 0.865 | 0.00 | 0.00 |
| ATOM | 1637 | CG2 | VAL | 207 | 72.003 | 1.234 | 0.262 | 0.00 | 0.00 |
| ATOM | 1638 | N | PHE | 208 | 76.617 | 2.067 | 0.547 | 0.00 | 0.00 |
| ATOM | 1639 | CA | PHE | 208 | 78.047 | 1.795 | 0.661 | 0.00 | 0.00 |
| ATOM | 1640 | C | PHE | 208 | 78.642 | 2.727 | 1.803 | 0.00 | 0.00 |
| ATOM | 1641 | O | PHE | 208 | 79.508 | 2.200 | 2.525 | 0.00 | 0.00 |
| ATOM | 1642 | CB | PHE | 208 | 78.644 | 1.998 | -0.753 | 0.00 | 0.00 |
| ATOM | 1643 | CG | PHE | 208 | 80.103 | 1.526 | -0.967 | 0.00 | 0.00 |
| ATOM | 1644 | CD1 | PHE | 208 | 80.473 | 0.279 | -0.430 | 0.00 | 0.00 |
| ATOM | 1645 | CD2 | PHE | 208 | 80.968 | 2.246 | -1.729 | 0.00 | 0.00 |
| ATOM | 1646 | CE1 | PHE | 208 | 81.792 | -0.169 | -0.604 | 0.00 | 0.00 |
| ATOM | 1647 | CE2 | PHE | 208 | 82.287 | 1.798 | -1.904 | 0.00 | 0.00 |
| ATOM | 1648 | CZ | PHE | 208 | 82.740 | 0.631 | -1.364 | 0.00 | 0.00 |
| ATOM | 1649 | N | VAL | 209 | 78.523 | 4.078 | 1.753 | 0.00 | 0.00 |
| ATOM | 1650 | CA | VAL | 209 | 78.948 | 4.989 | 2.806 | 0.00 | 0.00 |
| ATOM | 1651 | C | VAL | 209 | 78.434 | 4.450 | 4.186 | 0.00 | 0.00 |
| ATOM | 1652 | O | VAL | 209 | 79.261 | 4.410 | 5.095 | 0.00 | 0.00 |
| ATOM | 1653 | CB | VAL | 209 | 78.462 | 6.451 | 2.533 | 0.00 | 0.00 |
| ATOM | 1654 | CG1 | VAL | 209 | 78.838 | 7.390 | 3.658 | 0.00 | 0.00 |

```
ATOM   1655  CG2 VAL   209      78.953   6.972   1.175  0.00  0.00

ATOM   1656  N   TYR   210      77.102   4.258   4.384  0.00  0.00

ATOM   1657  CA  TYR   210      76.500   3.667   5.588  0.00  0.00

ATOM   1658  C   TYR   210      77.178   2.303   5.953  0.00  0.00

ATOM   1659  O   TYR   210      77.310   2.083   7.156  0.00  0.00

ATOM   1660  CB  TYR   210      74.966   3.491   5.424  0.00  0.00

ATOM   1661  CG  TYR   210      74.411   2.584   6.544  0.00  0.00

ATOM   1662  CD1 TYR   210      74.170   3.127   7.813  0.00  0.00

ATOM   1663  CD2 TYR   210      74.161   1.227   6.301  0.00  0.00

ATOM   1664  CE1 TYR   210      73.651   2.333   8.842  0.00  0.00

ATOM   1665  CE2 TYR   210      73.683   0.414   7.332  0.00  0.00

ATOM   1666  CZ  TYR   210      73.432   0.971   8.591  0.00  0.00

ATOM   1667  OH  TYR   210      72.969   0.164   9.591  0.00  0.00

ATOM   1668  N   SER   211      77.429   1.363   5.013  0.00  0.00

ATOM   1669  CA  SER   211      78.160   0.127   5.335  0.00  0.00

ATOM   1670  C   SER   211      79.514   0.467   6.068  0.00  0.00

ATOM   1671  O   SER   211      79.869  -0.299   6.964  0.00  0.00

ATOM   1672  CB  SER   211      78.381  -0.661   4.034  0.00  0.00

ATOM   1673  OG  SER   211      77.277  -1.409   3.530  0.00  0.00

ATOM   1674  N   ARG   212      80.358   1.425   5.572  0.00  0.00

ATOM   1675  CA  ARG   212      81.583   1.873   6.236  0.00  0.00

ATOM   1676  C   ARG   212      81.300   2.628   7.582  0.00  0.00

ATOM   1677  O   ARG   212      82.124   2.472   8.489  0.00  0.00

ATOM   1678  CB  ARG   212      82.602   2.513   5.317  0.00  0.00

ATOM   1679  CG  ARG   212      83.051   1.759   4.118  0.00  0.00
```

| ATOM | 1680 | CD | ARG | 212 | 84.006 | 2.667 | 3.259 | 0.00 | 0.00 |
|------|------|-----|-----|-----|--------|--------|--------|------|------|
| ATOM | 1681 | NE | ARG | 212 | 83.337 | 3.826 | 2.683 | 0.00 | 0.00 |
| ATOM | 1682 | CZ | ARG | 212 | 82.532 | 3.749 | 1.619 | 0.00 | 0.00 |
| ATOM | 1683 | NH1 | ARG | 212 | 82.313 | 2.600 | 0.972 | 0.00 | 0.00 |
| ATOM | 1684 | NH2 | ARG | 212 | 81.786 | 4.813 | 1.299 | 0.00 | 0.00 |
| ATOM | 1685 | N | VAL | 213 | 80.337 | 3.586 | 7.670 | 0.00 | 0.00 |
| ATOM | 1686 | CA | VAL | 213 | 79.950 | 4.258 | 8.926 | 0.00 | 0.00 |
| ATOM | 1687 | C | VAL | 213 | 79.649 | 3.222 | 10.076 | 0.00 | 0.00 |
| ATOM | 1688 | O | VAL | 213 | 79.668 | 3.654 | 11.232 | 0.00 | 0.00 |
| ATOM | 1689 | CB | VAL | 213 | 78.688 | 5.111 | 8.631 | 0.00 | 0.00 |
| ATOM | 1690 | CG1 | VAL | 213 | 77.985 | 5.787 | 9.777 | 0.00 | 0.00 |
| ATOM | 1691 | CG2 | VAL | 213 | 79.006 | 6.128 | 7.519 | 0.00 | 0.00 |
| ATOM | 1692 | N | PHE | 214 | 79.191 | 1.980 | 9.796 | 0.00 | 0.00 |
| ATOM | 1693 | CA | PHE | 214 | 78.971 | 0.905 | 10.743 | 0.00 | 0.00 |
| ATOM | 1694 | C | PHE | 214 | 80.115 | 0.842 | 11.802 | 0.00 | 0.00 |
| ATOM | 1695 | O | PHE | 214 | 79.779 | 0.484 | 12.931 | 0.00 | 0.00 |
| ATOM | 1696 | CB | PHE | 214 | 78.910 | -0.426 | 9.974 | 0.00 | 0.00 |
| ATOM | 1697 | CG | PHE | 214 | 78.678 | -1.631 | 10.873 | 0.00 | 0.00 |
| ATOM | 1698 | CD1 | PHE | 214 | 77.428 | -1.794 | 11.490 | 0.00 | 0.00 |
| ATOM | 1699 | CD2 | PHE | 214 | 79.690 | -2.558 | 11.093 | 0.00 | 0.00 |
| ATOM | 1700 | CE1 | PHE | 214 | 77.189 | -2.897 | 12.315 | 0.00 | 0.00 |
| ATOM | 1701 | CE2 | PHE | 214 | 79.454 | -3.682 | 11.899 | 0.00 | 0.00 |
| ATOM | 1702 | CZ | PHE | 214 | 78.199 | -3.841 | 12.502 | 0.00 | 0.00 |
| ATOM | 1703 | N | GLN | 215 | 81.425 | 1.030 | 11.451 | 0.00 | 0.00 |
| ATOM | 1704 | CA | GLN | 215 | 82.468 | 1.067 | 12.491 | 0.00 | 0.00 |

| | | | | | | | | | |
|------|------|-----|-----|-----|--------|--------|--------|------|------|
| ATOM | 1705 | C   | GLN | 215 | 82.074 | 2.093  | 13.589 | 0.00 | 0.00 |
| ATOM | 1706 | O   | GLN | 215 | 82.067 | 1.679  | 14.761 | 0.00 | 0.00 |
| ATOM | 1707 | CB  | GLN | 215 | 83.900 | 1.452  | 11.968 | 0.00 | 0.00 |
| ATOM | 1708 | CG  | GLN | 215 | 84.911 | 1.980  | 13.051 | 0.00 | 0.00 |
| ATOM | 1709 | CD  | GLN | 215 | 85.353 | 0.838  | 13.980 | 0.00 | 0.00 |
| ATOM | 1710 | OE1 | GLN | 215 | 86.020 | -0.152 | 13.358 | 0.00 | 0.00 |
| ATOM | 1711 | NE2 | GLN | 215 | 85.070 | 0.910  | 15.279 | 0.00 | 0.00 |
| ATOM | 1712 | N   | GLU | 216 | 81.969 | 3.362  | 13.300 | 0.00 | 0.00 |
| ATOM | 1713 | CA  | GLU | 216 | 81.655 | 4.429  | 14.283 | 0.00 | 0.00 |
| ATOM | 1714 | C   | GLU | 216 | 80.268 | 4.227  | 14.995 | 0.00 | 0.00 |
| ATOM | 1715 | O   | GLU | 216 | 80.300 | 3.933  | 16.191 | 0.00 | 0.00 |
| ATOM | 1716 | CB  | GLU | 216 | 81.855 | 5.817  | 13.692 | 0.00 | 0.00 |
| ATOM | 1717 | CG  | GLU | 216 | 81.404 | 7.059  | 14.462 | 0.00 | 0.00 |
| ATOM | 1718 | CD  | GLU | 216 | 82.539 | 7.480  | 15.416 | 0.00 | 0.00 |
| ATOM | 1719 | OE1 | GLU | 216 | 83.129 | 6.648  | 16.104 | 0.00 | 0.00 |
| ATOM | 1720 | OE2 | GLU | 216 | 82.767 | 8.704  | 15.326 | 0.00 | 0.00 |
| ATOM | 1721 | N   | ALA | 217 | 79.095 | 4.319  | 14.295 | 0.00 | 0.00 |
| ATOM | 1722 | CA  | ALA | 217 | 77.771 | 4.051  | 14.891 | 0.00 | 0.00 |
| ATOM | 1723 | C   | ALA | 217 | 77.010 | 2.946  | 14.061 | 0.00 | 0.00 |
| ATOM | 1724 | O   | ALA | 217 | 76.568 | 3.180  | 12.939 | 0.00 | 0.00 |
| ATOM | 1725 | CB  | ALA | 217 | 76.979 | 5.371  | 14.958 | 0.00 | 0.00 |
| ATOM | 1726 | N   | LYS | 218 | 77.117 | 1.712  | 14.561 | 0.00 | 0.00 |
| ATOM | 1727 | CA  | LYS | 218 | 76.512 | 0.568  | 13.887 | 0.00 | 0.00 |
| ATOM | 1728 | C   | LYS | 218 | 74.946 | 0.719  | 13.717 | 0.00 | 0.00 |
| ATOM | 1729 | O   | LYS | 218 | 74.543 | 0.981  | 12.573 | 0.00 | 0.00 |

```
ATOM   1730  CB   LYS   218      76.798  -0.721  14.702  0.00  0.00

ATOM   1731  CG   LYS   218      78.235  -0.952  15.075  0.00  0.00

ATOM   1732  CD   LYS   218      78.323  -1.936  16.240  0.00  0.00

ATOM   1733  CE   LYS   218      79.713  -1.947  16.864  0.00  0.00

ATOM   1734  NZ   LYS   218      79.773  -2.788  18.090  0.00  0.00

ATOM   1735  N    ARG   219      74.170   0.717  14.812  0.00  0.00

ATOM   1736  CA   ARG   219      72.728   0.926  14.717  0.00  0.00

ATOM   1737  C    ARG   219      72.107   1.677  15.892  0.00  0.00

ATOM   1738  O    ARG   219      70.871   1.796  15.860  0.00  0.00

ATOM   1739  CB   ARG   219      72.118  -0.485  14.624  0.00  0.00

ATOM   1740  CG   ARG   219      72.018  -1.272  15.936  0.00  0.00

ATOM   1741  CD   ARG   219      71.848  -2.724  15.524  0.00  0.00

ATOM   1742  NE   ARG   219      70.681  -3.343  16.145  0.00  0.00

ATOM   1743  CZ   ARG   219      70.759  -4.639  16.592  0.00  0.00

ATOM   1744  NH1  ARG   219      71.928  -5.378  16.432  0.00  0.00

ATOM   1745  NH2  ARG   219      69.657  -5.179  17.190  0.00  0.00

ATOM   1746  N    GLN   220      72.873   2.426  16.721  0.00  0.00

ATOM   1747  CA   GLN   220      72.243   3.014  17.864  0.00  0.00

ATOM   1748  C    GLN   220      72.785   4.370  18.259  0.00  0.00

ATOM   1749  O    GLN   220      74.001   4.612  18.253  0.00  0.00

ATOM   1750  CB   GLN   220      72.464   2.087  19.065  0.00  0.00

ATOM   1751  CG   GLN   220      71.968   0.673  18.971  0.00  0.00

ATOM   1752  CD   GLN   220      71.879  -0.216  20.219  0.00  0.00

ATOM   1753  OE1  GLN   220      71.791   0.357  21.311  0.00  0.00

ATOM   1754  NE2  GLN   220      71.834  -1.566  20.089  0.00  0.00
```

| ATOM | 1755 | N | LEU | 221 | 71.847 | 5.278 | 18.273 | 0.00 | 0.00 |
|------|------|-----|-----|-----|--------|--------|--------|------|------|
| ATOM | 1756 | CA | LEU | 221 | 71.978 | 6.632 | 18.682 | 0.00 | 0.00 |
| ATOM | 1757 | C | LEU | 221 | 71.074 | 6.698 | 19.974 | 0.00 | 0.00 |
| ATOM | 1758 | O | LEU | 221 | 69.901 | 7.073 | 19.829 | 0.00 | 0.00 |
| ATOM | 1759 | CB | LEU | 221 | 71.369 | 7.321 | 17.486 | 0.00 | 0.00 |
| ATOM | 1760 | CG | LEU | 221 | 72.215 | 7.670 | 16.271 | 0.00 | 0.00 |
| ATOM | 1761 | CD1 | LEU | 221 | 73.702 | 7.858 | 16.486 | 0.00 | 0.00 |
| ATOM | 1762 | CD2 | LEU | 221 | 71.820 | 6.499 | 15.385 | 0.00 | 0.00 |
| ATOM | 1763 | N | GLN | 222 | 71.590 | 6.541 | 21.195 | 0.00 | 0.00 |
| ATOM | 1764 | CA | GLN | 222 | 70.667 | 6.502 | 22.376 | 0.00 | 0.00 |
| ATOM | 1765 | C | GLN | 222 | 70.481 | 7.859 | 23.094 | 0.00 | 0.00 |
| ATOM | 1766 | O | GLN | 222 | 69.370 | 8.050 | 23.609 | 0.00 | 0.00 |
| ATOM | 1767 | CB | GLN | 222 | 71.203 | 5.416 | 23.333 | 0.00 | 0.00 |
| ATOM | 1768 | CG | GLN | 222 | 71.127 | 4.002 | 22.762 | 0.00 | 0.00 |
| ATOM | 1769 | CD | GLN | 222 | 69.739 | 3.338 | 22.638 | 0.00 | 0.00 |
| ATOM | 1770 | OE1 | GLN | 222 | 69.660 | 2.166 | 22.199 | 0.00 | 0.00 |
| ATOM | 1771 | NE2 | GLN | 222 | 68.665 | 3.984 | 22.990 | 0.00 | 0.00 |
| ATOM | 1772 | N | LYS | 223 | 71.512 | 8.708 | 23.252 | 0.00 | 0.00 |
| ATOM | 1773 | CA | LYS | 223 | 71.287 | 9.956 | 23.961 | 0.00 | 0.00 |
| ATOM | 1774 | C | LYS | 223 | 70.445 | 10.868 | 23.043 | 0.00 | 0.00 |
| ATOM | 1775 | O | LYS | 223 | 71.019 | 11.634 | 22.284 | 0.00 | 0.00 |
| ATOM | 1776 | CB | LYS | 223 | 72.621 | 10.547 | 24.424 | 0.00 | 0.00 |
| ATOM | 1777 | CG | LYS | 223 | 72.424 | 11.808 | 25.243 | 0.00 | 0.00 |
| ATOM | 1778 | CD | LYS | 223 | 73.743 | 12.229 | 25.842 | 0.00 | 0.00 |
| ATOM | 1779 | CE | LYS | 223 | 73.517 | 12.587 | 27.304 | 0.00 | 0.00 |

| ATOM | 1780 | NZ | LYS | 223 | 73.190 | 11.412 | 28.104 | 0.00 | 0.00 |
|------|------|------|-----|-----|--------|--------|--------|------|------|
| ATOM | 1781 | N | ILE | 224 | 69.244 | 11.178 | 23.517 | 0.00 | 0.00 |
| ATOM | 1782 | CA | ILE | 224 | 68.296 | 11.956 | 22.722 | 0.00 | 0.00 |
| ATOM | 1783 | C | ILE | 224 | 68.725 | 13.453 | 22.496 | 0.00 | 0.00 |
| ATOM | 1784 | O | ILE | 224 | 68.221 | 14.006 | 21.517 | 0.00 | 0.00 |
| ATOM | 1785 | CB | ILE | 224 | 66.907 | 11.792 | 23.357 | 0.00 | 0.00 |
| ATOM | 1786 | CG1 | ILE | 224 | 65.811 | 12.285 | 22.414 | 0.00 | 0.00 |
| ATOM | 1787 | CG2 | ILE | 224 | 66.871 | 12.596 | 24.680 | 0.00 | 0.00 |
| ATOM | 1788 | CD1 | ILE | 224 | 64.378 | 11.908 | 22.825 | 0.00 | 0.00 |
| ATOM | 1789 | N | ASP | 225 | 69.274 | 14.198 | 23.498 | 0.00 | 0.00 |
| ATOM | 1790 | CA | ASP | 225 | 69.560 | 15.635 | 23.200 | 0.00 | 0.00 |
| ATOM | 1791 | C | ASP | 225 | 70.484 | 15.778 | 21.942 | 0.00 | 0.00 |
| ATOM | 1792 | O | ASP | 225 | 69.968 | 16.252 | 20.940 | 0.00 | 0.00 |
| ATOM | 1793 | CB | ASP | 225 | 70.186 | 16.372 | 24.381 | 0.00 | 0.00 |
| ATOM | 1794 | CG | ASP | 225 | 69.318 | 16.402 | 25.578 | 0.00 | 0.00 |
| ATOM | 1795 | OD1 | ASP | 225 | 68.135 | 16.851 | 25.307 | 0.00 | 0.00 |
| ATOM | 1796 | OD2 | ASP | 225 | 69.630 | 16.047 | 26.726 | 0.00 | 0.00 |
| ATOM | 1797 | N | LYS | 226 | 71.842 | 15.724 | 22.076 | 0.00 | 0.00 |
| ATOM | 1798 | CA | LYS | 226 | 72.787 | 15.746 | 20.947 | 0.00 | 0.00 |
| ATOM | 1799 | C | LYS | 226 | 72.520 | 16.935 | 19.952 | 0.00 | 0.00 |
| ATOM | 1800 | O | LYS | 226 | 72.165 | 16.685 | 18.794 | 0.00 | 0.00 |
| ATOM | 1801 | CB | LYS | 226 | 72.554 | 14.394 | 20.384 | 0.00 | 0.00 |
| ATOM | 1802 | CG | LYS | 226 | 73.076 | 13.223 | 21.127 | 0.00 | 0.00 |
| ATOM | 1803 | CD | LYS | 226 | 73.295 | 12.026 | 20.221 | 0.00 | 0.00 |
| ATOM | 1804 | CE | LYS | 226 | 71.994 | 11.483 | 19.686 | 0.00 | 0.00 |

| ATOM | 1805 | NZ | LYS | 226 | 72.138 | 10.297 | 18.846 | 0.00 | 0.00 |
|------|------|----|----|-----|--------|--------|--------|------|------|
| ATOM | 1806 | N | SER | 227 | 72.325 | 18.177 | 20.471 | 0.00 | 0.00 |
| ATOM | 1807 | CA | SER | 227 | 72.140 | 19.391 | 19.681 | 0.00 | 0.00 |
| ATOM | 1808 | C | SER | 227 | 73.322 | 20.294 | 19.904 | 0.00 | 0.00 |
| ATOM | 1809 | O | SER | 227 | 73.232 | 21.248 | 20.724 | 0.00 | 0.00 |
| ATOM | 1810 | CB | SER | 227 | 70.855 | 20.078 | 20.015 | 0.00 | 0.00 |
| ATOM | 1811 | OG | SER | 227 | 69.647 | 19.476 | 19.624 | 0.00 | 0.00 |
| ATOM | 1812 | N | GLU | 228 | 74.203 | 20.256 | 18.956 | 0.00 | 0.00 |
| ATOM | 1813 | CA | GLU | 228 | 75.463 | 21.033 | 18.908 | 0.00 | 0.00 |
| ATOM | 1814 | C | GLU | 228 | 76.154 | 21.062 | 20.319 | 0.00 | 0.00 |
| ATOM | 1815 | O | GLU | 228 | 77.168 | 21.770 | 20.410 | 0.00 | 0.00 |
| ATOM | 1816 | CB | GLU | 228 | 75.101 | 22.480 | 18.526 | 0.00 | 0.00 |
| ATOM | 1817 | CG | GLU | 228 | 75.104 | 22.696 | 17.054 | 0.00 | 0.00 |
| ATOM | 1818 | CD | GLU | 228 | 76.197 | 22.309 | 16.184 | 0.00 | 0.00 |
| ATOM | 1819 | OE1 | GLU | 228 | 77.253 | 23.022 | 16.105 | 0.00 | 0.00 |
| ATOM | 1820 | OE2 | GLU | 228 | 76.044 | 21.186 | 15.472 | 0.00 | 0.00 |
| ATOM | 1821 | N | GLY | 229 | 75.957 | 20.074 | 21.221 | 0.00 | 0.00 |
| ATOM | 1822 | CA | GLY | 229 | 76.550 | 20.073 | 22.572 | 0.00 | 0.00 |
| ATOM | 1823 | C | GLY | 229 | 77.855 | 19.218 | 22.726 | 0.00 | 0.00 |
| ATOM | 1824 | O | GLY | 229 | 78.746 | 19.729 | 23.401 | 0.00 | 0.00 |
| ATOM | 1825 | N | PHE | 230 | 78.075 | 18.133 | 21.940 | 0.00 | 0.00 |
| ATOM | 1826 | CA | PHE | 230 | 79.253 | 17.277 | 22.092 | 0.00 | 0.00 |
| ATOM | 1827 | C | PHE | 230 | 79.369 | 16.271 | 20.897 | 0.00 | 0.00 |
| ATOM | 1828 | O | PHE | 230 | 78.778 | 16.546 | 19.859 | 0.00 | 0.00 |
| ATOM | 1829 | CB | PHE | 230 | 79.190 | 16.576 | 23.499 | 0.00 | 0.00 |

| | | | | | | | | | |
|---|---|---|---|---|---|---|---|---|---|
| ATOM | 1830 | CG | PHE | 230 | 80.326 | 15.542 | 23.641 | 0.00 | 0.00 |
| ATOM | 1831 | CD1 | PHE | 230 | 80.212 | 14.224 | 23.202 | 0.00 | 0.00 |
| ATOM | 1832 | CD2 | PHE | 230 | 81.520 | 15.937 | 24.223 | 0.00 | 0.00 |
| ATOM | 1833 | CE1 | PHE | 230 | 81.266 | 13.325 | 23.338 | 0.00 | 0.00 |
| ATOM | 1834 | CE2 | PHE | 230 | 82.570 | 15.039 | 24.361 | 0.00 | 0.00 |
| ATOM | 1835 | CZ | PHE | 230 | 82.439 | 13.731 | 23.915 | 0.00 | 0.00 |
| ATOM | 1836 | N | CYS | 231 | 80.544 | 15.636 | 20.828 | 0.00 | 0.00 |
| ATOM | 1837 | CA | CYS | 231 | 80.698 | 14.598 | 19.787 | 0.00 | 0.00 |
| ATOM | 1838 | C | CYS | 231 | 79.396 | 13.722 | 19.636 | 0.00 | 0.00 |
| ATOM | 1839 | O | CYS | 231 | 79.150 | 13.270 | 18.514 | 0.00 | 0.00 |
| ATOM | 1840 | CB | CYS | 231 | 81.908 | 13.691 | 20.025 | 0.00 | 0.00 |
| ATOM | 1841 | SG | CYS | 231 | 83.387 | 14.505 | 20.626 | 0.00 | 0.00 |
| ATOM | 1842 | N | LEU | 232 | 78.651 | 13.376 | 20.710 | 0.00 | 0.00 |
| ATOM | 1843 | CA | LEU | 232 | 77.372 | 12.649 | 20.637 | 0.00 | 0.00 |
| ATOM | 1844 | C | LEU | 232 | 76.293 | 13.328 | 19.719 | 0.00 | 0.00 |
| ATOM | 1845 | O | LEU | 232 | 75.622 | 12.590 | 19.054 | 0.00 | 0.00 |
| ATOM | 1846 | CB | LEU | 232 | 76.895 | 12.539 | 22.078 | 0.00 | 0.00 |
| ATOM | 1847 | CG | LEU | 232 | 77.182 | 11.299 | 22.871 | 0.00 | 0.00 |
| ATOM | 1848 | CD1 | LEU | 232 | 78.637 | 10.921 | 22.981 | 0.00 | 0.00 |
| ATOM | 1849 | CD2 | LEU | 232 | 76.608 | 11.362 | 24.329 | 0.00 | 0.00 |
| ATOM | 1850 | N | LYS | 233 | 76.158 | 14.671 | 19.665 | 0.00 | 0.00 |
| ATOM | 1851 | CA | LYS | 233 | 75.220 | 15.345 | 18.754 | 0.00 | 0.00 |
| ATOM | 1852 | C | LYS | 233 | 75.276 | 14.759 | 17.282 | 0.00 | 0.00 |
| ATOM | 1853 | O | LYS | 233 | 74.204 | 14.756 | 16.670 | 0.00 | 0.00 |
| ATOM | 1854 | CB | LYS | 233 | 75.486 | 16.863 | 18.709 | 0.00 | 0.00 |

```
ATOM   1855  CG   LYS   233      76.815  17.208  18.100  0.00  0.00

ATOM   1856  CD   LYS   233      77.120  18.669  17.932  0.00  0.00

ATOM   1857  CE   LYS   233      78.502  18.528  17.289  0.00  0.00

ATOM   1858  NZ   LYS   233      79.249  19.768  17.279  0.00  0.00

ATOM   1859  N    GLU   234      76.460  14.621  16.616  0.00  0.00

ATOM   1860  CA   GLU   234      76.566  13.976  15.277  0.00  0.00

ATOM   1861  C    GLU   234      75.746  12.633  15.161  0.00  0.00

ATOM   1862  O    GLU   234      75.680  12.119  14.051  0.00  0.00

ATOM   1863  CB   GLU   234      78.062  13.786  14.983  0.00  0.00

ATOM   1864  CG   GLU   234      78.884  15.041  14.735  0.00  0.00

ATOM   1865  CD   GLU   234      78.351  16.011  13.723  0.00  0.00

ATOM   1866  OE1  GLU   234      78.282  17.229  13.831  0.00  0.00

ATOM   1867  OE2  GLU   234      77.886  15.473  12.617  0.00  0.00

ATOM   1868  N    HIS   235      75.730  11.887  16.270  0.00  0.00

ATOM   1869  CA   HIS   235      75.002  10.647  16.476  0.00  0.00

ATOM   1870  C    HIS   235      73.483  10.871  16.164  0.00  0.00

ATOM   1871  O    HIS   235      73.065  10.338  15.138  0.00  0.00

ATOM   1872  CB   HIS   235      75.340  10.228  17.952  0.00  0.00

ATOM   1873  CG   HIS   235      75.482   8.723  18.295  0.00  0.00

ATOM   1874  ND1  HIS   235      74.898   8.154  19.433  0.00  0.00

ATOM   1875  CD2  HIS   235      76.174   7.703  17.703  0.00  0.00

ATOM   1876  CE1  HIS   235      75.242   6.878  19.493  0.00  0.00

ATOM   1877  NE2  HIS   235      76.002   6.594  18.471  0.00  0.00

ATOM   1878  N    LYS   236      72.846  11.965  16.668  0.00  0.00

ATOM   1879  CA   LYS   236      71.452  12.276  16.418  0.00  0.00
```

| ATOM | 1880 | C   | LYS | 236 | 71.284 | 12.725 | 14.933 | 0.00 | 0.00 |
|------|------|-----|-----|-----|--------|--------|--------|------|------|
| ATOM | 1881 | O   | LYS | 236 | 70.254 | 12.373 | 14.360 | 0.00 | 0.00 |
| ATOM | 1882 | CB  | LYS | 236 | 71.030 | 13.259 | 17.447 | 0.00 | 0.00 |
| ATOM | 1883 | CG  | LYS | 236 | 69.625 | 13.716 | 17.422 | 0.00 | 0.00 |
| ATOM | 1884 | CD  | LYS | 236 | 69.408 | 14.442 | 18.733 | 0.00 | 0.00 |
| ATOM | 1885 | CE  | LYS | 236 | 67.951 | 14.774 | 18.890 | 0.00 | 0.00 |
| ATOM | 1886 | NZ  | LYS | 236 | 67.202 | 13.545 | 18.715 | 0.00 | 0.00 |
| ATOM | 1887 | N   | ALA | 237 | 72.147 | 13.607 | 14.402 | 0.00 | 0.00 |
| ATOM | 1888 | CA  | ALA | 237 | 72.160 | 14.020 | 13.024 | 0.00 | 0.00 |
| ATOM | 1889 | C   | ALA | 237 | 72.320 | 12.753 | 12.108 | 0.00 | 0.00 |
| ATOM | 1890 | O   | ALA | 237 | 71.665 | 12.739 | 11.080 | 0.00 | 0.00 |
| ATOM | 1891 | CB  | ALA | 237 | 73.279 | 15.046 | 12.831 | 0.00 | 0.00 |
| ATOM | 1892 | N   | LEU | 238 | 73.303 | 11.837 | 12.341 | 0.00 | 0.00 |
| ATOM | 1893 | CA  | LEU | 238 | 73.480 | 10.580 | 11.595 | 0.00 | 0.00 |
| ATOM | 1894 | C   | LEU | 238 | 72.217 | 9.686  | 11.651 | 0.00 | 0.00 |
| ATOM | 1895 | O   | LEU | 238 | 71.878 | 9.172  | 10.589 | 0.00 | 0.00 |
| ATOM | 1896 | CB  | LEU | 238 | 74.717 | 9.788  | 12.115 | 0.00 | 0.00 |
| ATOM | 1897 | CG  | LEU | 238 | 75.012 | 8.424  | 11.447 | 0.00 | 0.00 |
| ATOM | 1898 | CD1 | LEU | 238 | 75.346 | 8.568  | 9.963  | 0.00 | 0.00 |
| ATOM | 1899 | CD2 | LEU | 238 | 76.123 | 7.662  | 12.177 | 0.00 | 0.00 |
| ATOM | 1900 | N   | LYS | 239 | 71.727 | 9.216  | 12.832 | 0.00 | 0.00 |
| ATOM | 1901 | CA  | LYS | 239 | 70.470 | 8.451  | 12.918 | 0.00 | 0.00 |
| ATOM | 1902 | C   | LYS | 239 | 69.354 | 9.133  | 12.089 | 0.00 | 0.00 |
| ATOM | 1903 | O   | LYS | 239 | 68.581 | 8.383  | 11.499 | 0.00 | 0.00 |
| ATOM | 1904 | CB  | LYS | 239 | 69.949 | 8.370  | 14.349 | 0.00 | 0.00 |

| ATOM | 1905 | CG | LYS | 239 | 68.826 | 7.425 | 14.652 | 0.00 | 0.00 |
|------|------|-----|-----|-----|--------|--------|--------|------|------|
| ATOM | 1906 | CD | LYS | 239 | 68.584 | 7.194 | 16.136 | 0.00 | 0.00 |
| ATOM | 1907 | CE | LYS | 239 | 67.935 | 5.846 | 16.428 | 0.00 | 0.00 |
| ATOM | 1908 | NZ | LYS | 239 | 68.024 | 5.406 | 17.844 | 0.00 | 0.00 |
| ATOM | 1909 | N | THR | 240 | 69.017 | 10.422 | 12.336 | 0.00 | 0.00 |
| ATOM | 1910 | CA | THR | 240 | 68.054 | 11.244 | 11.577 | 0.00 | 0.00 |
| ATOM | 1911 | C | THR | 240 | 68.254 | 11.093 | 10.042 | 0.00 | 0.00 |
| ATOM | 1912 | O | THR | 240 | 67.252 | 10.807 | 9.403 | 0.00 | 0.00 |
| ATOM | 1913 | CB | THR | 240 | 67.934 | 12.712 | 12.119 | 0.00 | 0.00 |
| ATOM | 1914 | OG1 | THR | 240 | 67.546 | 12.744 | 13.533 | 0.00 | 0.00 |
| ATOM | 1915 | CG2 | THR | 240 | 66.905 | 13.528 | 11.291 | 0.00 | 0.00 |
| ATOM | 1916 | N | LEU | 241 | 69.426 | 11.385 | 9.461 | 0.00 | 0.00 |
| ATOM | 1917 | CA | LEU | 241 | 69.717 | 11.198 | 8.022 | 0.00 | 0.00 |
| ATOM | 1918 | C | LEU | 241 | 69.502 | 9.703 | 7.573 | 0.00 | 0.00 |
| ATOM | 1919 | O | LEU | 241 | 69.173 | 9.534 | 6.400 | 0.00 | 0.00 |
| ATOM | 1920 | CB | LEU | 241 | 71.161 | 11.677 | 7.834 | 0.00 | 0.00 |
| ATOM | 1921 | CG | LEU | 241 | 71.289 | 13.196 | 8.072 | 0.00 | 0.00 |
| ATOM | 1922 | CD1 | LEU | 241 | 72.747 | 13.625 | 7.860 | 0.00 | 0.00 |
| ATOM | 1923 | CD2 | LEU | 241 | 70.450 | 13.899 | 7.116 | 0.00 | 0.00 |
| ATOM | 1924 | N | GLY | 242 | 69.875 | 8.688 | 8.382 | 0.00 | 0.00 |
| ATOM | 1925 | CA | GLY | 242 | 69.632 | 7.271 | 8.117 | 0.00 | 0.00 |
| ATOM | 1926 | C | GLY | 242 | 68.102 | 7.081 | 7.972 | 0.00 | 0.00 |
| ATOM | 1927 | O | GLY | 242 | 67.714 | 6.279 | 7.113 | 0.00 | 0.00 |
| ATOM | 1928 | N | ILE | 243 | 67.325 | 7.439 | 9.017 | 0.00 | 0.00 |
| ATOM | 1929 | CA | ILE | 243 | 65.884 | 7.418 | 8.994 | 0.00 | 0.00 |

| | | | | | | | | | |
|---|---|---|---|---|---|---|---|---|---|
| ATOM | 1930 | C | ILE | 243 | 65.387 | 8.209 | 7.722 | 0.00 | 0.00 |
| ATOM | 1931 | O | ILE | 243 | 64.332 | 7.830 | 7.218 | 0.00 | 0.00 |
| ATOM | 1932 | CB | ILE | 243 | 65.254 | 7.933 | 10.319 | 0.00 | 0.00 |
| ATOM | 1933 | CG1 | ILE | 243 | 65.394 | 6.822 | 11.394 | 0.00 | 0.00 |
| ATOM | 1934 | CG2 | ILE | 243 | 63.732 | 8.264 | 10.111 | 0.00 | 0.00 |
| ATOM | 1935 | CD1 | ILE | 243 | 64.436 | 5.632 | 11.111 | 0.00 | 0.00 |
| ATOM | 1936 | N | ILE | 244 | 65.957 | 9.397 | 7.354 | 0.00 | 0.00 |
| ATOM | 1937 | CA | ILE | 244 | 65.533 | 10.064 | 6.081 | 0.00 | 0.00 |
| ATOM | 1938 | C | ILE | 244 | 65.472 | 9.022 | 4.911 | 0.00 | 0.00 |
| ATOM | 1939 | O | ILE | 244 | 64.460 | 9.009 | 4.213 | 0.00 | 0.00 |
| ATOM | 1940 | CB | ILE | 244 | 66.443 | 11.250 | 5.744 | 0.00 | 0.00 |
| ATOM | 1941 | CG1 | ILE | 244 | 66.457 | 12.250 | 6.869 | 0.00 | 0.00 |
| ATOM | 1942 | CG2 | ILE | 244 | 66.055 | 11.857 | 4.401 | 0.00 | 0.00 |
| ATOM | 1943 | CD1 | ILE | 244 | 67.431 | 13.459 | 6.665 | 0.00 | 0.00 |
| ATOM | 1944 | N | MET | 245 | 66.554 | 8.258 | 4.646 | 0.00 | 0.00 |
| ATOM | 1945 | CA | MET | 245 | 66.611 | 7.177 | 3.656 | 0.00 | 0.00 |
| ATOM | 1946 | C | MET | 245 | 65.463 | 6.125 | 3.788 | 0.00 | 0.00 |
| ATOM | 1947 | O | MET | 245 | 65.105 | 5.585 | 2.734 | 0.00 | 0.00 |
| ATOM | 1948 | CB | MET | 245 | 67.909 | 6.422 | 3.900 | 0.00 | 0.00 |
| ATOM | 1949 | CG | MET | 245 | 69.188 | 7.090 | 3.600 | 0.00 | 0.00 |
| ATOM | 1950 | SD | MET | 245 | 70.635 | 5.990 | 3.860 | 0.00 | 0.00 |
| ATOM | 1951 | CE | MET | 245 | 70.024 | 4.891 | 5.129 | 0.00 | 0.00 |
| ATOM | 1952 | N | GLY | 246 | 65.224 | 5.553 | 5.000 | 0.00 | 0.00 |
| ATOM | 1953 | CA | GLY | 246 | 64.176 | 4.614 | 5.296 | 0.00 | 0.00 |
| ATOM | 1954 | C | GLY | 246 | 62.810 | 5.182 | 4.849 | 0.00 | 0.00 |

| ATOM | 1955 | O | GLY | 246 | 62.079 | 4.397 | 4.265 | 0.00 | 0.00 |
|------|------|-----|-----|-----|--------|-------|--------|------|------|
| ATOM | 1956 | N | THR | 247 | 62.391 | 6.396 | 5.249 | 0.00 | 0.00 |
| ATOM | 1957 | CA | THR | 247 | 61.152 | 6.999 | 4.760 | 0.00 | 0.00 |
| ATOM | 1958 | C | THR | 247 | 61.114 | 7.133 | 3.184 | 0.00 | 0.00 |
| ATOM | 1959 | O | THR | 247 | 59.995 | 7.101 | 2.676 | 0.00 | 0.00 |
| ATOM | 1960 | CB | THR | 247 | 60.821 | 8.295 | 5.558 | 0.00 | 0.00 |
| ATOM | 1961 | OG1 | THR | 247 | 60.599 | 8.072 | 6.963 | 0.00 | 0.00 |
| ATOM | 1962 | CG2 | THR | 247 | 59.561 | 9.047 | 5.009 | 0.00 | 0.00 |
| ATOM | 1963 | N | PHE | 248 | 62.181 | 7.619 | 2.483 | 0.00 | 0.00 |
| ATOM | 1964 | CA | PHE | 248 | 62.283 | 7.689 | 1.019 | 0.00 | 0.00 |
| ATOM | 1965 | C | PHE | 248 | 62.141 | 6.270 | 0.390 | 0.00 | 0.00 |
| ATOM | 1966 | O | PHE | 248 | 61.611 | 6.231 | -0.718 | 0.00 | 0.00 |
| ATOM | 1967 | CB | PHE | 248 | 63.578 | 8.394 | 0.547 | 0.00 | 0.00 |
| ATOM | 1968 | CG | PHE | 248 | 63.643 | 8.542 | -0.993 | 0.00 | 0.00 |
| ATOM | 1969 | CD1 | PHE | 248 | 63.342 | 9.754 | -1.525 | 0.00 | 0.00 |
| ATOM | 1970 | CD2 | PHE | 248 | 63.976 | 7.501 | -1.851 | 0.00 | 0.00 |
| ATOM | 1971 | CE1 | PHE | 248 | 63.364 | 9.932 | -2.899 | 0.00 | 0.00 |
| ATOM | 1972 | CE2 | PHE | 248 | 63.994 | 7.670 | -3.209 | 0.00 | 0.00 |
| ATOM | 1973 | CZ | PHE | 248 | 63.685 | 8.893 | -3.746 | 0.00 | 0.00 |
| ATOM | 1974 | N | THR | 249 | 62.881 | 5.225 | 0.837 | 0.00 | 0.00 |
| ATOM | 1975 | CA | THR | 249 | 62.639 | 3.889 | 0.294 | 0.00 | 0.00 |
| ATOM | 1976 | C | THR | 249 | 61.140 | 3.474 | 0.608 | 0.00 | 0.00 |
| ATOM | 1977 | O | THR | 249 | 60.476 | 3.164 | -0.333 | 0.00 | 0.00 |
| ATOM | 1978 | CB | THR | 249 | 63.542 | 2.807 | 0.942 | 0.00 | 0.00 |
| ATOM | 1979 | OG1 | THR | 249 | 63.556 | 2.703 | 2.337 | 0.00 | 0.00 |

```
ATOM   1980  CG2 THR   249    64.967   2.852    0.359   0.00   0.00

ATOM   1981  N   LEU   250    60.535   3.833    1.766   0.00   0.00

ATOM   1982  CA  LEU   250    59.089   3.514    1.999   0.00   0.00

ATOM   1983  C   LEU   250    58.184   4.200    0.938   0.00   0.00

ATOM   1984  O   LEU   250    57.451   3.508    0.232   0.00   0.00

ATOM   1985  CB  LEU   250    58.617   3.868    3.412   0.00   0.00

ATOM   1986  CG  LEU   250    59.394   3.135    4.496   0.00   0.00

ATOM   1987  CD1 LEU   250    59.159   3.911    5.774   0.00   0.00

ATOM   1988  CD2 LEU   250    58.856   1.718    4.637   0.00   0.00

ATOM   1989  N   CYS   251    58.416   5.496    0.732   0.00   0.00

ATOM   1990  CA  CYS   251    57.702   6.381   -0.195   0.00   0.00

ATOM   1991  C   CYS   251    57.973   6.022   -1.683   0.00   0.00

ATOM   1992  O   CYS   251    56.957   5.925   -2.429   0.00   0.00

ATOM   1993  CB  CYS   251    58.210   7.804    0.147   0.00   0.00

ATOM   1994  SG  CYS   251    56.967   9.048   -0.365   0.00   0.00

ATOM   1995  N   TRP   252    59.219   5.627   -2.004   0.00   0.00

ATOM   1996  CA  TRP   252    59.540   5.384   -3.419   0.00   0.00

ATOM   1997  C   TRP   252    60.035   3.907   -3.734   0.00   0.00

ATOM   1998  O   TRP   252    60.434   3.640   -4.906   0.00   0.00

ATOM   1999  CB  TRP   252    60.418   6.430   -4.031   0.00   0.00

ATOM   2000  CG  TRP   252    59.918   7.793   -3.916   0.00   0.00

ATOM   2001  CD1 TRP   252    60.406   8.823   -3.138   0.00   0.00

ATOM   2002  CD2 TRP   252    58.804   8.318   -4.641   0.00   0.00

ATOM   2003  NE1 TRP   252    59.674   9.954   -3.384   0.00   0.00

ATOM   2004  CE2 TRP   252    58.704   9.696   -4.293   0.00   0.00
```

```
ATOM    2005   CE3  TRP   252      57.895    7.783   -5.542   0.00   0.00

ATOM    2006   CZ2  TRP   252      57.703   10.499   -4.798   0.00   0.00

ATOM    2007   CZ3  TRP   252      56.919    8.610   -6.081   0.00   0.00

ATOM    2008   CH2  TRP   252      56.792    9.950   -5.689   0.00   0.00

ATOM    2009   N    LEU   253      59.663    2.949   -2.829   0.00   0.00

ATOM    2010   CA   LEU   253      59.877    1.552   -2.972   0.00   0.00

ATOM    2011   C    LEU   253      58.747    1.036   -3.962   0.00   0.00

ATOM    2012   O    LEU   253      59.069    1.000   -5.173   0.00   0.00

ATOM    2013   CB   LEU   253      60.088    0.734   -1.724   0.00   0.00

ATOM    2014   CG   LEU   253      61.482    0.397   -1.296   0.00   0.00

ATOM    2015   CD1  LEU   253      62.515    1.477   -1.534   0.00   0.00

ATOM    2016   CD2  LEU   253      61.469    0.209    0.195   0.00   0.00

ATOM    2017   N    PRO   254      57.474    0.690   -3.614   0.00   0.00

ATOM    2018   CA   PRO   254      56.600    0.329   -4.653   0.00   0.00

ATOM    2019   C    PRO   254      56.466    1.439   -5.672   0.00   0.00

ATOM    2020   O    PRO   254      56.260    1.057   -6.803   0.00   0.00

ATOM    2021   CB   PRO   254      55.326   -0.071   -3.974   0.00   0.00

ATOM    2022   CG   PRO   254      55.371    0.541   -2.581   0.00   0.00

ATOM    2023   CD   PRO   254      56.810    0.501   -2.259   0.00   0.00

ATOM    2024   N    PHE   255      56.677    2.738   -5.398   0.00   0.00

ATOM    2025   CA   PHE   255      56.605    3.648   -6.580   0.00   0.00

ATOM    2026   C    PHE   255      57.504    3.134   -7.753   0.00   0.00

ATOM    2027   O    PHE   255      57.048    3.178   -8.905   0.00   0.00

ATOM    2028   CB   PHE   255      57.198    5.002   -6.210   0.00   0.00

ATOM    2029   CG   PHE   255      57.293    5.981   -7.333   0.00   0.00
```

```
ATOM   2030  CD1 PHE   255    56.136   6.558  -7.872  0.00  0.00

ATOM   2031  CD2 PHE   255    58.493   6.174  -7.994  0.00  0.00

ATOM   2032  CE1 PHE   255    56.232   7.387  -8.977  0.00  0.00

ATOM   2033  CE2 PHE   255    58.617   6.958  -9.139  0.00  0.00

ATOM   2034  CZ  PHE   255    57.435   7.577  -9.640  0.00  0.00

ATOM   2035  N   PHE   256    58.830   2.954  -7.528  0.00  0.00

ATOM   2036  CA  PHE   256    59.804   2.430  -8.467  0.00  0.00

ATOM   2037  C   PHE   256    59.561   0.962  -8.906  0.00  0.00

ATOM   2038  O   PHE   256    60.112   0.620  -9.939  0.00  0.00

ATOM   2039  CB  PHE   256    61.214   2.630  -7.922  0.00  0.00

ATOM   2040  CG  PHE   256    61.768   3.984  -8.074  0.00  0.00

ATOM   2041  CD1 PHE   256    61.787   4.593  -9.319  0.00  0.00

ATOM   2042  CD2 PHE   256    62.272   4.643  -6.943  0.00  0.00

ATOM   2043  CE1 PHE   256    62.310   5.879  -9.470  0.00  0.00

ATOM   2044  CE2 PHE   256    62.781   5.933  -7.093  0.00  0.00

ATOM   2045  CZ  PHE   256    62.789   6.552  -8.336  0.00  0.00

ATOM   2046  N   ILE   257    59.384   0.018  -7.931  0.00  0.00

ATOM   2047  CA  ILE   257    59.046  -1.381  -8.133  0.00  0.00

ATOM   2048  C   ILE   257    57.835  -1.541  -9.090  0.00  0.00

ATOM   2049  O   ILE   257    57.949  -2.411  -9.955  0.00  0.00

ATOM   2050  CB  ILE   257    58.807  -2.085  -6.762  0.00  0.00

ATOM   2051  CG1 ILE   257    59.915  -1.949  -5.745  0.00  0.00

ATOM   2052  CG2 ILE   257    58.470  -3.590  -6.977  0.00  0.00

ATOM   2053  CD1 ILE   257    61.331  -2.125  -6.288  0.00  0.00

ATOM   2054  N   VAL   258    56.639  -0.952  -8.828  0.00  0.00
```

```
ATOM    2055  CA   VAL   258      55.514  -1.027   -9.805   0.00   0.00

ATOM    2056  C    VAL   258      56.003  -0.361  -11.144   0.00   0.00

ATOM    2057  O    VAL   258      55.726  -0.917  -12.209   0.00   0.00

ATOM    2058  CB   VAL   258      54.271  -0.286   -9.388   0.00   0.00

ATOM    2059  CG1  VAL   258      54.532   1.103   -8.878   0.00   0.00

ATOM    2060  CG2  VAL   258      53.183  -0.322  -10.405   0.00   0.00

ATOM    2061  N    ASN   259      56.702   0.816  -11.101   0.00   0.00

ATOM    2062  CA   ASN   259      57.291   1.494  -12.204   0.00   0.00

ATOM    2063  C    ASN   259      58.186   0.546  -13.083   0.00   0.00

ATOM    2064  O    ASN   259      57.886   0.442  -14.279   0.00   0.00

ATOM    2065  CB   ASN   259      58.203   2.499  -11.597   0.00   0.00

ATOM    2066  CG   ASN   259      58.225   3.852  -12.139   0.00   0.00

ATOM    2067  OD1  ASN   259      57.496   4.781  -11.768   0.00   0.00

ATOM    2068  ND2  ASN   259      59.179   3.827  -13.078   0.00   0.00

ATOM    2069  N    ILE   260      59.248  -0.082  -12.525   0.00   0.00

ATOM    2070  CA   ILE   260      60.084  -1.045  -13.199   0.00   0.00

ATOM    2071  C    ILE   260      59.271  -2.286  -13.726   0.00   0.00

ATOM    2072  O    ILE   260      59.590  -2.724  -14.833   0.00   0.00

ATOM    2073  CB   ILE   260      61.278  -1.376  -12.289   0.00   0.00

ATOM    2074  CG1  ILE   260      62.308  -2.224  -13.020   0.00   0.00

ATOM    2075  CG2  ILE   260      60.834  -2.101  -11.011   0.00   0.00

ATOM    2076  CD1  ILE   260      63.592  -2.510  -12.232   0.00   0.00

ATOM    2077  N    VAL   261      58.281  -2.862  -12.977   0.00   0.00

ATOM    2078  CA   VAL   261      57.429  -3.972  -13.439   0.00   0.00

ATOM    2079  C    VAL   261      56.699  -3.568  -14.761   0.00   0.00
```

```
ATOM    2080  O    VAL    261       56.798  -4.369 -15.698  0.00   0.00

ATOM    2081  CB   VAL    261       56.453  -4.456 -12.329  0.00   0.00

ATOM    2082  CG1  VAL    261       55.244  -5.148 -13.031  0.00   0.00

ATOM    2083  CG2  VAL    261       57.031  -5.617 -11.498  0.00   0.00

ATOM    2084  N    HIS    262       55.883  -2.490 -14.813  0.00   0.00

ATOM    2085  CA   HIS    262       55.264  -2.017 -16.064  0.00   0.00

ATOM    2086  C    HIS    262       56.318  -1.981 -17.240  0.00   0.00

ATOM    2087  O    HIS    262       55.930  -2.430 -18.313  0.00   0.00

ATOM    2088  CB   HIS    262       54.586  -0.653 -15.922  0.00   0.00

ATOM    2089  CG   HIS    262       53.465  -0.653 -14.942  0.00   0.00

ATOM    2090  ND1  HIS    262       52.252  -1.253 -15.135  0.00   0.00

ATOM    2091  CD2  HIS    262       53.411  -0.044 -13.715  0.00   0.00

ATOM    2092  CE1  HIS    262       51.513  -1.059 -14.063  0.00   0.00

ATOM    2093  NE2  HIS    262       52.174  -0.311 -13.192  0.00   0.00

ATOM    2094  N    VAL    263       57.475  -1.291 -17.132  0.00   0.00

ATOM    2095  CA   VAL    263       58.504  -1.174 -18.171  0.00   0.00

ATOM    2096  C    VAL    263       58.952  -2.564 -18.769  0.00   0.00

ATOM    2097  O    VAL    263       59.024  -2.617 -20.006  0.00   0.00

ATOM    2098  CB   VAL    263       59.703  -0.370 -17.619  0.00   0.00

ATOM    2099  CG1  VAL    263       60.929  -0.613 -18.479  0.00   0.00

ATOM    2100  CG2  VAL    263       59.359   1.091 -17.482  0.00   0.00

ATOM    2101  N    ILE    264       59.446  -3.546 -17.983  0.00   0.00

ATOM    2102  CA   ILE    264       59.781  -4.824 -18.639  0.00   0.00

ATOM    2103  C    ILE    264       58.498  -5.462 -19.314  0.00   0.00

ATOM    2104  O    ILE    264       58.587  -5.772 -20.504  0.00   0.00
```

```
ATOM   2105  CB   ILE   264      60.426  -5.898 -17.684  0.00  0.00

ATOM   2106  CG1  ILE   264      59.896  -5.787 -16.273  0.00  0.00

ATOM   2107  CG2  ILE   264      61.941  -6.266 -17.727  0.00  0.00

ATOM   2108  CD1  ILE   264      59.531  -7.266 -15.829  0.00  0.00

ATOM   2109  N    GLN   265      57.406  -5.786 -18.583  0.00  0.00

ATOM   2110  CA   GLN   265      56.161  -6.286 -19.136  0.00  0.00

ATOM   2111  C    GLN   265      55.269  -5.107 -19.555  0.00  0.00

ATOM   2112  O    GLN   265      54.469  -4.595 -18.754  0.00  0.00

ATOM   2113  CB   GLN   265      55.466  -7.159 -18.088  0.00  0.00

ATOM   2114  CG   GLN   265      56.191  -8.295 -17.424  0.00  0.00

ATOM   2115  CD   GLN   265      55.386  -8.854 -16.258  0.00  0.00

ATOM   2116  OE1  GLN   265      56.010  -9.102 -15.217  0.00  0.00

ATOM   2117  NE2  GLN   265      54.096  -9.079 -16.523  0.00  0.00

ATOM   2118  N    ASP   266      55.279  -4.882 -20.866  0.00  0.00

ATOM   2119  CA   ASP   266      54.460  -3.901 -21.513  0.00  0.00

ATOM   2120  C    ASP   266      53.108  -4.546 -21.958  0.00  0.00

ATOM   2121  O    ASP   266      53.111  -5.571 -22.639  0.00  0.00

ATOM   2122  CB   ASP   266      55.217  -3.311 -22.695  0.00  0.00

ATOM   2123  CG   ASP   266      56.391  -2.495 -22.240  0.00  0.00

ATOM   2124  OD1  ASP   266      56.395  -2.006 -21.116  0.00  0.00

ATOM   2125  OD2  ASP   266      57.321  -2.301 -23.013  0.00  0.00

ATOM   2126  N    ASN   267      52.084  -3.679 -21.996  0.00  0.00

ATOM   2127  CA   ASN   267      50.686  -4.000 -22.423  0.00  0.00

ATOM   2128  C    ASN   267      49.938  -5.072 -21.522  0.00  0.00

ATOM   2129  O    ASN   267      49.060  -5.779 -22.040  0.00  0.00
```

```
ATOM   2130  CB  ASN   267      50.656  -4.439 -23.912  0.00   0.00

ATOM   2131  CG  ASN   267      49.227  -4.372 -24.498  0.00   0.00

ATOM   2132  OD1 ASN   267      48.538  -3.417 -24.205  0.00   0.00

ATOM   2133  ND2 ASN   267      48.764  -5.361 -25.281  0.00   0.00

ATOM   2134  N   LEU   268      50.117  -5.110 -20.172  0.00   0.00

ATOM   2135  CA  LEU   268      49.394  -5.994 -19.281  0.00   0.00

ATOM   2136  C   LEU   268      49.129  -5.163 -17.967  0.00   0.00

ATOM   2137  O   LEU   268      50.049  -5.165 -17.138  0.00   0.00

ATOM   2138  CB  LEU   268      50.281  -7.216 -19.078  0.00   0.00

ATOM   2139  CG  LEU   268      49.764  -8.542 -18.611  0.00   0.00

ATOM   2140  CD1 LEU   268      50.880  -9.519 -18.873  0.00   0.00

ATOM   2141  CD2 LEU   268      49.393  -8.558 -17.128  0.00   0.00

ATOM   2142  N   ILE   269      47.864  -4.788 -17.614  0.00   0.00

ATOM   2143  CA  ILE   269      47.620  -4.054 -16.330  0.00   0.00

ATOM   2144  C   ILE   269      46.155  -3.503 -16.056  0.00   0.00

ATOM   2145  O   ILE   269      45.328  -3.444 -16.975  0.00   0.00

ATOM   2146  CB  ILE   269      48.672  -2.891 -16.204  0.00   0.00

ATOM   2147  CG1 ILE   269      48.741  -2.397 -14.710  0.00   0.00

ATOM   2148  CG2 ILE   269      48.235  -1.706 -17.018  0.00   0.00

ATOM   2149  CD1 ILE   269      49.210  -3.445 -13.712  0.00   0.00

ATOM   2150  N   ARG   270      45.859  -2.948 -14.842  0.00   0.00

ATOM   2151  CA  ARG   270      44.603  -2.293 -14.405  0.00   0.00

ATOM   2152  C   ARG   270      44.962  -0.869 -13.817  0.00   0.00

ATOM   2153  O   ARG   270      45.980  -0.762 -13.111  0.00   0.00

ATOM   2154  CB  ARG   270      43.936  -3.164 -13.328  0.00   0.00
```

```
ATOM   2155  CG   ARG   270    43.393  -4.473 -13.856  0.00  0.00

ATOM   2156  CD   ARG   270    42.279  -4.306 -14.815  0.00  0.00

ATOM   2157  NE   ARG   270    41.726  -5.580 -15.169  0.00  0.00

ATOM   2158  CZ   ARG   270    42.262  -6.381 -16.089  0.00  0.00

ATOM   2159  NH1  ARG   270    43.403  -6.110 -16.693  0.00  0.00

ATOM   2160  NH2  ARG   270    41.657  -7.523 -16.395  0.00  0.00

ATOM   2161  N    LYS   271    44.125   0.185 -13.973  0.00  0.00

ATOM   2162  CA   LYS   271    44.416   1.561 -13.510  0.00  0.00

ATOM   2163  C    LYS   271    44.773   1.678 -12.002  0.00  0.00

ATOM   2164  O    LYS   271    45.869   2.179 -11.752  0.00  0.00

ATOM   2165  CB   LYS   271    43.461   2.619 -14.049  0.00  0.00

ATOM   2166  CG   LYS   271    43.163   2.502 -15.516  0.00  0.00

ATOM   2167  CD   LYS   271    44.546   2.468 -16.218  0.00  0.00

ATOM   2168  CE   LYS   271    44.433   2.347 -17.748  0.00  0.00

ATOM   2169  NZ   LYS   271    45.606   1.513 -18.235  0.00  0.00

ATOM   2170  N    GLU   272    43.947   1.255 -11.060  0.00  0.00

ATOM   2171  CA   GLU   272    44.236   1.442  -9.627  0.00  0.00

ATOM   2172  C    GLU   272    45.627   0.880  -9.156  0.00  0.00

ATOM   2173  O    GLU   272    46.103   1.418  -8.159  0.00  0.00

ATOM   2174  CB   GLU   272    43.110   0.758  -8.824  0.00  0.00

ATOM   2175  CG   GLU   272    41.677   1.022  -9.237  0.00  0.00

ATOM   2176  CD   GLU   272    40.660   1.121  -8.126  0.00  0.00

ATOM   2177  OE1  GLU   272    41.090   1.356  -6.943  0.00  0.00

ATOM   2178  OE2  GLU   272    39.477   0.964  -8.364  0.00  0.00

ATOM   2179  N    VAL   273    46.131  -0.275  -9.652  0.00  0.00
```

```
ATOM    2180  CA   VAL   273      47.490  -0.780  -9.305  0.00  0.00

ATOM    2181  C    VAL   273      48.607   0.346  -9.340  0.00  0.00

ATOM    2182  O    VAL   273      49.265   0.508  -8.312  0.00  0.00

ATOM    2183  CB   VAL   273      47.811  -1.941 -10.258  0.00  0.00

ATOM    2184  CG1  VAL   273      49.118  -2.681  -9.908  0.00  0.00

ATOM    2185  CG2  VAL   273      46.675  -2.962 -10.214  0.00  0.00

ATOM    2186  N    TYR   274      48.750   1.172 -10.416  0.00  0.00

ATOM    2187  CA   TYR   274      49.801   2.204 -10.573  0.00  0.00

ATOM    2188  C    TYR   274      49.445   3.491  -9.714  0.00  0.00

ATOM    2189  O    TYR   274      50.315   3.880  -8.946  0.00  0.00

ATOM    2190  CB   TYR   274      50.052   2.444 -12.087  0.00  0.00

ATOM    2191  CG   TYR   274      51.061   3.556 -12.314  0.00  0.00

ATOM    2192  CD1  TYR   274      50.661   4.809 -12.783  0.00  0.00

ATOM    2193  CD2  TYR   274      52.405   3.283 -12.085  0.00  0.00

ATOM    2194  CE1  TYR   274      51.634   5.778 -12.981  0.00  0.00

ATOM    2195  CE2  TYR   274      53.372   4.263 -12.290  0.00  0.00

ATOM    2196  CZ   TYR   274      52.966   5.509 -12.743  0.00  0.00

ATOM    2197  OH   TYR   274      53.985   6.424 -12.940  0.00  0.00

ATOM    2198  N    ILE   275      48.134   3.768  -9.441  0.00  0.00

ATOM    2199  CA   ILE   275      47.616   4.863  -8.586  0.00  0.00

ATOM    2200  C    ILE   275      48.221   4.817  -7.166  0.00  0.00

ATOM    2201  O    ILE   275      48.543   5.876  -6.636  0.00  0.00

ATOM    2202  CB   ILE   275      46.068   4.638  -8.425  0.00  0.00

ATOM    2203  CG1  ILE   275      45.354   4.632  -9.818  0.00  0.00

ATOM    2204  CG2  ILE   275      45.490   5.833  -7.559  0.00  0.00
```

```
ATOM    2205  CD1 ILE   275      43.787    4.754   -9.902   0.00   0.00

ATOM    2206  N   LEU   276      47.982    3.678   -6.487   0.00   0.00

ATOM    2207  CA  LEU   276      48.335    3.434   -5.062   0.00   0.00

ATOM    2208  C   LEU   276      49.703    3.956   -4.655   0.00   0.00

ATOM    2209  O   LEU   276      49.706    5.034   -4.073   0.00   0.00

ATOM    2210  CB  LEU   276      48.215    1.934   -4.779   0.00   0.00

ATOM    2211  CG  LEU   276      47.026    1.100   -5.128   0.00   0.00

ATOM    2212  CD1 LEU   276      47.317   -0.335   -4.697   0.00   0.00

ATOM    2213  CD2 LEU   276      45.786    1.594   -4.442   0.00   0.00

ATOM    2214  N   LEU   277      50.769    3.602   -5.284   0.00   0.00

ATOM    2215  CA  LEU   277      52.137    3.906   -4.909   0.00   0.00

ATOM    2216  C   LEU   277      52.586    5.292   -5.470   0.00   0.00

ATOM    2217  O   LEU   277      53.652    5.771   -5.064   0.00   0.00

ATOM    2218  CB  LEU   277      52.954    2.835   -5.540   0.00   0.00

ATOM    2219  CG  LEU   277      52.675    1.390   -5.424   0.00   0.00

ATOM    2220  CD1 LEU   277      53.594    0.494   -6.231   0.00   0.00

ATOM    2221  CD2 LEU   277      52.724    0.948   -3.973   0.00   0.00

ATOM    2222  N   ASN   278      51.996    5.703   -6.625   0.00   0.00

ATOM    2223  CA  ASN   278      52.233    7.003   -7.206   0.00   0.00

ATOM    2224  C   ASN   278      51.671    8.090   -6.259   0.00   0.00

ATOM    2225  O   ASN   278      52.444    9.000   -5.928   0.00   0.00

ATOM    2226  CB  ASN   278      51.558    6.984   -8.564   0.00   0.00

ATOM    2227  CG  ASN   278      52.413    6.323   -9.663   0.00   0.00

ATOM    2228  OD1 ASN   278      51.945    5.392  -10.335   0.00   0.00

ATOM    2229  ND2 ASN   278      53.668    6.733   -9.843   0.00   0.00
```

```
ATOM   2230  N   TRP  279    50.348   8.099  -5.942  0.00  0.00
ATOM   2231  CA  TRP  279    49.706   8.997  -4.951  0.00  0.00
ATOM   2232  C   TRP  279    50.449   8.788  -3.592  0.00  0.00
ATOM   2233  O   TRP  279    50.661   9.755  -2.908  0.00  0.00
ATOM   2234  CB  TRP  279    48.212   8.785  -4.804  0.00  0.00
ATOM   2235  CG  TRP  279    47.576   9.902  -3.997  0.00  0.00
ATOM   2236  CD1 TRP  279    47.004  11.047  -4.433  0.00  0.00
ATOM   2237  CD2 TRP  279    47.541   9.963  -2.558  0.00  0.00
ATOM   2238  NE1 TRP  279    46.622  11.838  -3.378  0.00  0.00
ATOM   2239  CE2 TRP  279    46.989  11.203  -2.221  0.00  0.00
ATOM   2240  CE3 TRP  279    47.932   9.051  -1.556  0.00  0.00
ATOM   2241  CZ2 TRP  279    46.795  11.570  -0.882  0.00  0.00
ATOM   2242  CZ3 TRP  279    47.802   9.446  -0.218  0.00  0.00
ATOM   2243  CH2 TRP  279    47.192  10.677   0.103  0.00  0.00
ATOM   2244  N   ILE  280    50.515   7.549  -3.012  0.00  0.00
ATOM   2245  CA  ILE  280    51.164   7.399  -1.670  0.00  0.00
ATOM   2246  C   ILE  280    52.581   8.079  -1.668  0.00  0.00
ATOM   2247  O   ILE  280    52.952   8.603  -0.619  0.00  0.00
ATOM   2248  CB  ILE  280    51.175   5.963  -1.124  0.00  0.00
ATOM   2249  CG1 ILE  280    52.028   5.758   0.112  0.00  0.00
ATOM   2250  CG2 ILE  280    51.781   5.015  -2.185  0.00  0.00
ATOM   2251  CD1 ILE  280    51.440   6.342   1.392  0.00  0.00
ATOM   2252  N   GLY  281    53.409   7.985  -2.729  0.00  0.00
ATOM   2253  CA  GLY  281    54.721   8.646  -2.810  0.00  0.00
ATOM   2254  C   GLY  281    54.671  10.215  -2.731  0.00  0.00
```

```
ATOM   2255  O    GLY  281      55.716  10.838  -2.924  0.00  0.00

ATOM   2256  N    TYR  282      53.489  10.801  -2.785  0.00  0.00

ATOM   2257  CA   TYR  282      53.226  12.249  -2.859  0.00  0.00

ATOM   2258  C    TYR  282      53.560  12.824  -1.417  0.00  0.00

ATOM   2259  O    TYR  282      53.796  14.044  -1.345  0.00  0.00

ATOM   2260  CB   TYR  282      51.673  12.226  -3.085  0.00  0.00

ATOM   2261  CG   TYR  282      51.023  12.220  -4.482  0.00  0.00

ATOM   2262  CD1  TYR  282      51.693  11.956  -5.685  0.00  0.00

ATOM   2263  CD2  TYR  282      49.639  12.471  -4.586  0.00  0.00

ATOM   2264  CE1  TYR  282      51.046  11.916  -6.915  0.00  0.00

ATOM   2265  CE2  TYR  282      48.933  12.432  -5.806  0.00  0.00

ATOM   2266  CZ   TYR  282      49.666  12.144  -6.968  0.00  0.00

ATOM   2267  OH   TYR  282      49.040  12.089  -8.170  0.00  0.00

ATOM   2268  N    VAL  283      53.349  12.058  -0.311  0.00  0.00

ATOM   2269  CA   VAL  283      53.713  12.452   1.002  0.00  0.00

ATOM   2270  C    VAL  283      55.246  12.774   1.024  0.00  0.00

ATOM   2271  O    VAL  283      55.699  13.121   2.117  0.00  0.00

ATOM   2272  CB   VAL  283      53.302  11.357   1.993  0.00  0.00

ATOM   2273  CG1  VAL  283      51.790  11.130   1.989  0.00  0.00

ATOM   2274  CG2  VAL  283      54.021  10.039   1.731  0.00  0.00

ATOM   2275  N    ASN  284      56.079  12.321   0.036  0.00  0.00

ATOM   2276  CA   ASN  284      57.465  12.684  -0.024  0.00  0.00

ATOM   2277  C    ASN  284      57.656  14.230   0.261  0.00  0.00

ATOM   2278  O    ASN  284      58.733  14.585   0.742  0.00  0.00

ATOM   2279  CB   ASN  284      58.096  12.408  -1.409  0.00  0.00
```

| ATOM | 2280 | CG  | ASN | 284 | 57.358 | 13.050 | -2.551 | 0.00 | 0.00 |
| ATOM | 2281 | OD1 | ASN | 284 | 56.326 | 13.699 | -2.484 | 0.00 | 0.00 |
| ATOM | 2282 | ND2 | ASN | 284 | 57.976 | 12.963 | -3.703 | 0.00 | 0.00 |
| ATOM | 2283 | N   | SER | 285 | 56.875 | 15.122 | -0.396 | 0.00 | 0.00 |
| ATOM | 2284 | CA  | SER | 285 | 56.903 | 16.549 | -0.264 | 0.00 | 0.00 |
| ATOM | 2285 | C   | SER | 285 | 56.839 | 16.908 | 1.241 | 0.00 | 0.00 |
| ATOM | 2286 | O   | SER | 285 | 57.919 | 17.014 | 1.809 | 0.00 | 0.00 |
| ATOM | 2287 | CB  | SER | 285 | 55.716 | 17.282 | -0.966 | 0.00 | 0.00 |
| ATOM | 2288 | OG  | SER | 285 | 55.092 | 18.389 | -0.295 | 0.00 | 0.00 |
| ATOM | 2289 | N   | GLY | 286 | 55.795 | 16.460 | 1.927 | 0.00 | 0.00 |
| ATOM | 2290 | CA  | GLY | 286 | 55.564 | 16.802 | 3.281 | 0.00 | 0.00 |
| ATOM | 2291 | C   | GLY | 286 | 56.404 | 16.070 | 4.366 | 0.00 | 0.00 |
| ATOM | 2292 | O   | GLY | 286 | 56.732 | 16.750 | 5.343 | 0.00 | 0.00 |
| ATOM | 2293 | N   | PHE | 287 | 56.912 | 14.841 | 4.154 | 0.00 | 0.00 |
| ATOM | 2294 | CA  | PHE | 287 | 57.622 | 14.062 | 5.182 | 0.00 | 0.00 |
| ATOM | 2295 | C   | PHE | 287 | 58.989 | 14.647 | 5.571 | 0.00 | 0.00 |
| ATOM | 2296 | O   | PHE | 287 | 59.435 | 14.349 | 6.678 | 0.00 | 0.00 |
| ATOM | 2297 | CB  | PHE | 287 | 57.874 | 12.617 | 4.806 | 0.00 | 0.00 |
| ATOM | 2298 | CG  | PHE | 287 | 58.932 | 12.240 | 3.839 | 0.00 | 0.00 |
| ATOM | 2299 | CD1 | PHE | 287 | 58.734 | 12.463 | 2.457 | 0.00 | 0.00 |
| ATOM | 2300 | CD2 | PHE | 287 | 60.143 | 11.724 | 4.305 | 0.00 | 0.00 |
| ATOM | 2301 | CE1 | PHE | 287 | 59.755 | 12.127 | 1.560 | 0.00 | 0.00 |
| ATOM | 2302 | CE2 | PHE | 287 | 61.164 | 11.389 | 3.398 | 0.00 | 0.00 |
| ATOM | 2303 | CZ  | PHE | 287 | 60.962 | 11.597 | 2.016 | 0.00 | 0.00 |
| ATOM | 2304 | N   | ASN | 288 | 59.687 | 15.347 | 4.653 | 0.00 | 0.00 |

| ATOM | 2305 | CA | ASN | 288 | 60.984 | 15.838 | 4.903 | 0.00 | 0.00 |
|------|------|-----|-----|-----|--------|--------|--------|------|------|
| ATOM | 2306 | C | ASN | 288 | 61.089 | 16.686 | 6.212 | 0.00 | 0.00 |
| ATOM | 2307 | O | ASN | 288 | 61.870 | 16.258 | 7.081 | 0.00 | 0.00 |
| ATOM | 2308 | CB | ASN | 288 | 61.354 | 16.617 | 3.680 | 0.00 | 0.00 |
| ATOM | 2309 | CG | ASN | 288 | 61.940 | 15.785 | 2.589 | 0.00 | 0.00 |
| ATOM | 2310 | OD1 | ASN | 288 | 63.134 | 15.559 | 2.557 | 0.00 | 0.00 |
| ATOM | 2311 | ND2 | ASN | 288 | 61.272 | 15.263 | 1.571 | 0.00 | 0.00 |
| ATOM | 2312 | N | PRO | 289 | 60.503 | 17.913 | 6.415 | 0.00 | 0.00 |
| ATOM | 2313 | CA | PRO | 289 | 60.673 | 18.524 | 7.718 | 0.00 | 0.00 |
| ATOM | 2314 | C | PRO | 289 | 59.952 | 17.695 | 8.855 | 0.00 | 0.00 |
| ATOM | 2315 | O | PRO | 289 | 60.210 | 18.045 | 10.012 | 0.00 | 0.00 |
| ATOM | 2316 | CB | PRO | 289 | 60.041 | 19.901 | 7.648 | 0.00 | 0.00 |
| ATOM | 2317 | CG | PRO | 289 | 60.239 | 20.300 | 6.145 | 0.00 | 0.00 |
| ATOM | 2318 | CD | PRO | 289 | 60.154 | 19.011 | 5.382 | 0.00 | 0.00 |
| ATOM | 2319 | N | LEU | 290 | 58.827 | 16.979 | 8.599 | 0.00 | 0.00 |
| ATOM | 2320 | CA | LEU | 290 | 58.241 | 16.136 | 9.655 | 0.00 | 0.00 |
| ATOM | 2321 | C | LEU | 290 | 59.349 | 15.322 | 10.400 | 0.00 | 0.00 |
| ATOM | 2322 | O | LEU | 290 | 59.169 | 15.107 | 11.613 | 0.00 | 0.00 |
| ATOM | 2323 | CB | LEU | 290 | 57.148 | 15.233 | 9.108 | 0.00 | 0.00 |
| ATOM | 2324 | CG | LEU | 290 | 56.015 | 15.864 | 8.342 | 0.00 | 0.00 |
| ATOM | 2325 | CD1 | LEU | 290 | 55.058 | 14.757 | 7.868 | 0.00 | 0.00 |
| ATOM | 2326 | CD2 | LEU | 290 | 55.223 | 16.827 | 9.223 | 0.00 | 0.00 |
| ATOM | 2327 | N | ILE | 291 | 60.291 | 14.685 | 9.651 | 0.00 | 0.00 |
| ATOM | 2328 | CA | ILE | 291 | 61.409 | 13.990 | 10.251 | 0.00 | 0.00 |
| ATOM | 2329 | C | ILE | 291 | 62.342 | 14.914 | 11.131 | 0.00 | 0.00 |

| | | | | | | | | | |
|------|------|-----|-----|-----|--------|--------|--------|------|------|
| ATOM | 2330 | O   | ILE | 291 | 63.009 | 14.329 | 11.996 | 0.00 | 0.00 |
| ATOM | 2331 | CB  | ILE | 291 | 62.214 | 13.293 | 9.106  | 0.00 | 0.00 |
| ATOM | 2332 | CG1 | ILE | 291 | 61.299 | 12.232 | 8.421  | 0.00 | 0.00 |
| ATOM | 2333 | CG2 | ILE | 291 | 63.538 | 12.630 | 9.634  | 0.00 | 0.00 |
| ATOM | 2334 | CD1 | ILE | 291 | 62.003 | 11.632 | 7.161  | 0.00 | 0.00 |
| ATOM | 2335 | N   | TYR | 292 | 62.176 | 16.257 | 11.159 | 0.00 | 0.00 |
| ATOM | 2336 | CA  | TYR | 292 | 63.060 | 17.077 | 12.003 | 0.00 | 0.00 |
| ATOM | 2337 | C   | TYR | 292 | 62.685 | 17.023 | 13.546 | 0.00 | 0.00 |
| ATOM | 2338 | O   | TYR | 292 | 63.146 | 17.817 | 14.349 | 0.00 | 0.00 |
| ATOM | 2339 | CB  | TYR | 292 | 63.100 | 18.487 | 11.322 | 0.00 | 0.00 |
| ATOM | 2340 | CG  | TYR | 292 | 64.441 | 19.164 | 11.676 | 0.00 | 0.00 |
| ATOM | 2341 | CD1 | TYR | 292 | 64.670 | 19.539 | 12.977 | 0.00 | 0.00 |
| ATOM | 2342 | CD2 | TYR | 292 | 65.459 | 19.314 | 10.731 | 0.00 | 0.00 |
| ATOM | 2343 | CE1 | TYR | 292 | 65.865 | 20.064 | 13.372 | 0.00 | 0.00 |
| ATOM | 2344 | CE2 | TYR | 292 | 66.677 | 19.849 | 11.120 | 0.00 | 0.00 |
| ATOM | 2345 | CZ  | TYR | 292 | 66.860 | 20.211 | 12.459 | 0.00 | 0.00 |
| ATOM | 2346 | OH  | TYR | 292 | 68.024 | 20.730 | 12.993 | 0.00 | 0.00 |
| ATOM | 2347 | N   | CYS | 293 | 61.609 | 16.254 | 13.885 | 0.00 | 0.00 |
| ATOM | 2348 | CA  | CYS | 293 | 61.156 | 15.906 | 15.268 | 0.00 | 0.00 |
| ATOM | 2349 | C   | CYS | 293 | 62.382 | 15.353 | 16.066 | 0.00 | 0.00 |
| ATOM | 2350 | O   | CYS | 293 | 62.369 | 15.384 | 17.293 | 0.00 | 0.00 |
| ATOM | 2351 | CB  | CYS | 293 | 59.860 | 15.010 | 15.326 | 0.00 | 0.00 |
| ATOM | 2352 | SG  | CYS | 293 | 58.480 | 16.041 | 16.011 | 0.00 | 0.00 |
| ATOM | 2353 | N   | ARG | 294 | 63.359 | 14.741 | 15.346 | 0.00 | 0.00 |
| ATOM | 2354 | CA  | ARG | 294 | 64.587 | 14.227 | 15.842 | 0.00 | 0.00 |

```
ATOM    2355  C   ARG   294       65.719  15.315  16.077  0.00  0.00

ATOM    2356  O   ARG   294       66.871  14.859  16.297  0.00  0.00

ATOM    2357  CB  ARG   294       65.022  13.178  14.810  0.00  0.00

ATOM    2358  CG  ARG   294       64.114  12.025  14.548  0.00  0.00

ATOM    2359  CD  ARG   294       63.528  11.319  15.771  0.00  0.00

ATOM    2360  NE  ARG   294       64.516  10.399  16.275  0.00  0.00

ATOM    2361  CZ  ARG   294       64.983  10.419  17.492  0.00  0.00

ATOM    2362  NH1 ARG   294       64.572  11.257  18.401  0.00  0.00

ATOM    2363  NH2 ARG   294       65.903   9.557  17.797  0.00  0.00

ATOM    2364  N   SER   295       65.600  16.611  15.807  0.00  0.00

ATOM    2365  CA  SER   295       66.760  17.457  16.187  0.00  0.00

ATOM    2366  C   SER   295       66.261  18.523  17.141  0.00  0.00

ATOM    2367  O   SER   295       65.771  19.526  16.619  0.00  0.00

ATOM    2368  CB  SER   295       67.307  17.985  14.905  0.00  0.00

ATOM    2369  OG  SER   295       68.527  18.521  14.611  0.00  0.00

ATOM    2370  N   PRO   296       66.431  18.481  18.494  0.00  0.00

ATOM    2371  CA  PRO   296       65.960  19.644  19.221  0.00  0.00

ATOM    2372  C   PRO   296       66.698  20.907  18.800  0.00  0.00

ATOM    2373  O   PRO   296       66.236  21.960  19.234  0.00  0.00

ATOM    2374  CB  PRO   296       66.166  19.516  20.762  0.00  0.00

ATOM    2375  CG  PRO   296       67.030  18.319  20.791  0.00  0.00

ATOM    2376  CD  PRO   296       67.054  17.519  19.458  0.00  0.00

ATOM    2377  N   ASP   297       67.808  20.910  18.009  0.00  0.00

ATOM    2378  CA  ASP   297       68.351  22.227  17.581  0.00  0.00

ATOM    2379  C   ASP   297       67.282  23.057  16.788  0.00  0.00
```

111

| | | | | | | | | | |
|---|---|---|---|---|---|---|---|---|---|
| ATOM | 2380 | O | ASP | 297 | 67.032 | 24.199 | 17.174 | 0.00 | 0.00 |
| ATOM | 2381 | CB | ASP | 297 | 69.638 | 21.952 | 16.784 | 0.00 | 0.00 |
| ATOM | 2382 | CG | ASP | 297 | 70.446 | 23.184 | 16.410 | 0.00 | 0.00 |
| ATOM | 2383 | OD1 | ASP | 297 | 69.919 | 24.229 | 16.061 | 0.00 | 0.00 |
| ATOM | 2384 | OD2 | ASP | 297 | 71.660 | 23.077 | 16.488 | 0.00 | 0.00 |
| ATOM | 2385 | N | PHE | 298 | 66.727 | 22.582 | 15.630 | 0.00 | 0.00 |
| ATOM | 2386 | CA | PHE | 298 | 65.651 | 23.239 | 14.838 | 0.00 | 0.00 |
| ATOM | 2387 | C | PHE | 298 | 64.318 | 23.282 | 15.583 | 0.00 | 0.00 |
| ATOM | 2388 | O | PHE | 298 | 63.574 | 24.189 | 15.267 | 0.00 | 0.00 |
| ATOM | 2389 | CB | PHE | 298 | 65.485 | 22.706 | 13.441 | 0.00 | 0.00 |
| ATOM | 2390 | CG | PHE | 298 | 64.597 | 23.488 | 12.539 | 0.00 | 0.00 |
| ATOM | 2391 | CD1 | PHE | 298 | 63.355 | 22.956 | 12.173 | 0.00 | 0.00 |
| ATOM | 2392 | CD2 | PHE | 298 | 64.948 | 24.766 | 12.091 | 0.00 | 0.00 |
| ATOM | 2393 | CE1 | PHE | 298 | 62.485 | 23.667 | 11.345 | 0.00 | 0.00 |
| ATOM | 2394 | CE2 | PHE | 298 | 64.097 | 25.506 | 11.251 | 0.00 | 0.00 |
| ATOM | 2395 | CZ | PHE | 298 | 62.852 | 24.936 | 10.884 | 0.00 | 0.00 |
| ATOM | 2396 | N | ARG | 299 | 63.763 | 22.110 | 15.990 | 0.00 | 0.00 |
| ATOM | 2397 | CA | ARG | 299 | 62.545 | 22.183 | 16.797 | 0.00 | 0.00 |
| ATOM | 2398 | C | ARG | 299 | 62.565 | 23.296 | 17.864 | 0.00 | 0.00 |
| ATOM | 2399 | O | ARG | 299 | 61.515 | 23.934 | 17.985 | 0.00 | 0.00 |
| ATOM | 2400 | CB | ARG | 299 | 62.093 | 20.863 | 17.378 | 0.00 | 0.00 |
| ATOM | 2401 | CG | ARG | 299 | 60.677 | 20.767 | 17.804 | 0.00 | 0.00 |
| ATOM | 2402 | CD | ARG | 299 | 60.299 | 19.327 | 18.181 | 0.00 | 0.00 |
| ATOM | 2403 | NE | ARG | 299 | 58.932 | 19.361 | 18.627 | 0.00 | 0.00 |
| ATOM | 2404 | CZ | ARG | 299 | 57.915 | 19.224 | 17.783 | 0.00 | 0.00 |

| | | | | | | | | |
|---|---|---|---|---|---|---|---|---|
| ATOM | 2405 | NH1 | ARG | 299 | 58.067 | 19.018 | 16.487 | 0.00 | 0.00 |
| ATOM | 2406 | NH2 | ARG | 299 | 56.690 | 19.488 | 18.221 | 0.00 | 0.00 |
| ATOM | 2407 | N | ILE | 300 | 63.569 | 23.380 | 18.791 | 0.00 | 0.00 |
| ATOM | 2408 | CA | ILE | 300 | 63.611 | 24.528 | 19.734 | 0.00 | 0.00 |
| ATOM | 2409 | C | ILE | 300 | 63.586 | 25.893 | 18.950 | 0.00 | 0.00 |
| ATOM | 2410 | O | ILE | 300 | 63.016 | 26.840 | 19.511 | 0.00 | 0.00 |
| ATOM | 2411 | CB | ILE | 300 | 64.762 | 24.377 | 20.773 | 0.00 | 0.00 |
| ATOM | 2412 | CG1 | ILE | 300 | 64.493 | 25.285 | 22.012 | 0.00 | 0.00 |
| ATOM | 2413 | CG2 | ILE | 300 | 66.152 | 24.667 | 20.162 | 0.00 | 0.00 |
| ATOM | 2414 | CD1 | ILE | 300 | 65.538 | 25.020 | 23.115 | 0.00 | 0.00 |
| ATOM | 2415 | N | ALA | 301 | 64.238 | 26.045 | 17.779 | 0.00 | 0.00 |
| ATOM | 2416 | CA | ALA | 301 | 64.211 | 27.191 | 16.930 | 0.00 | 0.00 |
| ATOM | 2417 | C | ALA | 301 | 62.782 | 27.423 | 16.286 | 0.00 | 0.00 |
| ATOM | 2418 | O | ALA | 301 | 62.426 | 28.618 | 16.175 | 0.00 | 0.00 |
| ATOM | 2419 | CB | ALA | 301 | 65.210 | 26.859 | 15.785 | 0.00 | 0.00 |
| ATOM | 2420 | N | PHE | 302 | 62.065 | 26.409 | 15.708 | 0.00 | 0.00 |
| ATOM | 2421 | CA | PHE | 302 | 60.714 | 26.833 | 15.240 | 0.00 | 0.00 |
| ATOM | 2422 | C | PHE | 302 | 59.801 | 27.261 | 16.410 | 0.00 | 0.00 |
| ATOM | 2423 | O | PHE | 302 | 58.830 | 27.948 | 16.099 | 0.00 | 0.00 |
| ATOM | 2424 | CB | PHE | 302 | 60.075 | 25.893 | 14.194 | 0.00 | 0.00 |
| ATOM | 2425 | CG | PHE | 302 | 59.848 | 24.468 | 14.770 | 0.00 | 0.00 |
| ATOM | 2426 | CD1 | PHE | 302 | 60.754 | 23.474 | 14.424 | 0.00 | 0.00 |
| ATOM | 2427 | CD2 | PHE | 302 | 58.746 | 24.177 | 15.569 | 0.00 | 0.00 |
| ATOM | 2428 | CE1 | PHE | 302 | 60.542 | 22.187 | 14.889 | 0.00 | 0.00 |
| ATOM | 2429 | CE2 | PHE | 302 | 58.560 | 22.889 | 16.020 | 0.00 | 0.00 |

| | | | | | | | | | |
|---|---|---|---|---|---|---|---|---|---|
| ATOM | 2430 | CZ | PHE | 302 | 59.455 | 21.893 | 15.683 | 0.00 | 0.00 |
| ATOM | 2431 | N | GLN | 303 | 59.904 | 26.698 | 17.634 | 0.00 | 0.00 |
| ATOM | 2432 | CA | GLN | 303 | 59.123 | 27.146 | 18.813 | 0.00 | 0.00 |
| ATOM | 2433 | C | GLN | 303 | 59.517 | 28.620 | 19.138 | 0.00 | 0.00 |
| ATOM | 2434 | O | GLN | 303 | 58.661 | 29.365 | 19.632 | 0.00 | 0.00 |
| ATOM | 2435 | CB | GLN | 303 | 59.365 | 26.232 | 20.010 | 0.00 | 0.00 |
| ATOM | 2436 | CG | GLN | 303 | 58.873 | 24.828 | 19.752 | 0.00 | 0.00 |
| ATOM | 2437 | CD | GLN | 303 | 59.077 | 23.945 | 20.967 | 0.00 | 0.00 |
| ATOM | 2438 | OE1 | GLN | 303 | 58.269 | 23.987 | 21.885 | 0.00 | 0.00 |
| ATOM | 2439 | NE2 | GLN | 303 | 60.152 | 23.186 | 20.988 | 0.00 | 0.00 |
| ATOM | 2440 | N | GLU | 304 | 60.794 | 29.003 | 19.007 | 0.00 | 0.00 |
| ATOM | 2441 | CA | GLU | 304 | 61.303 | 30.354 | 19.170 | 0.00 | 0.00 |
| ATOM | 2442 | C | GLU | 304 | 60.581 | 31.341 | 18.184 | 0.00 | 0.00 |
| ATOM | 2443 | O | GLU | 304 | 60.492 | 32.513 | 18.544 | 0.00 | 0.00 |
| ATOM | 2444 | CB | GLU | 304 | 62.814 | 30.381 | 19.090 | 0.00 | 0.00 |
| ATOM | 2445 | CG | GLU | 304 | 63.478 | 31.639 | 19.595 | 0.00 | 0.00 |
| ATOM | 2446 | CD | GLU | 304 | 63.473 | 31.839 | 21.066 | 0.00 | 0.00 |
| ATOM | 2447 | OE1 | GLU | 304 | 63.507 | 32.913 | 21.649 | 0.00 | 0.00 |
| ATOM | 2448 | OE2 | GLU | 304 | 63.513 | 30.768 | 21.725 | 0.00 | 0.00 |
| ATOM | 2449 | N | LEU | 305 | 60.516 | 31.021 | 16.860 | 0.00 | 0.00 |
| ATOM | 2450 | CA | LEU | 305 | 59.767 | 31.857 | 15.900 | 0.00 | 0.00 |
| ATOM | 2451 | C | LEU | 305 | 58.256 | 31.822 | 16.170 | 0.00 | 0.00 |
| ATOM | 2452 | O | LEU | 305 | 57.667 | 32.907 | 16.090 | 0.00 | 0.00 |
| ATOM | 2453 | CB | LEU | 305 | 60.008 | 31.530 | 14.376 | 0.00 | 0.00 |
| ATOM | 2454 | CG | LEU | 305 | 59.184 | 32.344 | 13.310 | 0.00 | 0.00 |

| ATOM | 2455 | CD1 | LEU | 305 | 59.606 | 33.796 | 13.228 | 0.00 | 0.00 |
|------|------|-----|-----|-----|--------|--------|--------|------|------|
| ATOM | 2456 | CD2 | LEU | 305 | 59.407 | 31.680 | 11.935 | 0.00 | 0.00 |
| ATOM | 2457 | N   | LEU | 306 | 57.599 | 30.636 | 16.264 | 0.00 | 0.00 |
| ATOM | 2458 | CA  | LEU | 306 | 56.146 | 30.615 | 16.525 | 0.00 | 0.00 |
| ATOM | 2459 | C   | LEU | 306 | 55.799 | 31.622 | 17.698 | 0.00 | 0.00 |
| ATOM | 2460 | O   | LEU | 306 | 54.863 | 32.405 | 17.497 | 0.00 | 0.00 |
| ATOM | 2461 | CB  | LEU | 306 | 55.637 | 29.179 | 16.733 | 0.00 | 0.00 |
| ATOM | 2462 | CG  | LEU | 306 | 55.784 | 28.300 | 15.503 | 0.00 | 0.00 |
| ATOM | 2463 | CD1 | LEU | 306 | 55.370 | 26.899 | 15.867 | 0.00 | 0.00 |
| ATOM | 2464 | CD2 | LEU | 306 | 54.919 | 28.815 | 14.366 | 0.00 | 0.00 |
| ATOM | 2465 | N   | CYS | 307 | 56.347 | 31.433 | 18.898 | 0.00 | 0.00 |
| ATOM | 2466 | CA  | CYS | 307 | 56.116 | 32.388 | 19.902 | 0.00 | 0.00 |
| ATOM | 2467 | C   | CYS | 307 | 57.186 | 33.428 | 19.641 | 0.00 | 0.00 |
| ATOM | 2468 | O   | CYS | 307 | 58.354 | 33.106 | 19.927 | 0.00 | 0.00 |
| ATOM | 2469 | CB  | CYS | 307 | 56.230 | 31.815 | 21.309 | 0.00 | 0.00 |
| ATOM | 2470 | SG  | CYS | 307 | 54.827 | 30.888 | 21.901 | 0.00 | 0.00 |
| ATOM | 2471 | N   | LEU | 308 | 56.863 | 34.660 | 19.313 | 0.00 | 0.00 |
| ATOM | 2472 | CA  | LEU | 308 | 57.935 | 35.567 | 18.975 | 0.00 | 0.00 |
| ATOM | 2473 | C   | LEU | 308 | 58.904 | 35.717 | 20.173 | 0.00 | 0.00 |
| ATOM | 2474 | O   | LEU | 308 | 58.567 | 36.270 | 21.235 | 0.00 | 0.00 |
| ATOM | 2475 | CB  | LEU | 308 | 57.351 | 36.943 | 18.658 | 0.00 | 0.00 |
| ATOM | 2476 | CG  | LEU | 308 | 56.351 | 36.951 | 17.547 | 0.00 | 0.00 |
| ATOM | 2477 | CD1 | LEU | 308 | 55.967 | 38.398 | 17.216 | 0.00 | 0.00 |
| ATOM | 2478 | CD2 | LEU | 308 | 56.936 | 36.272 | 16.295 | 0.00 | 0.00 |
| ATOM | 2479 | N   | ARG | 309 | 60.121 | 35.353 | 19.863 | 0.00 | 0.00 |

ATOM 2480 CA ARG 309 61.301 35.437 20.713 0.00 0.00

ATOM 2481 C ARG 309 61.201 34.837 22.150 0.00 0.00

ATOM 2482 O ARG 309 62.158 35.080 22.905 0.00 0.00

ATOM 2483 CB ARG 309 61.427 36.995 20.897 0.00 0.00

ATOM 2484 CG ARG 309 61.330 37.803 19.646 0.00 0.00

ATOM 2485 CD ARG 309 61.623 39.285 19.834 0.00 0.00

ATOM 2486 NE ARG 309 62.987 39.543 20.307 0.00 0.00

ATOM 2487 CZ ARG 309 64.045 39.626 19.496 0.00 0.00

ATOM 2488 NH1 ARG 309 63.906 39.463 18.172 0.00 0.00

ATOM 2489 NH2 ARG 309 65.293 39.875 19.918 0.00 0.00

ATOM 2490 N ARG 310 60.416 33.778 22.419 0.00 0.00

ATOM 2491 CA ARG 310 60.238 33.280 23.776 0.00 0.00

ATOM 2492 C ARG 310 60.046 31.748 23.834 0.00 0.00

ATOM 2493 O ARG 310 59.157 31.206 23.159 0.00 0.00

ATOM 2494 CB ARG 310 58.993 33.952 24.356 0.00 0.00

ATOM 2495 CG ARG 310 59.138 35.402 24.730 0.00 0.00

ATOM 2496 CD ARG 310 57.772 36.041 25.028 0.00 0.00

ATOM 2497 NE ARG 310 57.075 35.512 26.208 0.00 0.00

ATOM 2498 CZ ARG 310 57.353 35.859 27.475 0.00 0.00

ATOM 2499 NH1 ARG 310 56.596 35.415 28.479 0.00 0.00

ATOM 2500 NH2 ARG 310 58.379 36.646 27.770 0.00 0.00

ATOM 2501 N SER 311 60.830 31.111 24.681 0.00 0.00

ATOM 2502 CA SER 311 60.739 29.678 24.960 0.00 0.00

ATOM 2503 C SER 311 60.478 29.493 26.481 0.00 0.00

ATOM 2504 O SER 311 61.083 30.235 27.281 0.00 0.00

```
ATOM    2505  CB   SER   311      62.046  28.975  24.529  0.00  0.00

ATOM    2506  OG   SER   311      62.082  27.568  24.804  0.00  0.00

ATOM    2507  N    SER   312      59.574  28.548  26.855  0.00  0.00

ATOM    2508  CA   SER   312      59.250  28.452  28.274  0.00  0.00

ATOM    2509  C    SER   312      60.290  27.586  29.083  0.00  0.00

ATOM    2510  O    SER   312      59.838  26.876  30.006  0.00  0.00

ATOM    2511  CB   SER   312      57.822  27.866  28.324  0.00  0.00

ATOM    2512  OG   SER   312      56.866  28.432  27.441  0.00  0.00

ATOM    2513  N    LEU   313      61.479  28.196  29.207  0.00  0.00

ATOM    2514  CA   LEU   313      62.666  27.715  29.860  0.00  0.00

ATOM    2515  C    LEU   313      63.651  28.918  29.867  0.00  0.00

ATOM    2516  O    LEU   313      63.540  29.839  29.011  0.00  0.00

ATOM    2517  CB   LEU   313      63.248  26.479  29.131  0.00  0.00

ATOM    2518  CG   LEU   313      62.667  25.083  29.286  0.00  0.00

ATOM    2519  CD1  LEU   313      61.711  24.789  30.426  0.00  0.00

ATOM    2520  CD2  LEU   313      62.075  24.639  27.976  0.00  0.00

ATOM    2521  N    LYS   314      64.725  28.877  30.661  0.00  0.00

ATOM    2522  CA   LYS   314      65.674  29.977  30.551  0.00  0.00

ATOM    2523  C    LYS   314      66.544  29.936  29.259  0.00  0.00

ATOM    2524  O    LYS   314      67.164  30.972  28.998  0.00  0.00

ATOM    2525  CB   LYS   314      66.557  29.897  31.815  0.00  0.00

ATOM    2526  CG   LYS   314      65.847  30.215  33.122  0.00  0.00

ATOM    2527  CD   LYS   314      65.146  31.579  33.225  0.00  0.00

ATOM    2528  CE   LYS   314      64.150  31.544  34.398  0.00  0.00

ATOM    2529  NZ   LYS   314      63.338  32.757  34.503  0.00  0.00
```

| ATOM | 2530 | N | ALA | 315 | 66.407 | 28.939 | 28.348 | 0.00 | 0.00 |
|------|------|------|------|-----|--------|--------|--------|------|------|
| ATOM | 2531 | CA | ALA | 315 | 67.281 | 28.832 | 27.199 | 0.00 | 0.00 |
| ATOM | 2532 | C | ALA | 315 | 68.770 | 28.840 | 27.725 | 0.00 | 0.00 |
| ATOM | 2533 | O | ALA | 315 | 69.664 | 29.342 | 27.033 | 0.00 | 0.00 |
| ATOM | 2534 | CB | ALA | 315 | 66.978 | 29.922 | 26.178 | 0.00 | 0.00 |
| ATOM | 2535 | N | TYR | 316 | 69.035 | 28.128 | 28.852 | 0.00 | 0.00 |
| ATOM | 2536 | CA | TYR | 316 | 70.279 | 28.007 | 29.503 | 0.00 | 0.00 |
| ATOM | 2537 | C | TYR | 316 | 71.174 | 27.242 | 28.525 | 0.00 | 0.00 |
| ATOM | 2538 | O | TYR | 316 | 70.711 | 26.194 | 28.026 | 0.00 | 0.00 |
| ATOM | 2539 | CB | TYR | 316 | 70.033 | 27.327 | 30.847 | 0.00 | 0.00 |
| ATOM | 2540 | CG | TYR | 316 | 71.171 | 27.314 | 31.819 | 0.00 | 0.00 |
| ATOM | 2541 | CD1 | TYR | 316 | 72.071 | 26.266 | 31.818 | 0.00 | 0.00 |
| ATOM | 2542 | CD2 | TYR | 316 | 71.237 | 28.298 | 32.783 | 0.00 | 0.00 |
| ATOM | 2543 | CE1 | TYR | 316 | 73.049 | 26.174 | 32.798 | 0.00 | 0.00 |
| ATOM | 2544 | CE2 | TYR | 316 | 72.216 | 28.211 | 33.772 | 0.00 | 0.00 |
| ATOM | 2545 | CZ | TYR | 316 | 73.102 | 27.153 | 33.760 | 0.00 | 0.00 |
| ATOM | 2546 | OH | TYR | 316 | 74.061 | 27.036 | 34.726 | 0.00 | 0.00 |
| ATOM | 2547 | N | GLY | 317 | 72.482 | 27.401 | 28.684 | 0.00 | 0.00 |
| ATOM | 2548 | CA | GLY | 317 | 73.328 | 26.788 | 27.720 | 0.00 | 0.00 |
| ATOM | 2549 | C | GLY | 317 | 73.384 | 25.252 | 27.813 | 0.00 | 0.00 |
| ATOM | 2550 | O | GLY | 317 | 74.343 | 24.732 | 28.392 | 0.00 | 0.00 |
| ATOM | 2551 | N | ASN | 318 | 72.837 | 24.746 | 26.765 | 0.00 | 0.00 |
| ATOM | 2552 | CA | ASN | 318 | 72.720 | 23.385 | 26.324 | 0.00 | 0.00 |
| ATOM | 2553 | C | ASN | 318 | 72.034 | 23.469 | 24.919 | 0.00 | 0.00 |
| ATOM | 2554 | O | ASN | 318 | 70.982 | 24.167 | 24.862 | 0.00 | 0.00 |

| | | | | | | | | | |
|---|---|---|---|---|---|---|---|---|---|
| ATOM | 2555 | CB | ASN | 318 | 71.880 | 22.515 | 27.271 | 0.00 | 0.00 |
| ATOM | 2556 | CG | ASN | 318 | 72.541 | 22.233 | 28.577 | 0.00 | 0.00 |
| ATOM | 2557 | OD1 | ASN | 318 | 71.839 | 22.169 | 29.579 | 0.00 | 0.00 |
| ATOM | 2558 | ND2 | ASN | 318 | 73.857 | 22.123 | 28.649 | 0.00 | 0.00 |
| ATOM | 2559 | N | GLY | 319 | 72.190 | 22.504 | 24.017 | 0.00 | 0.00 |
| ATOM | 2560 | CA | GLY | 319 | 71.492 | 22.665 | 22.753 | 0.00 | 0.00 |
| ATOM | 2561 | C | GLY | 319 | 71.934 | 24.051 | 22.171 | 0.00 | 0.00 |
| ATOM | 2562 | O | GLY | 319 | 71.027 | 24.887 | 22.035 | 0.00 | 0.00 |
| ATOM | 2563 | N | TYR | 320 | 73.239 | 24.353 | 21.979 | 0.00 | 0.00 |
| ATOM | 2564 | CA | TYR | 320 | 73.773 | 25.621 | 21.548 | 0.00 | 0.00 |
| ATOM | 2565 | C | TYR | 320 | 72.730 | 26.352 | 20.695 | 0.00 | 0.00 |
| ATOM | 2566 | O | TYR | 320 | 72.457 | 25.975 | 19.542 | 0.00 | 0.00 |
| ATOM | 2567 | CB | TYR | 320 | 75.123 | 25.390 | 20.841 | 0.00 | 0.00 |
| ATOM | 2568 | CG | TYR | 320 | 76.327 | 25.649 | 21.751 | 0.00 | 0.00 |
| ATOM | 2569 | CD1 | TYR | 320 | 77.566 | 25.900 | 21.202 | 0.00 | 0.00 |
| ATOM | 2570 | CD2 | TYR | 320 | 76.182 | 25.565 | 23.118 | 0.00 | 0.00 |
| ATOM | 2571 | CE1 | TYR | 320 | 78.676 | 26.054 | 22.021 | 0.00 | 0.00 |
| ATOM | 2572 | CE2 | TYR | 320 | 77.285 | 25.721 | 23.947 | 0.00 | 0.00 |
| ATOM | 2573 | CZ | TYR | 320 | 78.532 | 25.961 | 23.393 | 0.00 | 0.00 |
| ATOM | 2574 | OH | TYR | 320 | 79.679 | 26.091 | 24.244 | 0.00 | 0.00 |
| ATOM | 2575 | N | SER | 321 | 72.426 | 27.546 | 21.202 | 0.00 | 0.00 |
| ATOM | 2576 | CA | SER | 321 | 71.390 | 28.398 | 20.647 | 0.00 | 0.00 |
| ATOM | 2577 | C | SER | 321 | 71.956 | 29.150 | 19.439 | 0.00 | 0.00 |
| ATOM | 2578 | O | SER | 321 | 72.508 | 30.242 | 19.539 | 0.00 | 0.00 |
| ATOM | 2579 | CB | SER | 321 | 70.831 | 29.297 | 21.761 | 0.00 | 0.00 |

```
ATOM    2580   OG   SER   321      69.425  29.191  22.005   0.00   0.00

ATOM    2581   N    SER   322      71.553  28.628  18.308   0.00   0.00

ATOM    2582   CA   SER   322      71.906  29.091  16.982   0.00   0.00

ATOM    2583   C    SER   322      70.747  29.807  16.199   0.00   0.00

ATOM    2584   O    SER   322      70.860  29.872  14.974   0.00   0.00

ATOM    2585   CB   SER   322      72.510  27.862  16.286   0.00   0.00

ATOM    2586   OG   SER   322      71.613  26.757  16.125   0.00   0.00

ATOM    2587   N    ASN   323      69.613  30.058  16.761   0.00   0.00

ATOM    2588   CA   ASN   323      68.567  30.779  16.162   0.00   0.00

ATOM    2589   C    ASN   323      69.027  32.252  16.015   0.00   0.00

ATOM    2590   O    ASN   323      69.981  32.711  16.699   0.00   0.00

ATOM    2591   CB   ASN   323      67.461  30.781  17.191   0.00   0.00

ATOM    2592   CG   ASN   323      67.177  29.878  18.342   0.00   0.00

ATOM    2593   OD1  ASN   323      67.757  28.790  18.677   0.00   0.00

ATOM    2594   ND2  ASN   323      66.351  30.336  19.312   0.00   0.00

ATOM    2595   N    GLY   324      68.393  32.982  15.113   0.00   0.00

ATOM    2596   CA   GLY   324      68.742  34.412  15.039   0.00   0.00

ATOM    2597   C    GLY   324      68.606  35.080  16.445   0.00   0.00

ATOM    2598   O    GLY   324      69.173  36.167  16.597   0.00   0.00

ATOM    2599   N    ASN   325      67.569  34.696  17.211   0.00   0.00

ATOM    2600   CA   ASN   325      67.299  35.108  18.553   0.00   0.00

ATOM    2601   C    ASN   325      68.175  34.289  19.555   0.00   0.00

ATOM    2602   O    ASN   325      68.598  33.160  19.247   0.00   0.00

ATOM    2603   CB   ASN   325      65.835  34.723  18.792   0.00   0.00

ATOM    2604   CG   ASN   325      64.857  35.706  18.178   0.00   0.00
```

| | | | | | | | | | |
|---|---|---|---|---|---|---|---|---|---|
| ATOM | 2605 | OD1 | ASN | 325 | 63.634 | 35.526 | 18.329 | 0.00 | 0.00 |
| ATOM | 2606 | ND2 | ASN | 325 | 65.214 | 36.745 | 17.401 | 0.00 | 0.00 |
| ATOM | 2607 | N | THR | 326 | 68.206 | 34.714 | 20.817 | 0.00 | 0.00 |
| ATOM | 2608 | CA | THR | 326 | 68.895 | 34.008 | 21.922 | 0.00 | 0.00 |
| ATOM | 2609 | C | THR | 326 | 70.413 | 33.767 | 21.675 | 0.00 | 0.00 |
| ATOM | 2610 | O | THR | 326 | 70.793 | 32.678 | 21.201 | 0.00 | 0.00 |
| ATOM | 2611 | CB | THR | 326 | 68.151 | 32.674 | 22.275 | 0.00 | 0.00 |
| ATOM | 2612 | OG1 | THR | 326 | 66.717 | 32.785 | 22.294 | 0.00 | 0.00 |
| ATOM | 2613 | CG2 | THR | 326 | 68.626 | 32.016 | 23.614 | 0.00 | 0.00 |
| ATOM | 2614 | N | GLY | 327 | 71.182 | 34.874 | 21.579 | 0.00 | 0.00 |
| ATOM | 2615 | CA | GLY | 327 | 72.611 | 34.764 | 21.424 | 0.00 | 0.00 |
| ATOM | 2616 | C | GLY | 327 | 73.096 | 34.584 | 19.965 | 0.00 | 0.00 |
| ATOM | 2617 | O | GLY | 327 | 73.803 | 33.587 | 19.755 | 0.00 | 0.00 |
| ATOM | 2618 | N | GLU | 328 | 72.479 | 35.240 | 18.940 | 0.00 | 0.00 |
| ATOM | 2619 | CA | GLU | 328 | 72.913 | 35.171 | 17.564 | 0.00 | 0.00 |
| ATOM | 2620 | C | GLU | 328 | 74.480 | 35.212 | 17.396 | 0.00 | 0.00 |
| ATOM | 2621 | O | GLU | 328 | 74.920 | 34.645 | 16.395 | 0.00 | 0.00 |
| ATOM | 2622 | CB | GLU | 328 | 72.166 | 36.287 | 16.824 | 0.00 | 0.00 |
| ATOM | 2623 | CG | GLU | 328 | 72.969 | 37.630 | 16.948 | 0.00 | 0.00 |
| ATOM | 2624 | CD | GLU | 328 | 72.145 | 38.850 | 16.655 | 0.00 | 0.00 |
| ATOM | 2625 | OE1 | GLU | 328 | 72.117 | 39.178 | 15.440 | 0.00 | 0.00 |
| ATOM | 2626 | OE2 | GLU | 328 | 71.563 | 39.479 | 17.538 | 0.00 | 0.00 |
| ATOM | 2627 | N | GLN | 329 | 75.254 | 36.040 | 18.150 | 0.00 | 0.00 |
| ATOM | 2628 | CA | GLN | 329 | 76.717 | 36.092 | 18.148 | 0.00 | 0.00 |
| ATOM | 2629 | C | GLN | 329 | 77.347 | 34.799 | 17.577 | 0.00 | 0.00 |

```
ATOM   2630  O   GLN  329      77.228  33.699  18.114  0.00  0.00

ATOM   2631  CB  GLN  329      77.241  36.291  19.567  0.00  0.00

ATOM   2632  CG  GLN  329      76.507  37.286  20.443  0.00  0.00

ATOM   2633  CD  GLN  329      75.835  38.541  19.964  0.00  0.00

ATOM   2634  OE1 GLN  329      76.479  39.546  19.598  0.00  0.00

ATOM   2635  NE2 GLN  329      74.458  38.613  20.013  0.00  0.00

ATOM   2636  OXT GLN  329      77.961  34.937  16.519  0.00  0.00

TER    2637      GLN  329

END
```

Based on the resulting atomic coordinates, the steric structure model of QRHUB2 (β2 adrenaline receptor) was produced using BIOCES (version 3.10) manufactured by NEC Corporation, and a position into which the agonist (isoproterenol) is placed and a position into which the antagonist (propranolol) is placed were compared. The results are shown in Figs. 6 to 14.

From Figs. 6 to 9, it is seen that this antagonist (isoproterenol) has optical isomers (R isomer and S isomer), and the R isomer binds with the receptor better. From Figs. 10 to 13, it is seen that a position of antagonist (propranolol) binding is clearly different from a position of agonist (isopretenol) binding.

Fig.14 shows relative arrangement in the receptor of the antagonist (propranolol) (left) and the agonist (isoproterenol) (right) in the β2 adrenaline receptor obtained from the above-mentioned model. From Fig.14, it is seen that the agonist and the antagonist are introduced into pockets at different positions, respectively, as predicted from an experiment of site-specific mutation.

Example 2 Construction of steric structures of amino acid sequences registered in GCRDb, GPCRDB, ExPASy and ORDB

Regarding amino acid sequences of all GPCRs registered in GCRDb, GPCRDB, ExPASy and ORDB, atomic coordinates defining the steric structure were obtained using 1F88 as a reference protein as in Example 1. Each amino acid sequence was obtained from GCRDb: http://www.gcrdb.uthscsa.edu/, GPCRDB: http://www.gpcr.org/7tm/, ExPASy: http:// www.expasy.ch/cgi-bin/sm-gpcr.pl, ORDB:
http://ycmi.med.yale.edu/senselab/ordb/. In alignments of these amino acid sequences, the E values calculated by PSI-BLAST were as follows:

Table 2

|        | 1e-3〜1e-10 | 1e-11〜1e-50 | 1e-51〜1e-100 | 1e-100 or less | Total |
|--------|-----------|------------|--------------|----------------|-------|
| GCRDb  | 5         | 71         | 709          | 46             | 831   |
| GPCRDB | 0         | 0          | 13           | 570            | 583   |
| ExPASY | 0         | . 15       | 44           | 318            | 377   |
| ORDB   | 0         | 78         | 232          | 107            | 417   |

There was one E value of 0.001 (1e-3) or more in GPCRDB and ORDB, respectively, and these were removed from the Table 2. Therefore, the number of GPCRs for which the steric structure was produced was as follows: GCRDb:

831, GPCRDB:584, ORDB:418, ExPASY:377 (total: 2210).

Example 3 Construction of steric structures of amino acid sequences registered in GeneBank

Regarding sequences of human, Drosophila, nematode, yeast, thearecress and the like registered in databases such as GeneBank and the like, atomic coordinates defining the steric structure were obtained as in Example 1. Alignment was performed by employing PSAI-BLAST and using 1F88 as a reference protein as in Example 1 and, when the E value was 0.1 or less, it was judged to be GPCRs. Sequences of Drosophila, nematode, yeast and thearecress were obtained from GeneBank: ftp://ncbi.nlm.nih.gov/genbank/genomes/, and a sequence of human was obtained from

ftp://ftp.expasy.ch/databases/swiss-prot/special selection/human.seq, http://cdna.ims.u-tokyo.ac.jp/.

The numbers of sequences for which construction of the steric structure was performed are as follows:

Table 3

| | Registered sequence number | Total ORF number | Overlapped ORF number | Non-overlapped ORF number |
|---|---|---|---|---|
| Human | 6032 | 272 | 156 | 116 |
| Drosophila | 14100 | 147 | 5 | 142 |
| Nematode | 15808 | 541 | 19 | 522 |
| Thearecress | 403 | 5 | 0 | 5 |
| Yeast | 6295 | 5 | 1 | 4 |

In Table 3, the "registered sequence number" denotes the number of all sequences registered in databases, the "total ORF number" denotes the number of all ORFs (Open Reading Frame) which were determined to be GPCRs, the "overlapped ORF number" denotes the number of ORFs overlapped with sequences of the above four databases (GCRDb, GPCRDb, ORDB, ExPASy), and the "non-overlapped ORF number" denotes the number of ORFs which were not overlapped with sequences of the above-mentioned four databases and were newly determined to be GPCRs, and for which atomic coordinates defining the steric structure were obtained.

Example 4 Construction of steric structures of amino acid sequences registered in PIR, SwissPlot and DAD

Further, sequences were obtained from PIR: http:// www-nbrf.georgetown.edu/pir/, Swiss Plot: http://www.expasy.ch/sprot/sprot-top.html, TrEMBL: http://www.expasy.ch/sprot/sprot-top.html, TrEMBLNEW: http://www.expasy.ch/sprot/sprot-top.html, DAD: ftp://ftp.ddbj.nig.ac.Jp, and atomic coordinates defining the steric structure were obtained as in Example 1. Alignment was performed employing PSAI-BLAST and using 1F88 as a reference protein as in Example 1 and, when the E value was 0.1 or less, it was determined to be GPCRs.

The number of sequences registered in each database are as follows:

Table 4

| | |
|---|---|
| PIR 65 | 182,162 |
| SWISSPROT 39 | 87,252 |
| TrEMBL 14 | 296,917 |
| TrEMBLNEW | 71,201 |
| DAD 12 | 514,763 |

Regarding the foregoing total 1,152,295, the number of sequences from which overlapping is omitted is 481,870 and, among them, 5,335 sequences were determined to be GPCRs. Among them, the number of sequences from which overlapping with the above-mentioned four databases (GCRDb, GPCRDb, ORDB, ExPASy) are omitted was 3,475 and, regarding them, atomic coordinates defining the steric structure were obtained.

The present invention has been explained in detail by referring to specific embodiments, but it would be apparent to a person skilled in the art that various variations and modifications can be made without departing from the spirit and the scope of the present invention.

The present application is based on Japanese Patent Application (Patent Application No.2000-279155) filed on September 14, 2000 and Japanese Patent Application (Patent Application No. 2000-350378) filed on November 17, 2000, the entire content of which is incorporated herein by reference.

INDUSTRIAL APPLICABILITY

The excellent points of the present invention compared with the previous methods are as follows:

(1) Alignment is performed by using the amino acid sequence database "GCRDb" as a profile, a model of an arbitrary G-protein-coupled receptor is produced, for example, based on the steric structure of rhodopsin (PDB ID:1F88), models of a 7-transmembrane $\alpha$ helix structure which is the common nature of GPCRs structures and loop structures of a variety of lengths connecting them are reproduced, whereby, the function of GCRDb serving as an information transmitting mechanism can be presumed.

(2) In particular, by using a crystal structure of rhodopsin (PDB ID:1F88) as a reference protein, it becomes possible to remarkably improve the steric structure model in a precision, although only structures having different helix. arrangements which are not classified into GCRDb of bacteriorhodopsin could be obtained previously.

(3) The amino acid sequence database "GCRDb" contains only amino acid sequences of GPCRs and, by using alignment software, for example; PSI-BLAST as a motif profile, it is possible to produce a model even when homology is low (20% or less) as compared with PDB:1F88 and, at the same time, suitable alignment is provided regarding arbitrary GPCRs, and an excellent method which does not give unnecessary alignment is provided regarding amino acid sequences other than GPCRs. Previously, it has been said that when homology is reduced to 20% or less, there is a possibility that a structure greatly differs and, therefore, this is a limit of homology modeling (Chochia, C. and Lesk, A.M., EMBO J. 5, 823-826 (1986)). However, a structure of seven helices arrangement of GPCRs obtained by the present method is well conserved, showing that this can be the steric structure model which is reliable in generation of drugs.

(4) In particular, the modeling software "FAMS" developed by the present inventors adopts an algorism for calculating structures similar to those of natural proteins, from proteins having high homology to proteins having low homology, and from a main chain to a side chain of an amino acid and, by performing modeling based on 1F88 which is a detailed steric structure coordinate present in PDB experimentally obtained using this, the reproductivity of a side chain of GPCRs has been remarkably improved.

(5) The database of the present invention contains atomic coordinates defining steric structures of GPCRs obtained from almost all sequences which can be currently obtained, of human, Drosophila, nematode, thearecress, yeast and the like, and proteins having presumed function of GPCRs, and is a database covering steric structures of almost all GPCRs.

As described above, the present method is a method which can construct the steric structure of arbitrary GPCRs with good precision, and the database of the present invention containing atomic coordinates defining the thus obtained GPCRs steric structures is extremely useful for designing, in particular, drug molecules such as medicines, agricultural chemicals and the like.

**Claims**

1.  A method for constructing the steric structure of a transmembrane protein, comprising:

    selecting a protein having the steric structure to be referred to from the known steric structures of 7-transmembrane G-protein-coupled receptors using a database of amino acid sequences of the receptors as a profile; performing alignment of a desired protein having an unknown steric structure and, based on the alignment information; and
    producing the steric structure of the desired protein having an unknown steric structure as a G-protein-coupled receptor.

2.  The method according to claim 1, wherein the performing alignment includes selecting a protein having the steric structure to be referred to from a database of known steric structures of G-protein-coupled receptors, and juxtaposing an amino acid sequence of the selected reference protein and an amino acid sequence of the desired protein.

**3.** The method according to claim 2, wherein the steric structure to be referred to is the steric structure of rhodopsin (PDB ID:1F88).

**4.** The method according to any one of claims 1 to 3, wherein the database of amino acid sequences to be used as a profile is selected from the group consisting of GCRDb, GPCRDB, ExPASy and ORDB.

**5.** The method according to any one of claims 1 to 4, wherein the database of amino acid sequences is used as a motif profile of PSI-BLAST.

**6.** The method according to claim 5, wherein in the alignment performed by PSI-BLAST, the identity between a final site-specific score matrix and the reference protein are calculated as an E value and the production of the steric structure is performed when the E value has a value of 0.1 or less.

**7.** The method according to any one of claims 1 to 6, further comprising performing re-alignment is so as to the given alignment information, in view of a combination of a disulfide bond and, the producing of the steric structure is performed based on the re-alignment information.

**8.** The method according to any one of claims 1 to 7, wherein the producing comprises:

obtaining a coordinate with regard to a C$\alpha$ atom in an amino acid from the steric structure to be referred to based on the resulting alignment information;
optimizing an atomic coordinate of C$\alpha$ so as to minimize an objective function;
adding other atoms of a main chain to the optimized atomic coordinate of C$\alpha$ to optimize an atomic coordinate of the main chain so as to minimize an objective function; and
adding other atoms of a side chain to the optimized atomic coordinate of the main chain to optimize so as to minimize an objective function.

**9.** An atomic coordinate defining the steric structure of a 7-transmembrane G-protein-coupled receptor obtained by the method according to any one of claims 1 to 8.

**10.** A computer readable recording medium in which the atomic coordinate according to claim 9 is recorded.

**11.** A database comprising the atomic coordinate according to claim 9.

**12.** A method for presuming the function of a protein, comprising:

selecting a protein having the steric structure to be referred to from the known steric structures of 7-transmembrane G-protein-coupled receptors using a database of amino acid sequences of the receptors as a profile;
performing alignment of a desired protein having an unknown function; and
judging that the desired protein is a 7-transmembrane G-protein-coupled receptor when the steric structure of the desired protein can be produced.

**13.** The method according to claim 12, wherein the performing alignment comprises:

selecting the steric structure to be referred to from a database of the known steric structures of G-protein-coupled receptors;
juxtaposing an amino acid sequence of the selected reference protein and an amino acid sequence of the desired protein, the database of amino acid sequences used as a profile is selected from the group consisting of GCRDb, GPCRDB, ExPASy and ORDB; and
judging that the steric structure of the desired protein can be produced when the alignment has reliability of 98% or more.

**14.** A method for designing a drug molecule, comprising:

identifying, retrieving, assessing or designing a desired drug molecule based on the interaction between the steric structure of a drug candidate molecule and the steric structure of a 7-transmembrane G-protein-coupled receptor produced by using the atomic coordinate according to claim 9, or the atomic coordinate of the recording medium according to claim 10 or the atomic coordinate of the database according to claim 11.

15. A method for screening a medicine or an agricultural chemical, comprising:

examining the drug molecule obtained by the method according to claim 14 for its activity and safety as a medicine or an agricultural chemical by a desired pharmacological or physiological test; and
selecting a drug molecule having the desired nature.

16. A method for manufacturing a medicine or an agricultural chemical, comprising:

formulating by blending the drug molecule obtained by the method according to claim 15 and a pharmaceutically or agriculturally or horticulturally acceptable carrier.

17. A drug molecule obtained by the method according to claim 14.

# FIG.1

```
┌─────────────────────┐
│   DESIRED AMINO     │ 10
│   ACID SEQUENCE     │
└─────────────────────┘
           │
           ▼
┌─────────────────────────────────────┐
│ ┌─────────────────────────────────┐ │
│ │  DATA BASE RETRIEVAL AND        │ │ 20
│ │  SEQUENCE ALIGNMENT             │ │
│ └─────────────────────────────────┘ │
│              │                       │
│              ▼                       │
│ ┌─────────────────────────────────┐ │
│ │ CONSTRUCTION AND OPTIMIZATION OF│ │ 30
│ │ Cα ATOM INITIAL COORDINATE      │ │
│ └─────────────────────────────────┘ │
│              │                       │
│              ▼                       │
│ ┌─────────────────────────────────┐ │
│ │ CONSTRUCTION AND OPTIMIZATION OF│ │ 40
│ │ MAIN CHAIN ATOMIC COORDINATE    │ │
│ └─────────────────────────────────┘ │
│              │                       │
│              ▼                       │
│ ┌─────────────────────────────────┐ │
│ │ CONSTRUCTION AND OPTIMIZATION   │ │ 50
│ │ OF SIDE CHAIN ATOMIC COORDINATE │ │
│ └─────────────────────────────────┘ │
└─────────────────────────────────────┘
              │
              ▼
      ┌───────────────┐
      │    FINAL      │ 60
      │  STRUCTURE    │
      └───────────────┘
```

# FIG.2

REFERENCE
PROTEIN

N-TERMINAL

C-TERMINAL

FRAGMENT A          FRAGMENT B          FRAGMENT C

CONSTRUCTION

REFERENCE
PROTEIN                    REFERENCE
PROTEIN

FRAGMENT
B

FRAGMENT
A                                              FRAGMENT
C

CONSTRUCTION OF Cα INITIAL
COORDINATE OF DESIRED PROTEIN

# FIG.3

```
AKG ERQ-S  PV DIDTHTAKYD PSL K PL
ARG NREAS  SP NVDTHSARYD --L K PL
 *  *   *     *      *** * **     *  *  **
```

# FIG.4

# FIG.5

EP 1 350 795 A1

```
>QRHUB2        354    HOMOLOGY    MATCH    MISMATCH   INSERTION   DELETION
>1F88          332     19.6%       65        262          5          27


FLLAPNRSHAPDHDVTQQRDEVWVV-GMGIVMSLIVLAIVFGNVLVITAIAKFERLQTVTNYFITSLACADLVMGLAVVP
FSNKTGVVRSPFEAPQYYLAEPWQFSMLAAYMFLLIMLGFPINFLTLYVTVQHKKLRTPLNYILLNLAVADLFMVFGGFT
*+++++++++*++++++++++*+++++++++++*++++++++++++++*++++++++++++++++*+++*+++++*++++++


FGAAHILMKMWTFGNFWCEFWTSIDVLCVTASIETLCVIAVDRYFAITSPFKYQSLLTKNKARVIILMVWIVSGLTSFLP
TTLYTSLHGYFVFGPTGCNLEGFFATLGGEIALWSLVVLAIERYVVVCKPM-SNFRFGENHAIMGVAFTWVMALACAAPP
+++++*+++++*+++++++++++++++*+++++++++++*+*+*+++++*+++++++++++++*++++++++++++++++++*


IQMHWYRATH-QEAINCYANE-TCCDFFTNQAYAIASSIVSFYVPLVIMVFVYSRVFQEAKRQLQKIDKSEGRFHVQNLS
LV-GWSRYIPEGMQCSCGIDYYTPHEETNNESFVIYMFVVHFIIPLIVIFFCYGQLVFTVKEAAAQQQESAT--------
+++++*+*+++++++++++++*+++++++++*++++++++++*+++++*+++++*+++++++++*+++++++*+++++++++


QVEQDGRTGHGLRRSSKFCLKEHKALKTLGIIMGTFTLCWLPFFIVNIVHVIQDNLI-RKEVYILLNWIGYVNSGFNPLI
---------------------TQKAEKEVTRMVIIMVIAFLICWLPYAGVAFYIFTHQGSDFGPIFMTIPAFFAKTSAVYNPVI
+++++++++++++++++++++++++*+++++++*++*+*++*+++*+++++++++++++++++++++++++++++++++*+*


Y-CRSPDFRIAFQELLCLRRSSLKAYGNGYSSNGNTGEQ
YIMMNKQFRNCMVTTLCCGKNPLGDDEASTTVSKTETSQ
*+++++*+++++*+++++*+++++++++++++++++*
```

# FIG.6

# FIG.7

## FIG.8

# FIG.9

# FIG.10

# FIG.11

ASN278

ASP104

# FIG.12

# FIG.13

ASN278

ASP104

# FIG.14

<div align="center">

**INTERNATIONAL SEARCH REPORT**

</div>

| | International application No. |
|---|---|
| | PCT/JP01/07918 |

| A. CLASSIFICATION OF SUBJECT MATTER |
|---|
| Int.Cl⁷  C07K1/00, C07K14/705, G06F17/30, G06F17/50 |

According to International Patent Classification (IPC) or to both national classification and IPC

| B. FIELDS SEARCHED |
|---|
| Minimum documentation searched (classification system followed by classification symbols)<br>Int.Cl⁷  C07K1/00, C07K14/705, G06F17/30, G06F17/50 |
| Documentation searched other than minimum documentation to the extent that such documents are included in the fields searched |
| Electronic data base consulted during the international search (name of data base and, where practicable, search terms used)<br>JICST FILE (JOIS), CA (STN), MEDLINE (STN), WPI (DIALOG), BIOSIS (DIALOG) |

| C. DOCUMENTS CONSIDERED TO BE RELEVANT | | |
|---|---|---|
| Category* | Citation of document, with indication, where appropriate, of the relevant passages | Relevant to claim No. |
| Y<br>A | Koji OGATA et al., "An automatic homology modeling method consisting of database searches and simulated annealing", Journal of Molecular Graphics and Modelling, June, 2000, Vol.18, pages 258 to 272 | 1-6,8,12-16<br>7 |
| Y<br>A | Krzysztof PALCZEWSKI et al., "Crystal Structure of Rhodopsin: A G Protein-Coupled Receptor", Science, August, 2000, Vol.289, pages 739 to 745 | 1-6,8,12-16<br>7 |
| Y | Alejandro A. SCHAFFER et al., "IMPALA: matching a protein sequence against a collection of PSI-BLAST-constructed position-specific score matrices", Bioinformatics, December, 1999, Vol.15, No.12, pages 1000 to 1011 | 5,6,8,13-16 |
| A | Julie D. THOMPSON et al., "CLUSTAL W: improving the sensitivity of progressive multiple sequence alignment through sequence weighting, position-specific gap penalties and weight matrix choice", Nucleic Acids Research, November, 1994, Vol.22, No.22, pages 4673 to 4680 | 1-8,12-16 |

| ☒  Further documents are listed in the continuation of Box C. | ☐  See patent family annex. |
|---|---|

| | | |
|---|---|---|
| *<br>"A"<br><br><br>"E"<br><br>"L"<br><br><br>"O"<br><br>"P" | Special categories of cited documents:<br>document defining the general state of the art which is not considered to be of particular relevance<br>earlier document but published on or after the international filing date<br>document which may throw doubts on priority claim(s) or which is cited to establish the publication date of another citation or other special reason (as specified)<br>document referring to an oral disclosure, use, exhibition or other means<br>document published prior to the international filing date but later than the priority date claimed | "T"<br><br><br><br>"X"<br><br><br><br>"Y"<br><br><br><br>"&" | later document published after the international filing date or priority date and not in conflict with the application but cited to understand the principle or theory underlying the invention<br>document of particular relevance; the claimed invention cannot be considered novel or cannot be considered to involve an inventive step when the document is taken alone<br>document of particular relevance; the claimed invention cannot be considered to involve an inventive step when the document is combined with one or more other such documents, such combination being obvious to a person skilled in the art<br>document member of the same patent family |

| Date of the actual completion of the international search<br>12 December, 2001 (12.12.01) | Date of mailing of the international search report<br>25 December, 2001 (25.12.01) |
|---|---|
| Name and mailing address of the ISA/<br>Japanese Patent Office | Authorized officer |
| Facsimile No. | Telephone No. |

Form PCT/ISA/210 (second sheet) (July 1992)

**EP 1 350 795 A1**

## INTERNATIONAL SEARCH REPORT

International application No.

PCT/JP01/07918

C (Continuation). DOCUMENTS CONSIDERED TO BE RELEVANT

| Category* | Citation of document, with indication, where appropriate, of the relevant passages | Relevant to claim No. |
|---|---|---|
| PY | Toshiaki UMEDA et al., "Tanpakushitsu Rittai Kouzou no Jidou Modeling", Jikken Igaku, July, 2001, Vol.19, No.11, pages 1470 to 1474 | 1-6,8,12-16 |

Form PCT/ISA/210 (continuation of second sheet) (July 1992)

**INTERNATIONAL SEARCH REPORT**

International application No.

PCT/JP01/07918

| Box I   Observations where certain claims were found unsearchable (Continuation of item 1 of first sheet) |
|---|

*This international search report has not been established in respect of certain claims under Article 17(2)(a) for the following reasons:*

1. ☒ Claims Nos.: 9-11
because they relate to subject matter not required to be searched by this Authority, namely:

The inventions as set forth in the above claims are merely offer of information.

2. ☒ Claims Nos.: 17
because they relate to parts of the international application that do not comply with the prescribed requirements to such an extent that no meaningful international search can be carried out, specifically:

Concerning the "medicinal molecule" as described in the above claim, it is unknown what particular molecules are involved in the scope thereof and what are not, even though the statement of the description is taken into consideration. Thus, the above claim is described in an extremely unclear manner. Such being the case, no meaningful search can be made on the above claim.

3. ☐ Claims Nos.:
because they are dependent claims and are not drafted in accordance with the second and third sentences of Rule 6.4(a).

| Box II   Observations where unity of invention is lacking (Continuation of item 2 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)